# EUROPÄISCHE PATENTSCHRIFT

(11) **EP 1 721 002 B1**
(45) Veröffentlichungstag und Bekanntmachung des Hinweises auf die Patenterteilung: **24.02.2010**
(21) Anmeldenummer: 04803924.2
(22) Anmeldetag: 15.12.2004
(51) Int. Cl.: C12Q 1/68

(54) **VERFAHREN ZUR ERKENNUNG VON SEPSIS**
METHOD FOR THE IDENTIFICATION OF SEPSIS
METHODE POUR IDENTIFIER UNE SEPSIE

(30) Priorität: 01.03.2004 DE 102004009952
(43) Veröffentlichungstag der Anmeldung: 15.11.2006
(73) Patentinhaber: SIRS-Lab GmbH, 07745 Jena (DE)
(72) Erfinder: RUSSWURM, Stefan, 07743 Jena (DE); DEIGNER, Hans-Peter, 68623 Lampertheim (DE)
(74) Vertreter: Kaiser, Jürgen
(86) Internationale Anmeldenummer: PCT/EP2004/014310
(87) Internationale Veröffentlichungsnummer: WO 2005/083115

(56) Entgegenhaltungen:
- WO-A-2004/087949
- US-A1- 2004 096 917
- CHINNAIYAN A M ET AL.: "Molecular signatures of sepsis. Multiorgan gene expression profiles of systemic inflammation" AMERICAN JOURNAL OF PATHOLOGY, Bd. 159, Oktober 2001 (2001-10), Seiten 1199-1209, XP008037039 in der Anmeldung erwähnt
- PATHAN N ET AL.: "The complexity of the inflammatory response to meningcoccal sepsis revealed by gene expression profiling using cDNA microarrays" CRITICAL CARE MEDICINE, Bd. 30, Nr. 12, Dezember 2002 (2002-12), Seite A47, XP008037050
- WIEGAND G ET AL.: "Gene expression pattern in human monocytes as a surrogate marker for systemic inflammatory response syndrome (SIRS)" MOLECULAR MEDICINE, Bd. 5, 1999, Seiten 192-202, XP008037023
- PRUCHA M ET AL.: "Expression profling: Toward an application in sepsis diagnostics" SHOCK, Bd. 22, Nr. 1, Juli 2004 (2004-07), Seiten 29-33, XP008036997

## Beschreibung

Die vorliegende Erfindung betrifft ein Verfahren zur *in vitro* Unterscheidung zwischen generalisierten, inflammatorischen, nichtinfektiösen Zuständen und generalisierten, inflammatorischen, infektiösen Zuständen gemäß Anspruch 1.

Die im folgenden verwendeten Begriffe "generalisierter, inflammatorischer, nichtinfektiöser Zustand" entspricht der Definition SIRS nach [1] und "generalisierter, inflammatorischer, infektiöser Zustand" entspricht der Definition Sepsis nach [1].

Insbesondere betrifft die vorliegende Erfindung die Anwendung von Genaktivitätsmarkern für die Diagnose der Sepsis.

Weiterhin betrifft die vorliegende Erfindung neue Diagnosemöglichkeiten, die sich aus experimentell abgesicherten Erkenntnissen im Zusammenhang mit dem Auftreten von Änderungen der Genaktivitäten (Transkription) bei Patienten mit SIRS und Sepsis ableiten lassen.

Trotz Fortschritte im pathophysiologischen Verständnis und der supportiven Behandlung von Intensivpatienten sind generalisierte inflammatorische Zustände wie SIRS und Sepsis, definiert entsprechend der ACCP/SCCM Konsensuskonferenz aus dem Jahre 1992 [1], bei Patienten auf Intensivstationen sehr häufig auftretende und erheblich zur Sterblichkeit beitragende Erkrankungen [2-3]. Die Sterblichkeit beträgt ca. 20 % bei SIRS, ca. 40 % bei Sepsis und steigt bei Entwicklung von multiplen Organdysfunktionen bis auf 70-80 % an [4-6]. Der Morbiditäts- und Letalitätsbeitrag von SIRS und Sepsis ist von fachübergreifender klinischmedizinischer Bedeutung, denn dadurch werden in zunehmendem Maße die Behandlungserfolge der fortgeschrittensten Therapieverfahren zahlreicher medizinischer Fachgebiete (z.B. Traumatologie, Neurochirurgie, Herz- /Lungenchirurgie, Viszeratchirurgie, Transplantationsmedizin, Hämatologie/ Onkologie, etc. ) gefährdet, denen ohne Ausnahme eine Erhöhung des Krankheitsrisikos für SIRS und Sepsis immanent ist. Dies drückt sich auch im kontinuierlichen Anstieg der Häufigkeit der Sepsis aus: zwischen 1979 und 1987 um 139% von 73,6 auf 176 Krankheitsfälle je 100.000 Krankenhauspatienten) [7]. Die Senkung der Morbidität und Letalität einer Vielzahl von schwer erkrankten Patienten ist daher an einen gleichzeitigen Fortschritt in der Vorbeugung, Behandlung und insbesondere der Erkennung und Verlaufsbeobachtung der Sepsis und schweren Sepsis gebunden.

Auf molekularer Ebene wird als Sepsis ein Krankheitsbild bezeichnet, welches durch pathogene Mikroorganismen verursacht wird. Auf dem Boden der Erschöpfung Infektionsort-naher, molekularer Kontroll- und Regulationsmöglichkeiten entwickelt sich eine generalisierte, den ganzen Organismus umfassende Entzündungsreaktion, die für die vom Arzt nachgewiesenen klinischen Symptome/Diagnosekriterien/SIRS-Kriterien nach [1] verantwortlich ist. Dieser generalisierte, inflammatorische Zustand (als Sepsis nach [1] definiert) geht mit Zeichen der Aktivierung verschiedener Zellsysteme (endotheliale Zellen, aber auch aller leukozytären Zellsysteme und vor allem des Monozyten/ Makrophagensystems) einher. Schließlich schädigen molekulare Mechanismen, die eigentlich den Wirt gegen invasive Mikroorganismen schützen sollen, dessen eigene Organe/Gewebe und tragen so entscheidend zur Entwicklung der vom Kliniker gefürchteten Organdysfunktionen bei [8-11].

Der Sepsisbegriff hat im Laufe der Zeit einen erheblichen Bedeutungswandel erfahren. Eine Infektion bzw. der dringliche Verdacht auf eine Infektion sind auch heute noch wesentlicher Bestandteil aktueller Sepsisdefinitionen.

Besondere Berücksichtigung findet jedoch dabei die Beschreibung Infektionsort-ferner Organfehlfunktionen im Rahmen der inflammatorischen Wirtsreaktion. Im internationalen Schrifttum haben sich zwischenzeitlich die Kriterien der Konsensuskonferenz des "American College of Chest Physicians/Society of Critical Care Medicine Consensus Conference (ACCP/SCCM)" aus dem Jahr 1992 am breitesten zur Definition des Sepsis-Begriffs durchgesetzt [1]. Entsprechend dieser Kriterien [1] werden die klinisch definierten Schweregrade "systemic inflammatory response syndrom" (SIRS), "Sepsis", "severe Sepsis" und "septic shock" unterschieden. Als SIRS wird dabei die systemische Antwort des inflammatorischen Systems auf einen infektiösen oder nichtinfektiösen Reiz definiert. Dazu müssen mindestens zwei der folgenden klinischen Kriterien erfüllt sein: Fieber >38°C oder Hypothermie <36°C, eine Leukozytose >12G/l oder eine Leukopenie <4G/l bzw. eine Linksverschiebung im Differentialblutbild, eine Herzfrequenz von über 90/min, eine Tachypnoe >20 Atemzüge/min oder ein PaCO2 (Partialdruck des Kohlendioxid im arteriellen Blut) <4,3 kPa. Als Sepsis werden solche klinischen Zustände definiert, bei denen die SIRS-Kriterien erfüllt sind und ursächlich eine Infektion nachgewiesen wird oder zumindest sehr wahrscheinlich ist. Eine schwere Sepsis ist vom zusätzlichen Auftreten von Organfehlfunktionen gekennzeichnet. Häufige Organfehlfunktionen sind Änderungen der Bewusstseinslage, eine Oligurie, eine Laktazidose oder eine Sepsisinduzierte Hypotension mit einem systolischen Blutdruck von weniger als 90 mmHg bzw. ein Druckabfall um mehr als 40 mmHg vom Ausgangswert. Wenn eine solche Hypotension nicht durch die Verabreichung von Kristalloiden und/oder Kolloiden zu beheben ist und es zusätzlich zu einer Katecholaminpflichtigkeit des Patienten kommt, so spricht man von einem septischen Schock. Dieser wird bei etwa 20 % aller Sepsispatienten nachgewiesen.

Sepsis ist das klinische Ergebnis von komplexen und stark heterogenen molekularen Vorgängen, die gekennzeichnet sind durch eine Einbeziehung von vielen Komponenten und deren Wechselwirkungen auf jeder organisatorischen Ebene des menschlichen Körpers: Gene, Zellen, Gewebe, Organe. Die Komplexität der zugrunde liegenden biologischen und immunologischen Prozesse haben viele Arten von Forschungsstudien hervorgerufen, die einen weiten Bereich klinischer Aspekte umfassen. Eines der hieraus zu erkennenden Ergebnisse war, dass die Bewertung neuer Sepsis-Therapien durch relativ unspezifische, klinisch-basierte Einschlusskriterien, welche die molekularen Mechanismen in nicht ausreichender Weise wiedergeben, erschwert wird [12]. Gleichfalls bestehen auf Grund der mangelnden Spezifität der heutigen Sepsis- und SIRS-Diagnose beim Kliniker große Unsicherheiten, ab welchem Zeitpunkt ein Patient einer spezialisierten Therapie, beispielsweise mit Antibiotika, die ihrerseits .beträchtliche Nebenwirkungen haben können, zugeführt werden soll [12]. So zeigte eine von der European Society of Intensive Care Medicine (ESICM) durchgeführte Umfrage, dass 71 % der befragten Ärzte Unsicherheit bei der Diagnosestellung einer Sepsis, trotz langjähriger klinischer Erfahrungen, hatten [22].

Bahnbrechende Entdeckungen in Molekularbiologie und Immunologie während der letzten zwei Jahrzehnte ließen ein vertieftes, mehr an den grundlegenden Mechanismen orientiertes Verständnis der Sepsis entstehen. Das dadurch entstandene Wissen um relevante Targets bildete wiederum die Basis für die Entwicklung gezielter und adjuvanter Therapiekonzepte, welche hauptsächlich auf der Neutralisierung wesentlicher Sepsismediatoren beruhen [13-16]. Eine Ursache für das Scheitern fast aller immunmodulatorischer Therapieansätze in klinischen Studien - trotz Effektivität im Tierexperiment - wird in der nur schlechten Korrelation zwischen den klinischen, eher symptomatisch orientierten Diagnosekriterien und den grundlegenden Mechanismen einer generalisierten Immunantwort gesehen [12, 17-18].

Rückblickend erstaunt dies nicht, da bereits gesunde Menschen bei alltäglichen Verrichtungen Veränderungen der Herz- bzw. Atemfrequenz aufweisen können, welche per Definition bereits die Diagnose eines SIRS zuließen. Bei Berücksichtigung unserer heutigen biomedizinischen Möglichkeiten muss es als Anachronismus erscheinen, dass jährlich 751.000 Patienten in den USA anhand o.g. ACCP/SCCM Kriterien diagnostiziert, klassifiziert und behandelt werden. Von namhaften Autoren wird deshalb schon lange kritisiert, dass zu Lasten einer verbesserten Sepsisdiagnose in der vergangenen Dekade zuviel Energie und finanzielle Ressourcen für die Suche nach einem "magic bullet" der Sepsistherapie aufgewendet wurden [19]. Auch fordern kürzlich publizierte Expertenmeinungen, dass zu einem besseren pathophysiologischen Verständnis der Sepsis eine Modifizierung der Konsensuskriterien nach [1] erforderlich ist [20-21]. Außerdem besteht unter vielen Medizinern Einigung darüber, dass die Konsensuskriterien nach [1] keiner spezifischen Definition von Sepsis entsprechen. So zeigte eine von der European Society of Intensive Care Medicine (ESICM) durchgeführte Umfrage, dass 71 % der befragten Ärzte Unsicherheit bei der Diagnosestellung einer Sepsis, trotz langjähriger klinischer Erfahrungen, hatten [22].

Aufgrund der oben genannten Probleme mit der Anwendung der Konsensuskriterien nach [1] werden unter Intensivmedizinern Vorschläge für eine sensitivere und spezifische Definitionen der verschiedenen Schweregrade der Sepsis diskutiert [2,23]. Neu ist dabei vor allem, dass molekulare Veränderungen direkt in die Beurteilung der Schwere einer Sepsis, aber auch den Einschluss in innovative Behandlungsverfahren der Sepsis (wie z.B. die Therapie mit aktiviertem rekombinanten Protein C) einbezogen werden sollen. Dieser Konsensusprozess [23], der gegenwärtig von fünf internationalen Fachgesellschaften getragen wird, ist zum gegenwärtigen Zeitpunkt noch längst nicht abgeschlossen. Ziel ist die Etablierung eines Systems zur Schweregradbeurteilung der Sepsis, das es ermöglicht, Patienten anhand ihrer individuellen Patientenreaktion auf der Basis ihrer prädisponierenden Bedingungen, der Art und des Ausmaße der Infektion, der Art und der Schwere der Wirtsantwort sowie des Grads der begleitenden Organdysfunktionen zu klassifizieren. Das beschriebene System wird mit PIRO, abkürzt nach den englischen Begriffen für "Predisposition", "Insult Infection", "Response" und "Organ dysfunction", bezeichnet. Davon kann dann die individuelle Wahrscheinlichkeit des Überlebens sowie des potentiellen Ansprechens auf die Therapie abgeleitet werden [23]. Gleichfalls sollen nichtinfektiöse Zustände, die gegenwärtig nach [1] unter dem Begriff SIRS subsummiert werden, entsprechend der individuellen Schwere des SIRS genauer klassifiziert werden. Auch hierfür werden Biomarker gesucht, die die Schwere des SIRS auch auf molekularer Ebene widerspiegeln und eine klare Abgrenzung von infektiösen Zuständen (gegenwärtig als Sepsis nach [1] klassifiziert) ermöglichen. Ähnliche Stadieneinteilungen werden bereits heute von anderen medizinischen Fachdisziplinen mit Erfolg angewendet, beispielsweise zur Klassifizierung der verschiedenen Krankheitsstadien im Bereich der Onkologie verwendet (TNM System, [24]).

Ein wesentliches Kriterium für die Diagnose einer Sepsis ist neben der generalisierten Entzündungsreaktion der Nachweis einer Infektion. Aus [25] ist jedoch bekannt, das beispielsweise von ca. 8500 Blutkulturen aus einer inneren medizinischen Abteilung nur bei ca. 15% aller Blutkulturen der Erreger bestimmt werden konnte. Von dem im gleichen Zeitraum (1 Jahr) bestimmten Blutkulturen einer Anästhesiologischen Intensivstation konnten sogar nur bei ca. 10% aller Blutkulturen die Krankheitserreger nachgewiesen werden. Diese Untersuchungen belegen die Problematik, einen frühzeitigen Nachweis der Infektion und somit einer frühe Diagnose der Sepsis zu ermöglichen. Als Ursache für den fehlenden Nachweis der Krankheitserreger mittels Blutkulturen können die mangelnde Eignung der Methode des Anzüchtens spezieller Erreger im allgemeinen sowie die meist oft begleitend eingesetzte Antibiotikatherapie, die dazu führt, dass die Erreger nicht mehr metabolisch aktiv und somit nicht anzuzüchten sind, im speziellen angesehen werden.

Verglichen mit den Konsensuskriterien nach [1] sollen in der Zukunft zusätzliche molekulare Parameter in die Diagnosestellung einbezogen werden [23], um so eine verbesserte Korrelation der molekularen inflammatorischen/ immunologischen Wirtsantwort mit dem Schweregrad der Sepsis zu ermöglichen. Nach solchen molekularen Biomarkern wird derzeit von verschiedenen wissenschaftlichen und kommerziellen Gruppen intensiv gesucht, da bisherige Parameter wie z.B. die Bestimmung des C-reaktiven Proteins oder des Procalcitonins nicht allen klinischen Anforderungen gerecht werden [26]. Auch aufgrund der unzureichenden Spezifität und Sensivität der Konsensuskriterien nach [1] und des mangelhaften oder verspäteten Nachweises der Ursache der Infektion besteht daher ein dringender Bedarf für neue diagnostische Verfahren, welche die Fähigkeit des Fachmanns verbessern sollen, eine Sepsis frühzeitig zu diagnostizieren, im klinischem Verlauf vergleichbar zu gestalten und bezüglich der individuellen Prognose und dem Ansprechen auf spezifische Behandlungen Aussagen abzuleiten.

Technologische Fortschritte, insbesondere die Entwicklung der Mikroarray-Technologie, versetzen den Fachmann nun in die Lage, 10000 oder mehr Gene und deren Genprodukte gleichzeitig zu vergleichen. Die Anwendung solcher Mikroarray-Technologien kann nun Hinweise auf den Status von Gesundheit, Regulationsmechanismen, biochemischer Wechselwirkungen und Signalübertragungsnetzwerken geben. Das Verbessern des Verständnisses darüber, wie ein Organismus auf Infektionen reagiert, sollte die Entwicklung von verstärkten Erkennungs-, Diagnose- und Behandlungsmodalitäten für Sepsis- Erkrankungen erleichtern.

Microarrays stammen vom "Southern blotting" [27] ab, was die erste Herangehensweise darstellt, DNA-Moleküle in einer räumlich ansprechbaren Art und Weise auf einer festen Matrix zu immobilisieren. Die ersten Mikroarrays bestanden aus DNA-Fragmenten, oft mit unbekannter Sequenz, und wurden auf eine poröse Membran (normalerweise Nylon) punktweise aufgebracht. Routinegemäß wurden cDNA, genomische DNA oder Plasmid-Bibliotheken verwendet, und das hybridisierte Material wurde mit einer radioaktiven Gruppe markiert [28-30].

Kürzlich hat es die Verwendung von Glas als Substrat und Fluoreszenz zur Detektion zusammen mit der Entwicklung neuer Technologien für die Synthese und für das Aufbringen der Nukleinsäuren in sehr hohen Dichten erlaubt, die Nukleinsäurearrays zu miniaturisierten bei gleichzeitiger Erhöhung des experimentellen Durchsatzes und des Informationsgehaltes [31-33].

Weiterhin ist aus WO 03/002763 bekannt, dass Microarrays grundsätzlich für die Diagnose von Sepsis und Sepsisähnlichen Zuständen verwendet werden können.

Eine Begründung für die Anwendbarkeit der Microarray-Technologie wurde zunächst durch klinische Untersuchungen auf dem Gebiet der Krebsforschung geliefert. Hier haben Expressionsprofile ihre Nützlichkeit bei der Identifizierung von Aktivitäten einzelner Gene oder Gengruppen gezeigt, die mit bestimmten klinischen Phänotypen korrelieren [34]. Durch die Analyse vieler Proben, die von Individuen mit oder ohne akute Leukämie oder diffusen B-Zell Lymphomen stammten, wurden Genexpressionsmarker (RNA) gefunden und anschließend für die klinisch relevante Klassifizierung dieser Krebsarten angewandt [34,35]. Golub et al. haben herausgefunden, daß verlässliche Vorhersagen nicht aufgrund von irgendeinem einzelnen Gen gemacht werden können, aber daß Vorhersagen, die auf der Veränderung der Transkritiption von 53 Genen (ausgewählt aus über 6000 Genen, die auf den Arrays vertreten waren) basieren, sehr genau sind [34].

Alisadeh et al. [35] untersuchten große B-Zell Lymphome (DLBCL). Die Autoren erarbeiteten Expressionsprofile mit einem "Lymphochip", einem Microarray, der 18 000 Klone komplementärer DNA trug und entwickelt worden war, um Gene zu überwachen, die in normale und abnormale Lymphozytenentwicklung involviert sind. Unter Anwendung von Cluster-Analysen waren sie in der Lage, DILBCL in zwei Kategorien einzuteilen, welche starke Unterschiede bezüglich der Überlebenschancen der Patienten aufzeigten. Die Genexpressionsprofile dieser Untergruppen entsprachen zwei bedeutsamen Stadien der B-Zelldifferenzierung.

Auch auf dem Gebiet der Neurobiologie sind eine Vielzahl von Studien zur Identifizierung von Genaktivitätsmarkern mittels Microarray-Technologie durchgeführt worden [36]. Gleiches gilt für die Untersuchung der molekularen Veränderungen, welche durch einzelne Bestandteile von bakteriellen Gram negativen Erregern (z.B. unter Verwendung von Stimulationsexperimenten mit Lipopolysacchariden) ausgelöst werden [37]. Solche Untersuchungen werden in der Regel mittels dem Fachmann bekannten zellulären Modellsystemen, z.B. menschlichen Endothelzellkulturen in [38], oder in menschlichen leukozytären Zellkulturen [41], oder auch mittels Untersuchungen menschlicher Gewebe, nicht aber Blut, z.B. in [39], durchgeführt. Dabei richtet sich das experimentelle Bestreben jeweils auf die Identifizierung bislang unbekannter Teilnehmer der zellulären Signalübertragungswege, um auf diesem Wege die molekulare Natur einer Entzündung besser beschreiben zu können. Alternativ werden regelmäßig für solche Fragestellungen auch Tierexperimente, z.B. in Mäusen siehe auch [40], durchgeführt.

Ein weiteres Beispiel für die Verwendung der differentiellen Genexpression zur vertiefenden Untersuchung der molekularen Vorgänge bei einer generalisierten Entzündungsreaktion konnte in [42] auf der Basis cDNA basierter Mikroarrays gezeigt werden.

Chinnaiayan et al. [39] untersuchten unter Verwendung von DNA Microarrays mit 7398 immobilisierten Genen die Sepsis-induzierten Genexpressionsprofile in verschiedenen Organsystemen. Es wurde gezeigt, dass obwohl es eine Gruppe von Genen gibt, deren Expressionsmuster in vielen Organen ähnlich ist, jedes der untersuchten Organe zusätzlich eine eigene spezifische molekulare Antwort auf eine Sepsis zeigt.

Auf ähnliche Weise wurde die Genexpressionsprofile von Blutproben von Patienten mit schwerer Meningokokken-Sepsis bestimmt und mit den Profilen von Gesunden verglichen [43]. Dazu wurden DNA-Microarrays mit 38000 immobilisierten Genen verwendet.

Unter Verwendung einer optimierten PCR-Technik und spezifischer Primer wurden die Genexpressionsprofile von SIRS-Patienten ebenfalls untersucht und mit den Expressionsprofilen Gesunder verglichen [44]. Es wurde festgestellt, dass bestimmte Banden auf einem nach der PCR angefertigten Gel mit SIRS assoziiert werden konnten, während zwei weitere Gelbanden nur bei gesunden Kontrollen beobachtet wurden.

Die Messung von Genexpressionsprofilen zur Unterscheidung zwischen SIRS entsprechend [1] und Sepsis entsprechend [1] wurde noch nicht beschrieben.

Ausgangspunkt für die in der vorliegenden Patentanmeldung offenbarten Erfindung ist die Erkenntnis, daß Genaktivitäten verschiedener Gene in biologischen Proben eines Individuums, bei dem Sepsis-typische Krankheitserscheinungen (entsprechend der Definition in [1]) festgestellt werden, sich von den Genaktivitäten der gleichen Gene in Proben von Individuen, bei denen eine SIRS diagnostiziert wurde, unterscheiden. Diese Unterschiede in den Genaktivitäten lassen es somit zu, Patienten mit einer Sepsis, also einer zusätzlichen infektiösen Komplikation, von Patienten ohne diese infektiöse Komplikation (SIRS entsprechend [1]) zu unterscheiden. Wie bereits an anderer Stelle dargelegt, ist diese Unterscheidung bislang mit erheblichen Nachteilen verbunden, aber für die Einleitung einer spezialisierten medizinischen Therapie und damit für das Verbessern der individuellen Prognose für das Überleben sehr bedeutungsvoll.

Der vorliegenden Erfindung liegt somit die Aufgabe zugrunde, ein Verfahren zur Verfügung zu stellen, das die Unterscheidung zwischen generalisierten, inflammatorischen, nichtinfektiösen Zuständen (SIRS entsprechend [1]) und generalisierten, inflammatorischen, infektiösen Zuständen (Sepsis entsprechend [1]) ermöglicht.

Diese Aufgabe wird durch ein Verfahren mit den kennzeichnenden Merkmalen des Anspruchs 1 gelöst.

Weiterhin liegt der vorliegenden Erfindung die Aufgabe zugrunde, eine Verwendungsmöglichkeit von Markern in einem Verfahren gemäß Anspruch 1-25 zur Verfügung zu stellen.

Diese Aufgabe wird durch die Verwendung gemäß Anspruch 26-32 gelöst.

Das erfindungsgemäße Verfahren ist **dadurch gekennzeichnet, daß** man in einer Probe einer biologischen Flüssigkeit eines Individuums die Aktivität eines oder mehrerer Markergene bestimmt und aus der festgestellten Anwesenheit und/oder Menge des bestimmten Genprodukts zwischen SIRS und Sepsis (beides entsprechend [1]) unterscheiden kann.

Eine Ausführungsform der Erfindung ist **dadurch gekennzeichnet, daß** das Verfahren zur *in vitro* Unterscheidung zwischen SIRS und Sepsis, wobei es folgende Schritte umfasst:
a) Isolieren von Proben-RNA aus einer biologischen Probe;
b) Markieren der Proben-RNA und/oder wenigstens einer DNA, die eine zur Unterscheidung zwischen SIRS und Sepsis (beides entsprechend [1]) spezifische Genaktivität repräsentiert und/oder ein spezifisches Gen oder Genfragment ist, mit einem detektierbaren Markerln-Kontakt-Bringen von Kontroll-RNA, mit wenigstens einer DNA, unter Hybridisierungsbedingungen, wobei die DNA ein zur Unterscheidung SIRS und Sepsis spezifisches Gen oder Genfragment ist;
c) quantitatives Erfassen der Markierungssignale der hybridisierten Proben-RNA und der Kontroll-RNA;
d) Vergleichen der quantitativen Daten der Markierungssignale, um eine Aussage zu treffen, ob zur Unterscheidung zwischen SIRS und Sepsis (beides entsprechend [1]) spezifische Gene oder Genfragmente in der Probe stärker oder schwächer exprimiert sind als in der Kontrolle.

Eine weitere Ausführungsform der Erfindung ist **dadurch gekennzeichnet, daß** man die Kontroll-RNA vor dem Messen der Proben-RNA mit der DNA hybridisiert und die Markierungssignale des Kontroll-RNA/DNA-Komplexes erfasst und gegebenenfalls in Form einer Kalibrierkurve oder -tabelle ablegt.

Eine weitere Ausführungsform der Erfindung ist **dadurch gekennzeichnet, daß** als Proben-RNA mRNA verwendet'wird.

Eine weitere Ausführungsform der Erfindung ist **dadurch gekennzeichnet, daß** die DNA an vorbestimmten Bereichen auf einem Träger in Form eines Microarrays angeordnet, insbesondere immobilisiert, wird.

Eine weitere Ausführungsform der Erfindung ist **dadurch gekennzeichnet, daß** das Verfahren zur differentialdiagnostischen Früherkennung, zur Kontrolle des therapeutischen Verlaufs und zur Risikoabschätzung für Patienten eingesetzt wird.

Eine weitere Ausführungsform der Erfindung ist **dadurch gekennzeichnet, daß** die Probe ausgewählt wird aus: Körperflüssigkeiten, insbesondere Blut, Liquor, Urin, Ascitesflüssigkeit, Seminalflüssigkeit, Speichel, Punktat; Zellinhalt oder eine Mischung davon.

Eine weitere Ausführungsform der Erfindung ist **dadurch gekennzeichnet, daß** Zellproben gegebenenfalls einer lytischen Behandlung unterzogen werden, um deren Zellinhalte freizusetzen.

Eine weitere Ausführungsform der Erfindung ist **dadurch gekennzeichnet, daß** es sich bei der biologischen Probe um die eines Menschen handelt.

Eine weitere Ausführungsform der Erfindung ist **dadurch gekennzeichnet, daß** das zur Unterscheidung SIRS und Sepsis spezifische Gen oder Genfragment ausgewählt wird aus der Gruppe bestehend aus SEQ-ID No. 1 bis SEQ-ID No. 91, sowie Genfragmenten davon mit wenigstens 5-2000, bevorzugt 20-200, mehr bevorzugt 20-80 Nukleotiden.

Diese Sequenzen mit der Sequenz ID: 1 bis zur Sequenz ID: 91 sind durch den Umfang der vorliegenden Erfindung mit umfaßt und sind dem angefügten 42-seitigen, 91 Sequenzen umfassenden, Sequenzprotokoll, das somit Teil der Erfindung ist, im Einzelnen offenbart. Dieses Sequenzprotokoll beinhaltet zudem eine Zuordnung der einzelnen Sequenzen mit der Sequenz ID: 1 bis zur Sequenz ID: 91 zu deren GenBank Accession Nr. (Internet-Zugang über http://www.ncbi.ntm.nih.gov/).

Eine weitere Ausführungsform der Erfindung ist **dadurch gekennzeichnet, dass** die immobilisierten oder freien DNA-Sonden markiert werden. Für diese Ausführungsform finden selbstkomplementäre Oligonukleotide, so genannte Molecular beacons, als Sonden Verwendung. Sie tragen an ihren Enden ein Fluorophor/Quencher-Paar, so daß sie in Abwesenheit einer komplementären Sequenz in einer gefalteten Haarnadelstruktur vorliegen und erst mit einer entsprechenden Probensequenz ein Fluoreszenzsignal liefern. Die Haarnadelstruktur der Molecular Beacons ist so lange stabil, bis die Probe an der spezifischen Fängersequenzsequenz hybridisiert, was zu einer Konformationsänderung und damit auch Freisetzung der Reporterfluoreszenz führt.

Eine weitere Ausführungsform der Erfindung ist **dadurch gekennzeichnet, daß** wenigstens 2 bis 100 unterschiedliche cDNAs verwendet werden.

Eine weitere Ausführungsform der Erfindung ist **dadurch gekennzeichnet, daß** wenigstens 200 unterschiedliche cDNAs verwendet werden.

Eine weitere Ausführungsform der Erfindung ist **dadurch gekennzeichnet, daß** wenigstens 200 bis 500 unterschiedliche cDNAs verwendet werden.

Eine weitere Ausführungsform der Erfindung ist **dadurch gekennzeichnet, daß** wenigstens 500 bis 1000 unterschiedliche cDNAs verwendet werden.

Eine weitere Ausführungsform der Erfindung ist **dadurch gekennzeichnet, daß** wenigstens 1000 bis 2000 unterschiedliche cDNAs verwendet werden.

Eine weitere Ausführungsform der Erfindung ist **dadurch gekennzeichnet, daß** die als DNA von den in Anspruch 10 aufgelisteten Genen ersetzt wird durch von deren RNA abgeleiteten Sequenzen, synthetische Analoga, Aptamere sowie Peptidonukleinsäuren.

Eine weitere Ausführungsform der Erfindung ist **dadurch gekennzeichnet, daß** die synthetische Analoga der Gene 5-100, insbesondere ca. 70 Basenpaare umfassen.

Eine weitere Ausführungsform der Erfindung ist **dadurch gekennzeichnet, daß** als detektierbarer Marker ein radioaktiver Marker, insbesondere ³²P, ¹⁴C, ¹²⁵I, ¹⁵⁵Eu, ³³P oder ³H verwendet wird.

Eine weitere Ausführungsform der Erfindung ist **dadurch gekennzeichnet, daß** als detektierbarer Marker ein nicht radioaktiver Marker, insbesondere ein Farb- oder Fluoreszenzmarker, ein Enzymmarker oder Immunmarker, und/oder quantum dots oder ein elektrisch messbares Signal, insbesondere Potential- und/oder Leitfähigkeits- und/oder Kapazitätsänderung bei Hybridisierungen, verwendet wird.

Eine weitere Ausführungsform der Erfindung ist **dadurch gekennzeichnet, daß** die Proben-RNA und Kontroll-RNA und/oder enzymatische oder chemische Derivate dieselbe Markierung tragen.

Eine weitere Ausführungsform der Erfindung ist **dadurch gekennzeichnet, daß** die Proben-RNA und Kontroll-RNA und/oder enzymatische oder chemische Derivate unterschiedliche Markierungen tragen.

Eine weitere Ausführungsform der Erfindung ist **dadurch gekennzeichnet, daß** die DNA-Sonden auf Glas oder Kunststoff, immobilisiert werden.

Eine weitere Ausführungsform der Erfindung ist **dadurch gekennzeichnet, daß** die einzelnen DNA Moleküle über eine kovalente Bindung an das Trägermaterial immobilisiert werden.

Eine weitere Ausführungsform der Erfindung ist **dadurch gekennzeichnet, daß** die einzelnen DNA Moleküle mittels elektrostatischer- und/oder Dipol-Dipol- und/oder hydrophobische Wechselwirkungen und/oder Wasserstoffbrücken an das Trägermaterial immobilisiert werden.

Eine weiter Ausführungsform der Erfindung besteht in der Verwendung von rekombinant oder synthetisch hergestellten, zur Unterscheidung zwischen SIRS und Sepsis (beides entsprechend [1]) spezifischen Nukleinsäuresequenzen, Partialsequenzen einzeln oder in Teilmengen als Kalibrator in Sepsis -Assays und/oder zur Bewertung der Wirkung und Toxizität beim Wirkstoffscreening und/oder zur Herstellung von Therapeutika und von Stoffen und Stoffgemischen, die als Therapeutikum vorgesehen sind, zur Vorbeugung und Behandlung von SIRS und Sepsis.

Es ist dem Fachmann klar, daß die in den Ansprüchen dargelegten einzelnen Merkmale der Erfindung ohne Einschränkung beliebig miteinander kombinierbar sind.

Als Markergene im Sinne der Erfindung werden alle abgeleiteten DNA-Sequenzen, Partialsequenzen und synthetischen Analoga (beispielsweise Peptido-Nukleinsäuren, PNA) verstanden. Die auf Bestimmung der Genexpression auf RNA-Ebene bezogene Beschreibung der Erfindung stellt keine Einschränkung sondern nur eine beispielhafte Anwendung dar.

Die auf Blut bezogene Beschreibung der Erfindung stellt nur eine beispielhafte Anwendung der Erfindung dar. Als biologische Flüssigkeiten im Sinne der Erfindung werden alle Körperflüssigkeiten des Menschen verstanden.

Eine Anwendung des erfindungsgemäßen Verfahrens liegt in der Messung der differentiellen Genexpression zur Unterscheidung zwischen SIRS und Sepsis (beides entsprechend [1]). Hierzu wird die RNA aus dem Vollblut von entsprechenden Patienten und eine Kontrollprobe eines gesunden Probanden oder nicht-infektiösen Patienten isoliert. Die RNA wird anschließend markiert, beispielsweise radioaktiv mit ³²P oder mit Farbstoffmolekülen (Fluoreszenz). Als Markierungsmoleküle können alle im Stand der Technik zu diesem Zwecke bekannten Moleküle und/oder Detektionssignale eingesetzt werden. Entsprechende Moleküle und/oder Verfahren sind dem Fachmann ebenfalls bekannt.

Die so markierte RNA wird anschließend mit auf einem Microarray immobilisierten DNA-Molekülen hybridisiert. Die auf dem Microarray immobilisierten DNA-Moleküle stellen eine spezifische Auswahl den Gene gemäß Anspruch 10 dieser Erfindung zur Unterscheidung SIRS und Sepsis dar.

Die Intensitätssignale der hybridisierten Moleküle werden im Anschluss durch geeignete Messgeräte (Phosporimager, Microarray-Scanner) gemessen und durch weitere softwaregestützte Auswertungen analysiert. Aus den gemessenen Signalintensitäten werden die Expressionsverhältnisse zwischen der Patientenprobe und der Kontrolle bestimmt. Aus den Expressionsverhältnissen der unter- und/oder überregulierten Gene lassen sich, wie in den nachstehend dargestellten Experimenten, Rückschlüsse auf die Unterscheidung SIRS und Sepsis ziehen.

Eine weitere Anwendung des erfindungsgemäßen Verfahrens besteht in der Messung der differentiellen Genexpression für die therapiebegleitende Bestimmung der Wahrscheinlichkeit, daß Patienten auf die geplante Therapie ansprechen werden, und/oder für die Bestimmung des Ansprechens auf eine spezialisierte Therapie und/oder auf die Festlegung des Therapieendes im Sinne eines "drug monitoring" bei Patienten mit SIRS und Sepsis. Hierzu wird aus den in zeitlichen Abständen gesammelten Blutproben des Patienten die RNA (Proben-RNA) isoliert. Die verschiedenen RNA-Proben werden zusammen mit der Kontrollprobe markiert und mit ausgewählten Genen gemäß dem Anspruch 10, welche auf einem Microarray immobilisiert sind, hybridisiert. Aus den jeweiligen Expressionsverhältnissen lässt sich somit beurteilen, welche Wahrscheinlichkeit besteht, daß Patienten auf die geplante Therapie ansprechen werden und/oder ob die begonnene Therapie wirksam ist und/oder wie lange die Patienten noch entsprechend therapiert werden müssen und/oder ob der maximale Therapieeffekt mit der verwendeten Dosis und Dauer schon erreicht worden ist.

Eine weitere Anwendung des erfindungsgemäßen Verfahrens besteht in der Verwendung der RNA der Gene nach Anspruch 10 zur Gewinnung von quantitativen Informationen durch Hybridisierungs-unabhängige Verfahren, insbesondere enzymatische oder chemische Hydrolyse, anschließende Quantifizierung der Nukleinsäuren und/oder von Derivaten und/oder Fragmenten derselben

Eine weitere Anwendung des erfindungsgemäßen Verfahrens besteht in der Verwendung der Genaktivitäten zur Unterscheidung SIRS und Sepsis für die elektronischen Weiterverarbeitung zum Zweck der Herstellung von Software für Diagnosezwecke (z.B. für Patientendatenmanagementsystemen), oder Expertensystemen zur Modellierung von zellulärer Signalübertragungswegen oder zum Zweck der Computer-gestützten Modellierung von Entzündungszuständen auch in Modellorganismen wie beispielsweise C. *elegans* oder *Saccharomyces cerevisiae.*

Weitere Vorteile und Merkmale der vorliegenden Erfindung ergeben sich aufgrund der Beschreibung des Ausführungsbeispiels.

### Ausführungsbeispiel:

Untersuchungen zur differentiellen Genexpression zur Unterscheidung von generalisierten, inflammatorischen, nichtinfektiösen Zuständen (entsprechend SIRS nach [1]) und generalisierten, inflammatorischen, infektiösen Zuständen (ensprechend Sepsis nach [1]).

Für die Messung der differentiellen Genexpression zur Unterscheidung SIRS und Sepsis wurden Untersuchungen von Vollblutproben von Patienten, welche auf einer operativen Intensivstation behandelt wurden, durchgeführt.

Es wurden Vollblutproben von fünf männlichen und einer weiblichen Patienten/in abgenommen (Patientenproben). Jeder dieser Patienten entwickelte im Rahmen seiner intensivmedizinischen Betreuung nach einer Bypass-Operation eine Sepsis. Die Patientenproben wurden sofort (innerhalb von 12 Stunden) nach erstemaliger Diagnose einer Sepsis entsprechend der Klassifikation nacht [1] entnommen. Ausgewählte Charakteristika der Patienten mit Sepsis sind in Tabelle 1 dargestellt. Dabei werden Angaben zum Alter, Geschlecht, der Ursache der Sepsis (siehe Diagnose) sowie klinischer Schwere, gemessen anhand der im klinischen Schrifttum gut belegten APACHE-II- und SOFA-Scores (jeweils in Punkte), gemacht. Gleichfalls sind die Plasmaproteinspiegel von Procalcitonin (PCT), einem neuartigen Sepsismarker, das Center of Disease (CDC)-Kriterium (siehe http://www.cdc.gov) und der individuelle Überlebensstatus angegeben.

Als Kontrollproben dienten Vollblutproben der gleichen Patienten. Diese wurden jeweils am 1. Tag postoperativ abgenommen. Zu diesem Zeitpunkt hatte jeder ein operationsbedingtes SIRS definiert entsprechend [1] (aufgrund des Einsatzes der Herz-Lungen-Maschine).

**Tabelle1: Daten der Patientengruppe**

| **Patient** | **Alter** | **Geschlecht** | **Probe** | **Diagnose** | **Klassifikation nach [1]** | **APACHE-II Score [Punkte]** | **SOFA Score [Punkte]** | **PCT [ng/ml]** | **CDC- Kriterien** | **Überlebensstat us** |
|---|---|---|---|---|---|---|---|---|---|---|
| Patient 1 | 60 | männlich | Kontrolle | 3-Gefäß-KHK | SIRS | 9 | 6 | 5,38 | Pneumonie | überlebt |
| | | | Probe | | Sepsis | | 11 | 13,1 | | |
| Patient 2 | 80 | weiblich | Kontrolle | Aortenklappenstenose | SIRS | 14 | 8 | 2,09 | Pneumonie | verstorben |
| | | | Probe | | Sepsis | | 8 | 3,81 | | |
| Patient 3 | 76 | männlich | Kontrolle | Mitralklappenin-suffizienz | SIRS | 15 | 9 | 9,11 | Pneumonie | überlebt |
| | | | Probe | | Sepsis | | 10 | 1,2 | | |
| Patient 4 | 61 | männlich | Kontrolle | Mitralklappen-stenose | SIRS | 11 | 12 | 14,5 | Intraab-domnielle Infektion | verstorben |
| | | | Probe | | Sepsis | | 21 | 44 | | |
| Patient 5 | 63 | männlich | Kontrolle | Atherosklerotische Herzkrankheit | SIRS | 12 | 11 | 1,23 | Fokus unklar | verstorben |
| | | | Probe | | Sepsis | | 14 | 3,64 | | |
| Patient 6 | 65 | männlich | Kontrolle | Atherosklerotische Herzkrankheit | SIRS | 16 | 8 | 4,22 | Pneumonie | überlebt* |
| | | | Probe | | Sepsis | | 5 | 0,3 | | |

Nach Abnahme des Vollblutes wurde die totale RNA unter Anwendungen des PAXGene Blood RNA Kit gemäß den Vorgaben des Herstellers (Qiagen) isoliert. Im Anschluss wurde aus der totalen RNA die doppelsträngige cDNA mittels reverser Transkription unter Verwendung des Agilent Low RNA Input Fluorescent Amplification Kit (Agilent) nach dem Protokoll des Herstellers synthetisiert, wobei am Poly-A-Ende der cDNA ein T7 RNA Polymerase-Promoter angehängt wurde. Anschließend wurde die cDNA unter Verwendung des T7 RNA Polymerase-Promoters und gleichzeitiger Einfügen von Fluoreszenz-Nukleotiden Cy3/Cy5-Cytosintriphosphat (Amersham) in cRNA synthetisiert, welche als Hybridisierungsmoleküle dienten. Alle RNA-Proben wurden in zwei Aliquote geteilt, wovon ein Aliquot mit Cy3-CTP und das andere Aliquot mit Cy5-CTP markiert wurde. Dadurch konnte jede Kohybridisierung zweifach unter Nutzung der umgekehrten RNA/Fluoreszenzfarbstoff Kombination durchgeführt werden.

Jede der vorbereiteten Kombination der Hybridisierungsmoleküle wurde sowohl mit dem Microarray 1A Oligo als 1B Oligo der Fa. Agilent entsprechend dem Protokoll des Herstellers hybridisiert. Zusammen enthalten diese beiden Microarrays 36000 Gene und ESTs (Expressed Sequence Tags). Die Fluoreszenzsignale der hybridisierten Moleküle wurden mittels eines Auslesegerätes (Agilent DNA Microarray Scanner) gemessen und mit der Software Agilent Feature Software berechnet.

### Auswertung

Für die Auswertung wurde die mittlere Intensität eines Spots als der Medianwert zugehörigen der Spotpixel bestimmt.

### Korrektur systematischer Fehler:

Von dem Median der Spotpixel wurde der Median der Pixel des lokalen Hintergrunds abgezogen. Für alle weiteren Berechnungen wurden die Signale mittels arcus sinus hyperbolicus transformiert. Die Normalisierung erfolgte nach dem Ansatz von Huber et al. [45]. Dabei wurden der additive und der multiplikative Bias innerhalb eines Microarrays aus 70% der vorhandenen Genproben geschätzt. Korrigiert wurden dann die Intensität-Signale aus dem roten Kanal.

### Statistischer Vergleich

Für den Vergleich wurde der gepaarte Student-Test verwendet. Der Test wurde unabhängig für beide experimentellen Bedingungen durchgeführt. Für die Auswahl der differenziert exprimierten Gene wurden der zugehörige p-Wert und die mittlere Expressionsänderung innerhalb der Gruppe bewertet.

### Ergebnisse

Für die Gruppe der ausgewählten Gene gilt, dass in beiden Experimenten der zugehörige p-Wert kleiner als 0.05 und die mittlere Expressionsänderung größer als 1.2 war.

Die Höhe des Expressionsverhältnisses jedes Gens stellte das Kriterium für eine Sortierung der untersuchten Gene dar. Von Interesse waren die Gene, die in den Patientenproben gegenüber Kontrollproben am meisten überexprimiert bzw. unterexprimiert wurden.

Aus Tabelle 2 ist ersichtlich, dass 51 Gene der Patientenprobe gefunden wurden, die in der Patientenprobe gegenüber der Kontrollprobe signifikant überexprimiert waren. Weiterhin wird aus Tabelle 3 deutlich, dass 17 Gene der Patientenprobe gegenüber der Kontrollprobe signifikant unterexprimiert waren. Aus den Ergebnissen wird deutlich, dass die in Tabelle 2 und Tabelle 3 aufgeführten Genaktivitäten zwischen generalisierten, inflammatorischen, infektiösen Zuständen (entsprechend Sepsis nach [1]) und generalisierten, inflammatorischen, nichtinfektiösen Zuständen (entsprechend SIRS nach [1]) unterscheiden. Somit stellen die aufgeführten Genaktivitäten Marker für eine Unterscheidung zwischen SIRS und Sepsis dar.

**Tabelle 2: Signifikant gesteigerte Genaktivitäten in Proben von Patienten mit Sepsis nach [1], dargestellt als deren relatives Verhältnis zu den korrespondierenden Genaktivitäten des selben Patienten im Zustand SIRS nach [1]**

| **GenBank Acc. Number** | **HUGO-Name** | **mean: Cy5vsCy3** | **mean: Cy3vsCy5 Cy5vsCy3** | **p:** | **Cy3vsCy5** | **Seq.-ID** |
|---|---|---|---|---|---|---|
| NM_006986.2 | MAGED1 | 1,33 | 1,36 | 0,01 | 0,01 | 1 |
| NM_005319.1 | H1F2 | 1,21 | 1,09 | 0,01 | 0,01 | 2 |
| NM_001925.1 | DEFA4 | 1,16 | 1,26 | 0,00 | 0,00 | 3 |
| NM_006516.1 | SLC2A1 | 1,02 | 0,84 | 0,02 | 0,02 | 4 |
| D87452.1 | IHPK1 | 0,97 | 0.88 | 0,01 | 0,01 | 5 |
| NM_020070.1 | IGLL1 | 0,97 | 0,98 | 0,02 | 0,01 | 6 |
| NM_022771.1 | FLJ12085 | 0,97 | 0,90 | 0,00 | 0,00 | 7 |
| NM_001738.1 | CA1 | 0,88 | 0,89 | 0,00 | 0,00 | 9 |
| L05148.1 | ZAF70 | 0,82 | 0,74 | 0,02 | 0,01 | 10 |
| BC021275.1 | FLJ32987 | 0,68 | 0,65 | 0,03 | 0,01 | 13 |
| NM_005321.1 | H1F4 | 0,65 | 0,61 | 0,01 | 0,01 | 15 |
| NM_005664.1 | LCN2 | 0,58 | 0,60 | 0,01 | 0,00 | 17 |
| NM_003250.1 | THRA | 0,56 | 0,45 | 0,04 | 0,02 | 18 |
| NM_005067.1 | SIAH2 | 0,54 | 0,54 | 0,00 | 0,00 | 19 |
| NM_016417.1 | LOC51218 | 0,49 | 0,30 | 0,01 | 0,04 | 21 |
| NM_005764.1 | DD96 | 0,47 | 0,60 | 0,04 | 0,01 | 22 |
| NM_033445.1 | H2AFA | 0,46 | 0,40 | 0,00 | 0,04 | 23 |
| M18728.1 | CEACAM6 | 0,45 | 0,29 | 0,01 | 0,03 | 24 |
| NM_003516.1 | H2AFO | 0,43 | 0,47 | 0,06 | 0,05 | 27 |
| NM_018639.1 | LOC55884 | 0,43 | 0,28 | 0,04 | 0,04 | 28 |
| BC029812.1 | ZNF145 | 0,40 | 0,27 | 0,02 | 0,02 | 29 |
| NM_021052.1 | H2AFA | 0,39 | 0,42 | 0,04 | 0,04 | 30 |
| NM_001911.1 | CTSG | 0,39 | 0,42 | 0,02 | 0,01 | 31 |
| NM_005907.1 | MAN1A1 | 0,38 | 0,28 | 0,01 | 0,05 | 32 |
| NM_003523.1 | H2BFN | 0,37 | 0,32 | 0,04 | 0,05 | 33 |
| NM_015523.1 | DXFZP566E144 | 0,37 | 0,29 | 0,01 | 0,01 | 34 |
| NM_003527.4 | H2BFN | 0,37 | 0,32 | 0,03 | 0,04 | 35 |
| NM_015277.1 | NEDD4L | 0,34 | 0,32 | 0,00 | 0,00 | 36 |
| NM_000250.1 | MPO | 0,33 | 0,30 | 0,01 | 0,02 | 37 |
| NM_015972.1 | LOC51082 | 0,33 | 0,31 | 0,04 | 0,03 | 39 |
| NM_021003.1 | H2BFB | 0,33 | 0,38 | 0,05 | 0,02 | 39 |
| NM_017802.1 | FLJ20397 | 0,32 | 0,33 | 0,03 | 0,04 | 40 |
| NM_003258.1 | TK1 | 0,32 | 0,37 | 0,04 | 0,03 | 41 |
| NM_003514.2 | H2AFN | 0,31 | 0,30 | 0,02 | 0,01 | 43 |
| NM_031894.1 | FTHL17 | 0,29 | 0,33 | 0,04 | 0,03 | 44 |
| AJ296290.1 | PRKWNK1 | 0,29 | 0,32 | 0,01 | 0,01 | 45 |
| NM_016614.1 | AD022 | 0,28 | 0,21 | 0,00 | 0,04 | 47 |
| NM_021064.2 | H2AFP | 0,26 | 0,29 | 0,03 | 0,04 | 48 |
| NM_006563.1 | KLF1 | 0,26 | 0,39 | 0,01 | 0,01 | 49 |
| NM_004617.1 | TM4SF4 | 0,25 | 0,22 | 0,00 | 0,00 | 50 |
| NM_006875.1 | PIM2 | 0,25 | 0,25 | 0,04 | 0,05 | 51 |
| NM_016068.1 | LOC51024 | 0,24 | 0,33 | 0,03 | 0,01 | 52 |
| NM_002466.1 | MYBL2 | 0,24 | 0,34 | 0,04 | 0,01 | 53 |
| NM_021014.1 | SSX3 | 0,24 | 0,41 | 0,00 | 0,00 | 54 |
| NM_003779.2 | B4GALT3 | 0,22 | 0,30 | 0,01 | 0,01 | 55 |
| NM_003511.2 | H2AFI | 0,20 | 0,25 | 0,04 | 0,02 | 56 |
| BC017356.1 | IGHM | 1,81 | 1,53 | 0,00 | 0,01 | 78 |
| AB007960.2 | KIAA0481 | 1,03 | 1,05 | 0,02 | 0,01 | 79 |
| X17263.1 | IGKV1D-12 | 0,96 | 0,94 | 0,04 | 0,04 | 81 |
| U65404.1 | KLF1 | 0,62 | 0,54 | 0,03 | 0,04 | 87 |
| K03195.1 | SLC2A1 | 0,29 | 0,25 | 0,03 | 0,00 | 90 |

**Tabelle 3: Signifikant reduzierte Genaktivitäten in Proben von Patienten mit Sepsis nach [1], dargestellt als deren relatives Verhältnis zu den korrespondierenden Genaktivitäten des selben Patienten im Zustand SIRS nach [1]**

| **GenBank Accession Number** | **HUGO-Name** | **mean: Cy5vsCy3** | **mean: Cy3vsCy5** | **p: Cy5vsCy3** | **p: Cy3vsCyS** | **Seq.-ID** |
|---|---|---|---|---|---|---|
| NM_000576.1 | IL1B | -0,21 | -0,22 | 0,05 | 0,00 | 58 |
| NM_003022.1 | SH3BGRL | -0,26 | -0,31 | 0,01 | 0,00 | 61 |
| NM_000581.1 | GPX1 | -0,26 | -0,32 | 0,01 | 0,00 | 62 |
| NM_016274.1 | LOC51177 | -0,30 | -0,29 | 0,02 | 0,05 | 63 |
| BC013980.1 | BOP1 | -0.30 | -0,23 | 0,01 | 0,04 | 64 |
| X00457.1 | HLA-DPA1 | -0,31 | -0,21 | 0,01 | 0,04 | 65 |
| NM_001671.2 | ASGR1 | -0,38 | -0,41 | 0,03 | 0,03 | 66 |
| NM_000072.1 | CD36 | -0,38 | -0,38 | 0,02 | 0,02 | 67 |
| BC005943.1 | LOC55974 | -0,42 | -0,30 | 0,02 | 0,01 | 68 |
| NM_004331.1 | BNIP3L | -0,44 | -0,35 | 0,01 | 0,01 | 69 |
| NM_002925.2 | RGS10 | -0,49 | -0,40 | 0,00 | 0,00 | 70 |
| NM_002923.1 | RGS2 | -0,55 | -0,67 | 0,03 | 0,02 | 71 |
| J03041.1 | HLA-DPB1 | -0,56 | -0,51 | 0,00 | 0,01 | 72 |
| NM_000239.1 | LYZ | -0,57 | -0,64 | 0,02 | 0,02 | 73 |
| NM_000345.2 | SNCA | -0,65 | -0.61 | 0,03 | 0,02 | 74 |
| NM_000358.1 | TGFBI | -0,75 | -0,66 | 0,01 | 0,02 | 76 |
| NM_000184.1 | HBG2 | -0,94 | -0,84 | 0,03 | 0,05 | 77 |

Diese in Tabelle 2 und 3 charakteristischen Veränderungen sind für das erfindungsgemäße Verfahren gemäß Anspruch 1 ausnutzbar.

Die in den Tabellen 2 und 3 aufgeführten GenBank Accession Nummern (Internet-Zugang über http://www.ncbi.nlm.nih.gov/) der einzelnen Sequenzen sind in dem dieser Anmeldung angefügten 42-seitigen Sequenzprotokoll, das somit Teil der Erfindung ist, im Einzelnen jeweils einer Sequenz ID (Sequenz ID: 1 bis zur Sequenz ID: 91) zugeordnet. Dieses Sequenzprotokoll ist Teil der vorliegenden Erfindung.

### Referenzen

1. Bone RC, Balk RA, Cerra FB, Dellinger EP, Fein AM, Knaus WA, Schein RM, Sibbald WJ, the ACCP/SCCM Consensus Conference Committee (1992) Definitions for Sepsis and organ failure and guidelines for the use of innovative therapies in Sepsis. Chest 101,1656-1662; und Crit Care Med 1992; 20: 864-874.
2. Marshall JC, Vincent JL, Fink MP, Cook DJ, Rubenfeld G, Foster D, Fisher CJ Jr, Faist E, Reinhart K (2003) Measures, markers, and mediators: toward a staging system for clinical Sepsis. A report of the Fifth Toronto Sepsis Roundtable, Toronto, Ontario, Canada, October 25-26, 2000. Crit Care Med. 31:1560-7.
3. Alberti C, Brun-Buisson C, Goodman SV, Guidici D, Granton J, Moreno R, Smithies M, Thomas O Artigas A, Le Gall JR; European Sepsis Group (2003) Influence of systemic inflammatory response syndrome and Sepsis on outcome of critically ill infected patients. Am J Respir Crit Care Med. 168:77-84.
4. Brun-Buisson C, Doyon F, Carlet J, Dellamonica P, Gouin F, Lepoutre A, Mercier JC, Offenstadt G, Regnier B: Incidence, risk factors, and outcome of severe Sepsis and septic shock in adults. A multicenter prospective study in intensive care units. French ICU Group for Severe Sepsis. JAMA 1995; 274: 968-974
5. Le-Gall JR, Lemeshow S, Leleu G, Klar J, Huillard J, Rue M, Teres D, Artigas A: Customized probability models for early severe Sepsis in adult intensive care patients. Intensive Care Unit Scoring Group. JAMA 1995; 273: 644-650
6. Brun-Buisson C, Roudot-Thoraval F, Girou E, Grenier-Sennelier C, Durand-Zaleski I. (2003) The costs of septic syndromes in the intensive care unit and influence of hospital-acquired Sepsis. Intensive Care Med. [Epub ahead of print]
7. Increase in National Hospital Discharge Survey rates for septicemia--United States, 1979-1987. MMWR Morb Mortal Wkly Rep 1990; 39: 31-34
8. Bone, R. C. Sepsis, the sepsis syndrome, multi-organ failure: a plea for comparable definitions. Ann Intern Med 1991; 114: 332-333
9. Matot, I., C. L. Sprung, et al. Definition of sepsis. Intensive Care Med 2001; 27 (suppl): S3-S9.
10. Friedland, J. S., J. C. Porter, et al. Plasma proinflammatory cytokine concentrations, Acute Physiology and Chronic Health Evaluation (APACHE) III scores and survival in patients in an intensive care unit. Crit Care Med 1996; 24: 1775-1781..
11.Beutler, B., A. Poltorak, et al. Sepsis and evolution of the innate immune response. Crit Care Med 2001; 29: S2-S6.
12.Vincent JL, Angus D, Annane D, et al. (2001) Clinical expert round table discussion (session 5) at the Margaux Conference on Critical IIIness: outcomes of clinical trials in Sepsis: lessons learned. Crit Care Med 29:S136-137.
13.Abraham, E., Laterre P. F., et al. Lenercept (p55 tumor necrosis factor receptor fusion protein) in severe sepsis and early septic shock: a randomized, double-blind, placebo-controlled, multicenter phase III trial with 1,342 patients. Crit Care Med 2001; 29: 503-510
14.Abraham, E., Reinhart K., et al. Assessment of the safety of recombinant tissue factor pathway inhibitor in patients with severe sepsis: a multicenter, randomized, placebo-controlled, single-blind, dose escalation study. Crit Care Med 2001; 29: 2081-2089
15. Pittet, D., Harbarth S., et al. Impact of immunomodulating therapy on morbidity in patients with severe sepsis. Am J Respir Crit Care Med 1999; 160: 852-857
16.Abraham, E., Marshall J. C., et al. Sepsis and mediator-directed therapy: rethinking the target populations. Mediator-directed therapy in sepsis: rethinking the target populations. Toronto, Canada, 31 October-1 November 1998. Mol Med Today 1999; 5: 56-58.40-43
17.Abraham, E., Raffin T. A. Sepsis therapy trials. Continued disappointment or reason for hope? JAMA 1994; 271: 1876-1878.
18. Zeni F., Freeman B., et al. Anti-inflammatory therapies to treat sepsis and septic shock: a reassessment. Crit Care Med 1997; 25: 1095-1100
19.Bone, R. C. The pathogenesis of sepsis. Ann Intern Med 1991; 115: 457-469
20. Marshall JC (2000) SIRS and MODS: What is there relevance to the science and practise of intensive care?, Shock 14:586-589
21.Vincent J-L (1997) Dear SIRS, l'm sorry to say that I don't like you. Crit Car Med 25:372-374
22.Ramsay G, Gerlach H, Levy MM et al (2003) An international sepsis survey: As tudy of doctor's knowledge and perception about sepsis. Crit Care Med 31
23.Levy MM, Fink MP, Marshall JC et al. (2003) 2001 SCCM/ESICM/ACCP/ATS/SIS International Sepsis Definitions Conference. Cri Car Med Vol 31, No 4
24. http://www.krebsinformation.de/tnm-system.html (Stand 1. März 2004)
25.Straube E (2003) Sepsis - microbiological diagnosis. Infection 31:284
26.Rußwurm S. (2002) Procalcitonin als Marker bakterieller Infektionen und Sepsis: Einfluss sepsisrelevanter Bedingungen auf die Expression von Procalcitonin, Habilitationsschrift eingereicht bei der Medizinischen Fakultät der Friedrich-Schiller-Universität Jena
27. Southern EM (1974) An improved method for transferring nucleotides from electrophoresis strips to thin layers of ion-exchange cellulose. Anal Biochem 62:317-318
28.Gillespie D, Spiegelman S (1965) A quantitative assay for DNA-RNA hybrids with DNA immobilized on a membrane. J Mol Biol 12:829-842
29.Lennon GG, Lehrach H (1991) Hybridization analyses of arrayed cDNA libraries. Trends Genet 7: 314-317
30. Kafatos FC, Jones CW, Efstratiadis A (1979) Determination of nucleic acid sequence homologies and relative concentrations by a dot hybridization procedure. Nucl Acid Res 7:1541-1552
31.Fodor SP, Read JL, Pirrung MC, Stryer L, Lu AT, Solas D (1991) Lightdirected, spatially addressable parallel chemical synthesis. Science 251:767-773
32. Pease AC, Solas D, Sullivan EJ, Cronin MT, Holmes CP, Fodor SP (1994) Light-generated oligonucleotide arrays for rapid DNA sequence analysis. Proc Natl Acad Sci USA 91:5022-5026
33.Schena M, Shalon D, Davis RW, Brown PO (1995) Quantitative monitoring of gene expression patterns with a complementary DNA microarray. Science 270:467-470
34. Golub TR, Slonim DK, Tamayo P, et al. (1999) Molecular classification of cancer: class discovery and class prediction by gene expression monitoring. Science 286:531-537
35.Alizadeh AA, Eisen MB, Davis RE, et al. (2000) Distinct types of diffuse large B-cell lymphoma identified by gene expression profiling. Nature 403:503-511
36. Henry GL, Zito K, Dubnau J, (2003) Chipping away at brain function: mining for insights with microarrays. Current Opinion in Neurobiology, 13:570-576
37. Fillion I, Ouellet N, Simard M, et al.(2002) Role of chemokines and formyl peptides in pneumococcal pneumonia-induced monocyte/macrophage recruitment. J Immunol.;166(12):7353-61.
38.Zhao B, Bowden RA, Stavchansky SA, Bowman PD (2001) Human endothelial cell response to gram-negative lipopolysaccharide assessed with cDNA microarrays. Am J Physiol Cell Physiol. Nov;281(5):C1587-95.
39. Chinnaiyan AM, Huber-Lang M, Kumar-Sinha C et al. (2001) Molecular signatures of Sepsis: multiorgan gene expression profiles of systemic inflammation. Am J Pathol. 159(4):1199-209.
40. Cobb JP, Laramie JM, Stormo GD et al. (2002) Sepsis gene expression profiling: Murine splenic compared with hepatic response determined by using complementary DNA microarrays. Crit Care Med Vol. 30, No.12, 2711-2721
41. Pathan N, Hemingway CA, Alizadeh AA, et al. (2004) Role of interleukine 6 in myocardial dysfunction of meningococcal septic shock. The Lancet Vol. 363 Nr. 9404: 203-209
42. Eiling K, Kotsch K, Strohmeyer J-C et al. (2003) Identification of differentially expressed genes during systemic inflammatory response syndrome using cDNA microarrays. Infection 31:301
43. Pathan N, Hemingway C, Levin M et al. (2000) The complexity of the inflammatory response to meningococcal sepsis revealed by gene expression profiling using cDNA microarrays. Critical Care Medicine, 30(12):A47
44.Wiegand G, Selleng K, Gründling M et al. (1999) Gene expression pattern in human monocytes as a surrogate marker for systemic inflammatory response syndrome (SIRS). Mol. Med. 5:192-202
45. Huber W, Heydebreck A, Sueltmann H, et al. (2003) Parameter estimation for the calibration and variance stabilization of microarray data. Stat. Appl. in Gen. and Mol. Biol.. Volume 2: No 1, Article 3

## Patentansprüche

1. Verfahren zur *in vitro* Unterscheidung von generalisierten, inflammatorischen, nichtinfektiösen Zuständen und generalisierten, inflammatorischen, infektiösen Zuständen,
**dadurch gekennzeichnet, daß**
es folgende Schritte umfasst:
a) Isolieren von Proben-RNA aus einer biologischen Probe;
b) Markieren der Proben-RNA und/oder wenigstens einer DNA, die eine zur Unterscheidung zwischen SIRS und Sepsis spezifische Genaktivität repräsentiert und/oder ein spezifisches Gen oder Genfragment ist, mit einem detektierbaren Marker;
c) In-Kontakt-Bringen der Proben-RNA mit der DNA unter Hybridisierungsbedingungen;
d) In-Kontakt-Bringen von Kontroll-RNA, mit wenigstens einer DNA, unter Hybridisierungsbedingungen, wobei die DNA ein zur Unterscheidung von zwischen SIRS und Sepsis spezifisches Gen oder Genfragmente ist; (
e) quantitatives Erfassen der Markierungssignale der hybridisierten Proben-RNA und der Kontroll-RNA;
f) Vergleichen der quantitativen Daten der Markierungssignale, um eine Aussage zu treffen, ob zur Unterscheidung zwischen SIRS und Sepsis spezifische Gene oder Genfragmente in der Probe stärker oder schwächer exprimiert sind als in der Kontrolle; wobei
g) das zur Unterscheidung zwischen SIRS und Sepsis spezifische Gen und/oder Genfragment ausgewählt wird aus der Gruppe bestehend aus SEQ-ID Nos. 1-7, 9-10, 13, 15, 17-19, 21-24, 27-41, 43-45, 47-56, 58, 61-74, 76-79, 81, 87, 90.

2. Verfahren nach Anspruch 1, **dadurch gekennzeichnet, dass** man die Kontroll-RNA vor dem Messen der Proben-RNA mit der DNA hybridisiert und die Markierungssignale des Kontroll-RNA/DNA-Komplexes erfasst und gegebenenfalls in Form einer Kalibrierkurve oder -tabelle ablegt.

3. Verfahren nach Anspruch 2, **dadurch gekennzeichnet, daß** nicht veränderte Gene aus der Proben- und/oder Kontroll-RNA als Bezugsgene für die Quantifizierung genutzt werden.

4. Verfahren nach Anspruch 1 bis 3, **dadurch gekennzeichnet, daß** als Proben-RNA mRNA verwendet wird.

5. Verfahren nach Anspruch 1 bis 4, **dadurch gekennzeichnet, daß** die DNA an vorbestimmten Bereichen auf einem Träger in Form eines Microarrays angeordnet, insbesondere immobilisiert, wird.

6. Verfahren nach Anspruch 1 bis 5, **dadurch gekennzeichnet, daß** das Verfahren zur differentialdiagnostischen Früherkennung, zur Kontrolle des klinischen Verlaufs, zur individuellen Risikoabschätzung für Patienten, zur Abschätzung des wahrscheinlichen Ansprechens auf eine spezifische Behandlung sowie zur post mortem Diagnose zur Unterscheidung von SIRS und Sepsis eingesetzt wird.

7. Verfahren nach Anspruch 1 bis 6, **dadurch gekennzeichnet, daß** die Probe ausgewählt wird aus: Körperflüssigkeiten, insbesondere Blut, Liquor, Urin, Ascitesflüssigkeit, Seminalflüssigkeit, Speichel, Punktat; Zellinhalt oder eine Mischung davon.

8. Verfahren nach einem der Ansprüche 1 bis 7, **dadurch gekennzeichnet, daß** Zellproben gegebenenfalls einer lytischen Behandlung unterzogen werden, um deren Zellinhalte freizusetzen.

9. Verfahren nach einem der Ansprüche 1 bis 8, **dadurch gekennzeichnet, daß** es sich bei der biologischen Probe um die eines Menschen handelt.

10. Verfahren nach einem der Ansprüche 1 bis 9 **dadurch gekennzeichnet, daß,** Genfragmente der SEQ-ID Nos. 1-7, 9-10, 13, 15, 17-19, 21-24, 27-41, 43-45, 47-56, 58, 61-74, 76-79, 81, 87, 90 wenigstens 5-2000, bevorzugt 20-200, mehr bevorzugt 20-80 Nukleotide aufweisen.

11. Verfahren nach einem der Ansprüche 1 bis 10 **dadurch gekennzeichnet, daß** wenigstens 2 bis 100 unterschiedliche cDNAs verwendet werden.

12. Verfahren nach einem der Ansprüche 1 bis 11 **dadurch gekennzeichnet, daß** wenigstens 200 unterschiedliche cDNAs verwendet werden.

13. Verfahren nach einem der Ansprüche 1 bis 12 **dadurch gekennzeichnet, daß** wenigstens 200 bis 500 unterschiedliche cDNAs verwendet werden.

14. Verfahren nach einem der Ansprüche 1 bis 13 **dadurch gekennzeichnet, daß** wenigstens 500 bis 1000 unterschiedliche cDNAs verwendet werden.

15. Verfahren nach einem der Ansprüche 1 bis 14 **dadurch gekennzeichnet, daß** wenigstens 1000 bis 2000 unterschiedliche cDNAs verwendet werden.

16. Verfahren nach einem der Ansprüche 1 bis 15, **dadurch gekennzeichnet, daß** als detektierbarer Marker ein radioaktiver Marker, insbesondere ³²P, ¹⁴C, ¹²⁵I ¹⁵⁵Eu, ³³P oder ³H verwendet wird.

17. Verfahren nach einem der Ansprüche 1 bis 16, **dadurch gekennzeichnet, daß** als detektierbarer Marker ein nicht radioaktiver Marker, insbesondere ein Farb- oder Fluoreszenzmarker, ein Enzymmarker oder Immunmarker, und/oder quantum dots oder ein elektrisch messbares Signal, insbesondere Potential- und/oder Leitfähigkeits- und/oder Kapazitätsänderung bei Hybridisierungen, verwendet wird.

18. Verfahren nach einem der Ansprüche 1 bis 17, **dadurch gekennzeichnet, daß** die Proben-RNA und Kontroll-RNA dieselbe Markierung tragen.

19. Verfahren nach einem der Ansprüche 1 bis 18, **dadurch gekennzeichnet, daß** die Proben-RNA und Kontroll-RNA unterschiedliche Markierungen tragen.

20. Verfahren nach einem der Ansprüche 1-19, **dadurch gekennzeichnet, dass** die immobilisierten oder nichtimmobilisierten DNA-Sonden eine Markierung tragen.

21. Verfahren nach einem der Ansprüche 1 bis 20 **dadurch gekennzeichnet, daß** die DNA-Sonden auf Glas oder Kunststoff, immobilisiert werden.

22. Verfahren nach einem der Ansprüche 1 bis 21, **dadurch gekennzeichnet, daß** die einzelnen DNA Moleküle über eine kovalente Bindung an das Trägermaterial immobilisiert werden.

23. Verfahren nach einem der Ansprüche 1 bis 22, **dadurch gekennzeichnet, daß** die einzelnen DNA Moleküle mittels elektrostatischer- und/oder Dipol-Dipol- und/oder hydrophobe Wechselwirkungen und/oder Wasserstolfbrücken an das Trägermaterial immobilisiert werden.

## Claims

1. A method for the *in vitro* differentiation between systemic inflammatory non-infectious conditions and systemic inflammatory infectious conditions, thereby **characterized**, that it includes the following steps:
a) isolation of sample RNA from a biological sample;
b) marking the sample RNA and/or at least one DNA, which represents a gene activity that is specific for distinguishing between SIRS and sepsis and/or is a specific gene or gene fragment, with a detectable marker;
c) bringing the sample RNA in contact with the DNA under hybridization conditions;
d) bringing control RNA in contact with at least one DNA, under hybridization conditions, said DNA representing a gene or gene fragment that is specific for distinguishing between SIRS and sepsis;
e) quantitatively measuring the marking signals of the hybridized sample RNA and control RNA; and
f) comparing the quantitative data of the marking signals in order to make a determination as to whether genes or gene fragments that are specific for distinguishing between SIRS and sepsis are expressed more prominently or less prominently in the sample than in the control; wherein
g) the gene and/or gene fragment specific for the differentiation between SIRS and sepsis is selected from the group consisting of SEQ ID NO: 1-7, 9-10, 13, 15, 17-19, 21-24, 27-41, 43-45, 47-56, 58, 61-74, 76-79, 81, 87, 90.

2. A method according the claim 1, thereby **characterized**, that prior to measurement of the sample RNA the control RNA is hybridized with the DNA and the marker signal of the control RNA/DNA complex is determined and optionally recorded in the form of a calibration curve or table.

3. A method according to claim 2, thereby **characterized**, that unchanged genes form the sample and/or control RNA are used as reference genes for quantification.

4. A method according to claims 1 through 3, thereby **characterized**, that mRNA is used as sample RNA.

5. A method according to claims 1 through 4, thereby **characterized**, that the DNA is provided in predetermined locations, in particular immobilized, on a carrier in the form of a microarray.

6. A method according to claims 1 through 5, thereby **characterized**, that the method is employed for differential diagnostic early recognition, for control of the clinical course, for individual risk assessment for patients, for evaluation of the probability of response to a specific treatment as well as for post-mortem diagnosis for distinguishing between SIRS and sepsis.

7. A method according to claims 1 through 6, thereby **characterized**, that the sample is selected from: body fluids, in particular blood, serum, urine, peritoneal fluid, seminal fluid, saliva, tissue fluids; cell contents or a mixture thereof.

8. A method according to any one of claims 1 through 7, thereby **characterized**, that cell samples are optionally subjected to lysis treatment in order to release cellular contents.

9. A method according to any one of claims 1 through 8, thereby **characterized**, that the biological sample is of human origin.

10. A method according to any one of claims 1 through 9, thereby **characterized**, that the gene fragments of the genes of SEQ ID NO: 1-7, 9-10, 13, 15, 17-19, 21-24, 27-41, 43-45, 47-56, 58, 61-74, 76-79, 81, 87, 90 have at least 5-2000, preferably 20-200, more preferably 20-80 nucleotides.

11. A method according to any one of claims 1 through 10, thereby **characterized**, that at least 2-100 different cDNAs are employed.

12. A method according to any one of claims 1 through 11, thereby **characterized**, that at least 200 different cDNAs are employed.

13. A method according to any one of claims 1 through 12, thereby **characterized**, that at least 200 to 500 different cDNAs are employed.

14. A method according to any one of claims 1 through 13, thereby **characterized**, that at least 500 to 1000 different cDNAs are employed.

15. A method according to any one of claims 1 through 14, thereby **characterized**, that at least 1000 to 2000 cDNAs are employed.

16. A method according to any one of claims 1 through 15, thereby **characterized**, that a radioactive marker, in particular ³²P, ¹⁴C, ¹²⁵I, ¹⁵⁵Eu or ³H, is employed as detectable marker.

17. A method according to any one of claims 1 through 16, thereby **characterized**, that as detectable markers a non-radioactive marker, in particular a colour or fluorescence marker, an enzyme marker or an immune marker and/or quantum dots or an electrically measurable signal, in particular potential and/or conductivity and/or capacity change upon hybridization, is employed.

18. A method according to any one of claims 1 through 17, thereby **characterized**, that sample RNA and the control RNA carry the same markers.

19. A method according to any one of claims 1 through 18, thereby **characterized**, that the sample RNA and the control RNA carry different markers.

20. A method according to any one of claims 1 trough 19, thereby **characterized**, that the immobilized or non-immobilized DNA probes carry a marker.

21. A method according to any one of claims 1 trough 20, thereby **characterized**, that the DNA probes are immobilized on glass or plastic.

22. A method according to any one of claims 1 to 21, thereby **characterized**, that the individual DNA molecules are immobilized by a covalent bonding to the carrier material.

23. A method according to any one of claims 1 to 22, thereby **characterized**, that the individual DNA molecules are immobilized by electrostatic and/or dipol-dipol and/or hydrophobic interaction and/or hydrogen bridges to the carrier materials.

## Revendications

1. Procédé de distinction *in vitro* entre des états généraux inflammatoires non infectieux et des états généraux inflammatoires infectieux,
**caractérisé en ce**
**qu'**il comprend les étapes suivantes :
a) isolation d'ARN échantillon d'un échantillon biologique ;
b) marquage des ARN échantillons et/ou d'au moins un ADN qui représente pour la distinction entre SRIS et septicémie une activité génique spécifique et/ou qui est un gène ou un fragment de gène spécifique, avec un marqueur détectable ;
c) mise en contact des échantillons d'ARN avec l'ADN sous des conditions d'hybridation ;
d) mise en contact d'ARN de contrôle avec au moins un ADN sous des conditions d'hybridation, l'ADN étant un gène ou un fragment de gène spécifique pour la distinction entre SRIS et septicémie ;
e) saisie quantitative des signaux de marquage de l'ARN échantillon et de l'ARN de contrôle hybridé ;
f) comparaison des données quantitatives des signaux de marquage pour en tirer une conclusion, à savoir si des gènes ou des fragments de gène spécifiques pour une distinction entre SRIS et septicémie sont plus fortement ou plus faiblement exprimés dans l'échantillon que dans le contrôle ; sachant que
g) le gène et/ou le fragment de gène spécifique pour une distinction entre SRIS et septicémie est choisi parmi le groupe consistant en les SEQ ID Nos 1-7, 9-10, 13, 15, 17-19, 21-24, 27-41, 43-45, 47-56, 58, 61-74, 76-79, 81, 87, 90.

2. Procédé selon la revendication 1, **caractérisé en ce que** l'on hybride l'ARN de contrôle avec l'ADN avant la mesure de l'ARN échantillon, et que l'on appréhende les signaux de marquage du complexe ARN de contrôle/ADN et qu'on les enregistre le cas échéant sous forme d'une courbe ou d'un tableau de calibrage.

3. Procédé selon la revendication 2, **caractérisé en ce que** les gènes non modifiés provenant de l'ARN échantillon et/ou de l'ARN de contrôle sont utilisés comme gènes de référence pour la quantification

4. Procédé selon les revendications 1 à 3, **caractérisé en ce que** comme ARN échantillon, on utilise de l'ARN-m.

5. Procédé selon les revendications 1 à 4, **caractérisé en ce que** l'ADN est disposé, en particulier immobilisé, en des zones prédéfinies sur un support de la forme d'un microtableau.

6. Procédé selon les revendications 1 à 5, **caractérisé en ce que** le procédé est utilisé à la distinction entre SRIS et septicémie pour le dépistage diagnostique différentiel précoce, pour le contrôle de l'évolution clinique, pour l'appréciation individuelle du risque des patients, pour l'estimation de la réaction probable à un traitement spécifique ainsi que pour le diagnostic post mortem.

7. Procédé selon les revendications 1 à 6, **caractérisé en ce que** l'échantillon est choisi parmi : des liquides corporels, en particulier le sang, le liquide cérébro-spinal, l'urine, le liquide intra-abdominal, le liquide séminal, la salive, un extrait de ponction ; un contenu de cellule ou un de leurs mélanges.

8. Procédé selon l'une quelconque des revendications 1 à 7, **caractérisé en ce que** des échantillons de cellules sont soumis le cas échéant à traitement de lyse pour libérer leurs contenus de cellule.

9. Procédé selon l'une quelconque des revendications 1 à 8, **caractérisé en ce que**, concernant l'échantillon biologique, il s'agit de celui d'une personne humaine.

10. Procédé selon l'une quelconque des revendications 1 à 9, **caractérisé en ce que** les fragments de gène des SEQ ID NOs 1-7, 9-10, 13, 15, 17-19, 21-24, 27-41, 43-45, 47-56, 58, 61-74, 76-79, 81, 87, 90 comportent au moins 5 à 2000, de préférence 20 à 200, plus préférablement 20 à 80 nucléotides.

11. Procédé selon l'une quelconque des revendications 1 à 10, **caractérisé en ce qu'**au moins 2 à 100 ADNc différents sont utilisés.

12. Procédé selon l'une quelconque des revendications 1 à 11, **caractérisé en ce qu'**au moins 200 ADNc différents sont utilisés.

13. Procédé selon l'une quelconque des revendications 1 à 12, **caractérisé en ce qu'**au moins 200 à 500 ADNc différents sont utilisés.

14. Procédé selon l'une quelconque des revendications 1 à 13, **caractérisé en ce qu'**au moins 500 à 1000 ADNc différents sont utilisés.

15. Procédé selon l'une quelconque des revendications 1 à 14, **caractérisé en ce qu'**au moins 1000 à 2000 ADNc différents sont utilisés.

16. Procédé selon l'une quelconque des revendications 1 à 15, **caractérisé en ce que** comme marqueur détectable, on utilise un marqueur radioactif, en particulier du ³²P, du ¹⁴C, du ¹²⁵I, du ¹⁵⁵Eu, du ³³P ou du 3H.

17. Procédé selon l'une quelconque des revendications 1 à 16, **caractérisé en ce que** comme marqueur détectable, on utilise un marqueur non radioactif, en particulier un marqueur coloré ou fluorescent, un marqueur enzymatique ou un marqueur immunologique et/ou des points quantiques ou un signal mesurable électriquement, en particulier un changement de potentiel et/ou de conductivité et/ou de capacité lors de l'hybridation.

18. Procédé selon l'une quelconque des revendications 1 à 17, **caractérisé en ce que** l'ARN échantillon et l'ARN de contrôle portent le même marquage.

19. Procédé selon l'une quelconque des revendications 1 à 18, **caractérisé en ce que** l'ARN échantillon et l'ARN de contrôle portent des marquages différents.

20. Procédé selon l'une quelconque des revendications 1 à 19, **caractérisé en ce que** les sondes d'ADN immobilisées ou non immobilisées portent un marquage.

21. Procédé selon l'une quelconque des revendications 1 à 20, **caractérisé en ce que** les sondes d'ADN sont immobilisées sur du verre ou du plastique.

22. Procédé selon l'une quelconque des revendications 1 à 21, **caractérisé en ce que** les molécules d'ADN individuelles sont immobilisées sur le matériau support par une liaison covalente.

23. Procédé selon l'une quelconque des revendications 1 à 22, **caractérisé en ce que** les molécules d'ADN individuelles sont immobilisées sur le matériau support par des interactions électrostatiques et/ou dipôle-dipôle et/ou hydrophobes et/ou par des ponts hydrogène.


### SEQUENZPROTOKOLL

<110> SIRS-Lab GmbH
<120> Verfahren zur Erkennung von Sepsis
<130> SL0511
<140>
   <141> 15. Dezember 2004
<160> 91
<170> PatentIn version 3.1
<210> 1
   <211> 2713
   <212> DNA
   <213> Homo sapiens
<400> 1
   ggcacgagga gagtgcggct gctgagagcc gagcccagca atcccgatcc tctgagtcgt 60
   gaagaaggga ggcagcgagg gggttggggt tggggcctga ggcaagcccc caggctccgc 120
   tcttgccaga gggacaggag ccatggctca gaaaatggac tgtggtgcgg gcctcctcgg 180
   cttccaggct gaggcctccg tagaagacag cgccttgctt atgcagacct tgatggaggc 240
   catccagatc tcagaggctc cacctactaa ccaggccacc gcagctgcta gtccccagag 300
   ttcacagccc ccaactgcca atgagatggc tgacattcag gtttcagcag ctgccgctag 360
   gcctaagtca gcctttaaag tccagaatgc caccacaaaa ggcccaaatg gtgtctatga 420
   tttctctcag gctcataatg ccaaggatgt gcccaacacg cagcccaagg cagcctttaa 480
   gtcccaaaat gctacctcca aaggtccaaa tgctgcctat gatttttccc aggcagcaac 540
   cactggtgag ttagctgcta acaagtctga gatggccttc aaggcccaga atgccactac 600
   taaagtgggc ccaaatgcca cctacaattt ctctcagtct ctcaatgcca atgacctggc 660
   caacagcagg cctaagaccc ctttcaaggc ttggaatgat accactaagg ccccaacagc 720
   tgatacccag acccagaatg taaatcaggc caaaatggcc acttcccagg ctgacataga 780
   gaccgaccca ggtatctctg aacctgacgg tgcaactgca cagacatcag cagatggttc 840
   ccaggctcag aatctggagt cccggacaat aattcggggc aagaggaccc gcaagattaa 900
   taacttgaat gttgaagaga acagcagtgg ggatcagagg cgggccccac tggctgcagg 960
   gacctggagg tctgcaccag ttccagtgac cactcagaac ccacctggcg caccccccaa 1020
   tgtgctctgg cagacgccat tggcttggca gaacccctca ggctggcaaa accagacagc 1080
   caggcagacc ccaccagcac gtcagagccc tccagctagg cagaccccac cagcctggca 1140
   gaacccagtc gcttggcaga acccagtgat ttggccaaac ccagtaatct ggcagaaccc 1200
   agtgatctgg ccaaacccca ttgtctggcc cggccctgtt gtctggccga atccactggc 1260
   ctggcagaat ccacctggat ggcagactcc acctggatgg cagaccccac cgggctggca 1320
   gggtcctcca gactggcaag gtcctcctga ctggccgcta ccacccgact ggccactgcc 1380
   acctgattgg ccacttccca ctgactggcc actaccacct gactggatcc ccgctgattg 1440
   gccaattcca cctgactggc agaacctgcg cccctcgcct aacctgcgcc cttctcccaa 1500
   ctcgcgtgcc tcacagaacc caggtgctgc acagccccga gatgtggccc ttcttcagga 1560
   aagagcaaat aagttggtca agtacttgat gcttaaggac tacacaaagg tgcccatcaa 1620
   gcgctcagaa atgctgagag atatcatccg tgaatacact gatgtttatc cagaaatcat 1680
   tgaacgtgca tgctttgtcc tagagaagaa atttgggatt caactgaaag aaattgacaa 1740
   agaagaacac ctgtatattc tcatcagtac ccccgagtcc ctggctggca tactgggaac 1800
   gaccaaagac acacccaagc tcggtctcct cttggtgatt ctgggtgtca tcttcatgaa 1860
   tggcaaccgt gccagtgagg ctgtcctctg ggaggcacta cgcaagatgg gactgcgtcc 1920
   tggggtgaga catcccctcc ttggagatct aaggaaactt ctcacctatg agtttgtaaa 1980
   gcagaaatac ctggactaca gacgagtgcc caacagcaac cccccggagt atgagttcct 2040
   ctggggcctc cgttcctacc atgagactag caagatgaaa gtgctgagat tcattgcaga 2100
   ggttcagaaa agagaccctc gtgactggac tgcacagttc atggaggctg cagatgaggc 2160
   cttggatgct ctggatgctg ctgcagctga ggccgaagcc cgggctgaag caagaacccg 2220
   catgggaatt ggagatgagg ctgtgtctgg gccctggagc tgggatgaca ttgagtttga 2280
   gctgctgacc tgggatgagg aaggagattt tggagatccc tggtccagaa ttccatttac 2340
   cttctgggcc agataccacc agaatgcccg ctccagattc cctcagacct ttgccggtcc 2400
   cattattggt cctggtggta cagccagtgc caacttcgct gccaactttg gtgccattgg 2460
   tttcttctgg gttgagtgag atgttggata ttgctatcaa tcgcagtagt ctttcccctg 2520
   tgtgagctga agcctcagat tccttctaaa cacagctatc tagagagcca catcctgttg 2580
   actgaaagtg gcatgcaaga taaatttatt tgctgttcct tgtctactgc tttttttccc 2640
   cttgtgtgct gtcaagtttt ggtatcagaa ataaacattg aaattgcaaa gtgaaaaaaa 2700
   aaaaaaaaaa aaa 2713
<210> 2
   <211> 642
   <212> DNA
   <213> Homo sapiens
<400> 2
   atgtccgaga ctgctcctgc cgctcccgct gccgcgcctc ctgcggagaa ggcccctgta 60
   aagaagaagg cggccaaaaa ggctgggggt acgcctcgta aggcgtccgg tcccccggtg 120
   tcagagctca tcaccaaggc tgtggccgcc tctaaagagc gtagcggagt ttctctggct 180
   gctctgaaaa aagcgttggc tgccgccggc tatgatgtgg agaaaaacaa cagccgtatc 240
   aaacttggtc tcaagagcct ggtgagcaag ggcactctgg tgcaaacgaa aggcaccggt 300
   gcttctggct cctttaaact caacaagaag gcagcctccg gggaagccaa gcccaaggtt 360
   aaaaaggcgg gcggaaccaa acctaagaag ccagttgggg cagccaagaa gcccaagaag 420
   gcggctggcg gcgcaactcc gaagaagagc gctaagaaaa caccgaagaa agcgaagaag 480
   ccggccgcgg ccactgtaac caagaaagtg gctaagagcc caaagaaggc caaggttgcg 540
   aagcccaaga aagctgccaa aagtgctgct aaggctgtga agcccaaggc cgctaagccc 600
   aaggttgtca agcctaagaa ggcggcgccc aagaagaaat ag 642
<210> 3
   <211> 542
   <212> DNA
   <213> Homo sapiens
<400> 3
   gtctgccctc tctgctcgcc ctgcctagct tgaggatctg tcaccccagc catgaggatt 60
   atcgccctcc tcgctgctat tctcttggta gccctccagg tccgggcagg cccactccag 120
   gcaagaggtg atgaggctcc aggccaggag cagcgtgggc cagaagacca ggacatatct 180
   atttcctttg catgggataa aagctctgct cttcaggttt caggctcaac aaggggcatg 240
   gtctgctctt gcagattagt attctgccgg cgaacagaac ttcgtgttgg gaactgcctc 300
   attggtggtg tgagtttcac atactgctgc acgcgtgtcg attaacgttc tgctgtccaa 360
   gagaatgtca tgctgggaac gccatcatcg gtggtgttag cttcacatgc ttctgcagct 420
   gagcttgcag aatagagaaa aatgagctca taatttgctt tgagagctac aggaaatggt 480
   tgtttctcct atactttgtc cttaacatct ttcttgatcc taaatatata tctcgtaaca 540
   ag 542
<210> 4
   <211> 2856
   <212> DNA
   <213> Homo sapiens
<400> 4
   tagtcgcggg tccccgagtg agcacgccag ggagcaggag accaaacgac gggggtcgga      60
   gtcagagtcg cagtgggagt ccccggaccg gagcacgagc ctgagcggga gagcgccgct     120
   cgcacgcccg tcgccacccg cgtacccggc gcagccagag ccaccagcgc agcgctgcca     180
   tggagcccag cagcaagaag ctgacgggtc gcctcatgct ggctgtggga ggagcagtgc     240
   ttggctccct gcagtttggc tacaacactg gagtcatcaa tgccccccag aaggtgatcg     300
   aggagttcta caaccagaca tgggtccacc gctatgggga gagcatcctg cccaccacgc     360
   tcaccacgct ctggtccctc tcagtggcca tcttttctgt tgggggcatg attggctcct     420
   tctctgtggg ccttttcgtt aaccgctttg gccggcggaa ttcaatgctg atgatgaacc 480
   tgctggcctt cgtgtccgcc gtgctcatgg gcttctcgaa actgggcaag tcctttgaga 540
   tgctgatcct gggccgcttc atcatcggtg tgtactgcgg cctgaccaca ggcttcgtgc 600
   ccatgtatgt gggtgaagtg tcacccacag cctttcgtgg ggccctgggc accctgcacc 660
   agctgggcat cgtcgtcggc atcctcatcg cccaggtgtt cggcctggac tccatcatgg 720
   gcaacaagga cctgtggccc ctgctgctga gcatcatctt catcccggcc ctgctgcagt 780
   gcatcgtgct gcccttctgc cccgagagtc cccgcttcct gctcatcaac cgcaacgagg 840
   agaaccgggc caagagtgtg ctaaagaagc tgcgcgggac agctgacgtg acccatgacc 900
   tgcaggagat gaaggaagag agtcggcaga tgatgcggga gaagaaggtc accatcctgg 960
   agctgttccg ctcccccgcc taccgccagc ccatcctcat cgctgtggtg ctgcagctgt 1020
   cccagcagct gtctggcatc aacgctgtct tctattactc cacgagcatc ttcgagaagg 1080
   cgggggtgca gcagcctgtg tatgccacca ttggctccgg tatcgtcaac acggccttca 1140
   ctgtcgtgtc gctgtttgtg gtggagcgag caggccggcg gaccctgcac ctcataggcc 1200
   tcgctggcat ggcgggttgt gccatactca tgaccatcgc gctagcactg ctggagcagc 1260
   taccctggat gtcctatctg agcatcgtgg ccatctttgg ctttgtggcc ttctttgaag 1320
   tgggtcctgg ccccatccca tggttcatcg tggctgaact cttcagccag ggtccacgtc 1380
   cagctgccat tgccgttgca ggcttctcca actggacctc aaatttcatt gtgggcatgt 1440
   gcttccagta tgtggagcaa ctgtgtggtc cctacgtctt catcatcttc actgtgctcc 1500
   tggttctgtt cttcatcttc acctacttca aagttcctga gactaaaggc cggaccttcg 1560
   atgagatcgc ttccggcttc cggcaggggg gagccagcca aagtgataag acacccgagg 1620
   agctgttcca tcccctgggg gctgattccc aagtgtgagt cgccccagat caccagcccg 1680
   gcctgctccc agcagcccta aggatctctc aggagcacag gcagctggat gagacttcca 1740
   aacctgacag atgtcagccg agccgggcct ggggctcctt tctccagcca gcaatgatgt 1800
   ccagaagaat attcaggact taacggctcc aggattttaa caaaagcaag actgttgctc 1860
   aaatctattc agacaagcaa caggttttat aattttttta ttactgattt tgttattttt 1920
   atatcagcct gagtctcctg tgcccacatc ccaggcttca ccctgaatgg ttccatgcct 1980
   gagggtggag actaagccct gtcgagacac ttgccttctt cacccagcta atctgtaggg 2040
   ctggacctat gtcctaagga cacactaatc gaactatgaa ctacaaagct tctatcccag 2100
   gaggtggcta tggccacccg ttctgctggc ctggatctcc ccactctagg ggtcaggctc 2160
   cattaggatt tgccccttcc catctcttcc tacccaacca ctcaaattaa tctttcttta 2220
   cctgagacca gttgggagca ctggagtgca gggaggagag gggaagggcc agtctgggct 2280
   gccgggttct agtctccttt gcactgaggg ccacactatt accatgagaa gagggcctgt 2340
   gggagcctgc aaactcactg ctcaagaaga catggagact cctgccctgt tgtgtataga 2400
   tgcaagatat ttatatatat ttttggttgt caatattaaa tacagacact aagttatagt 2460
   atatctggac aagccaactt gtaaatacac cacctcactc ctgttactta cctaaacaga 2520
   tataaatggc tggtttttag aaacatggtt ttgaaatgct tgtggattga gggtaggagg 2580
   tttggatggg agtgagacag aagtaagtgg ggttgcaacc actgcaacgg cttagacttc 2640
   gactcaggat ccagtccctt acacgtacct ctcatcagtg tcctcttgct caaaaatctg 2700
   tttgatccct gttacccaga gaatatatac attctttatc ttgacattca aggcatttct 2760
   atcacatatt tgatagttgg tgttcaaaaa aacactagtt ttgtgccagc cgtgatgctc 2820
   aggcttgaaa tcgcattatt ttgaatgtga agggaa 2856
<210> 5
   <211> 4461
   <212> DNA
   <213> Homo sapiens
<400> 5
   cttgttgttg atccgtaccc agtgggcagc gccgggagct ggaccaagcg gccggtgaga     60
   ggccgctgta gcggtgctca gccacctgtg ctgcctgcca gggggcgggc cgaaacctgg    120
   aggcccgggg ggcccagctc ccgtagggag ccgtgggcgc tcggtgcccg ggccgggcag    180
   gacagaataa taagctgaat agaatctgac cattggcttt cacctggcca ggaccttcta    240
   tgtagctctc cttttgtggc ccatgtgctg catcctctgc cctcagtgtg caactggccc    300
   ccaacgcaat gtgtgtttgt caaaccatgg aagtggggca gtatggcaag aatgcaagtc    360
   gggctggaga ccggggagtc ctcctggagc ccttcatcca ccaagtaggc ggacacagca    420
   gcatgatgcg ttacgacgat cacactgtgt gcaagcccct catctcccgg gaacagcgct    480
   tttacgagtc cctccctccc gaaatgaagg agttcacccc tgaatacaaa ggcgtggtat    540
   ctgtctgttt tgagggggac agtgatggtt acatcaactt agtggcctat ccttatgtgg    600
   aaagtgagac tgtggaacag gatgacacaa cagaacggga gcaacctcgg cgcaaacact    660
   cccgccggag cctgcaccgg tcaggcagtg gcagtgacca caaggaggag aaagccagcc    720
   tgtcccttga gacctctgag agctcacagg aggcaaagag tccgaaggtg gagctgcaca    780
   gccactcaga ggtccctttc cagatgctag atggcaacag tggcttgagt tctgagaaga    840
   tcagccacaa cccctggagc ctgcgttgtc acaagcagca gctgagccgc atgcgctccg    900
   agtccaagga ccgaaagctc tacaagttcc tcctgcttga gaacgtggtg caccacttca    960
   agtacccctg cgtgttggac ctgaagatgg gcacgcggca gcatggcgat gacgcgtcag   1020
   ctgagaaggc agcccggcag atgcggaaat gcgagcagag cacatcagcc acgctgggcg   1080
   tcagggtctg cggcatgcag gtgtaccagc tggacacagg gcattacctc tgcaggaaca 1140
   agtactatgg ccgtgggctc tccattgaag gcttccgcaa tgccctctat caatatctgc 1200
   acaatggcct ggacctgcga cgtgacctgt ttgagcctat cctgagcaaa ctgcggggcc 1260
   tgaaagctgt gctggagcgg caggcctctt accgcttcta ctccagttcc ctgcttgtca 1320
   tctatgatgg caaggagtgc cgggctgagt cctgcctgga ccgccggtct gagatgcgtc 1380
   tcaagcacct ggacatggtg ctccctgagg tggcgtcatc ctgtggcccc agcaccagcc 1440
   ccagcaacac cagccccgag gcgggtccct cctctcagcc caaggtggat gtccgcatga 1500
   ttgactttgc acacagcaca ttcaagggct tccgggatga ccccaccgtg catgatgggc 1560
   cagacagagg ctacgtgttt ggcctggaga acctcatcag catcatggaa cagatgcggg 1620
   acgagaacca gtaggccctg ttctgggccc ccagaacccc ttcctctcca ctgcaggcag 1680
   ggaccattgt tctgaacttg ccgtgaggac acacagactt gcttttaaag ggttatattt 1740
   ctctttggtg taaactaaaa gaaatgtttt tagctgtagc ctggaatcca tatatataaa 1800
   gtgaaggagg gcagaccaca cgccctctca gccaggctcc tcagctttgt ggctctgact 1860
   ggtgtgtcca ggctgcctta ggaaggaaga ggtgcccctg gtgggcttgg cagcagggac 1920
   agggtgccct tggacattgg tttctcttgt ctagatcttt gagatctgtg gctgcagggc 1980
   cctgctgatt gtaaggtaaa gccctgggct ggtgcagggc ccctccacgc ccactcttcc 2040
   cttgttcccc agaagtagag ggctctgggt gcccatttct tgggggcttt ccagtcttat 2100
   gctgtgggtg tcagctagct ctttaatagg tgccctcagg gcaccacagg gctgactgca 2160
   caaagctgga cccatccttc ggtctgacct tagcatgggg ctagattaat gaagctgggc 2220
   tgaggccaac ttatggcaga gggcggcgcc tgggttcccc aggcacctgt tggcacgtga 2280
   caggttggca cctgtcctat tcctgaaaca gcctctctca ccaagttccc ttgcctaaga 2340
   aggccactcc ctcccacccc actgaagtgg gggatagtcg gtgtcctagc aggcctcagg 2400
   gcctctggtg gctctggccc agacagtatt tgcagttctt gtgctatggg tgggagtctt 2460
   cttcctcaag tttcggcagc tgtgctgctg ctggatgggc tgctcctccc agggctcaag 2520
   ggctgtggtc cgctcagggt ctcatttccc caggccaagt tcaaggcagc agccctttgt 2580
   gaggcgctct tggccctggg cctggaggga gaactttaag cttttttgct cacagggacg 2640
   tggtatgggc cctgggtgca ggtgcccaca ttctgctaat gagagctttg tctgatcagt 2700
   cctgggtcca tcagtttgtc catgtgtccg gctgccagcc cgtcccttgg gatccttccc 2760
   ctggggtgta gccttgttca ttagtatata ctcattcctt catgctttcc tcagcagaac 2820
   acttccactt ctgaggtgag cttttgcccc gtgcccttcc tccacaggtg ttgccttttt 2880
   ataaagacct gatagcagaa taaattggtg tttccctgtt gacccagcac catttctgtg 2940
   ggcctagaat atggccctca acccttagag tggggcagtg agggcttgag gagtgaccct 3000
   tcctttctca tggttttagt cattttggct gccagccctt aatggcacag atctgctgct 3060
   tctaacagat ggccaggagg tgacaccgat ttcagccatt gccaaggtta gcaccctctc 3120
   ctttgagcct agggccacac tgttcattgt cactttaggc aagtgcctgt ttggctttaa 3180
   aggtaagcct gccagctgtg agaagccttg gtaactgatg gactcatttc ctggtcctta 3240
   aagatgcagc ctcttaaggg ctccttgatg gatgccatct ctcctagccc ccagccctgg 3300
   tgccactggt gggcaggttc ccattctttg gggctgggag ggacagcttg cctgtttctg 3360
   gtcacaaatt acagtcttct ctcctgtacc attctgtggc ttcagccatg ggggcagtag 3420
   cccttcatta gtgtagatag tcattccctg gtagggtgga gggtaagaca tagggtctgg 3480
   aactgtttgg gaccttttgg ggatgtcctg tgcctcccag attcctagat tctgggagga 3540
   gaggctgccg cattctgctg ctcctcacag cgagcaaagc tgcacccact tacattcagt 3600
   attttcctgg cactacaaag agtgggaagg cctgggattt gctgctgctc ccttagagca 3660
   gggcccctct tttcagcact ttggacacct ggagacccag ccctgttatt taatggtagt 3720
   gggcaagtgt gtgtgcatac tgtctgccac tgctttctcc ctgccccatg ccagagagcc 3780
   ctgtccctgc caggcccagc cttcttagcc ccaacttggg aacaaagtgc aacatgggat 3840
   catgggttgg ggtgctcagg tgagccctct ctatagtgct tccctgggcc aagctgacac 3900
   cagcccctga gggtggggtg ggacgggtgg tgcttaaaag aggaagggga ccagtgtagc 3960
   aacttgccag ggaccccacc cctccctctc tgggcctgtg cagtgagcat ggggattccc 4020
   atcaaggggc ctggcacctg tgctagttac gtagccgctg ctcacgcgct cactcctgac 4080
   cacatgcacg ttccctagat gcagactgct ttgaacttta aagctgtaca atttggttat 4140
   gtttgtgctg acttaaaata tattttaatg aggaaaaaat aatggagaac cctgggaagg 4200
   acctggttct tttgcttctc ggggaactgt aagccctcgc gttctgggaa tcgctctctg 4260
   ctgctctttc ctggaagcta agcctgtctc caccgcccga ggcctgcgcc ggtggctccc 4320
   gccgcagttg cgtttgcttt ggaccttgcg tgcgggggag ggggtgctcg gtccgagccc 4380
   gctcctttct gtacacctag cgctgcccgc cccgcttgtg tctgaggtcg tgtatgtcaa 4440
   aaataaagcc gctagaaacg g 4461
<210> 6
   <211> 847
   <212> DNA
   <213> Homo sapiens
<400> 6
   ggccacatgg actggggtgc aatgggacag ctgctgccag cgagagggac cagggcacca    60
   ctctctaggg agcccacact gcaagtcagg ccacaaggac ctctgaccct gagggccgat   120
   gaggccaggg acaggccagg ggggccttga ggcccctggt gagccaggcc ccaacctcag   180
   gcagcgctgg cccctgctgc tgctgggtct ggccgtggta acccatggcc tgctgcgccc   240
   aacagctgca tcgcagagca gggccctggg ccctggagcc cctggaggaa gcagccggtc   300
   cagcctgagg agccggtggg gcaggttcct gctccagcgc ggctcctgga ctggccccag   360
   gtgctggccc cgggggtttc aatccaagca taactcagtg acgcatgtgt ttggcagcgg   420
   gacccagctc accgttttaa gtcagcccaa ggccaccccc tcggtcactc tgttcccgcc   480
   gtcctctgag gagctccaag ccaacaaggc tacgctggtg tgtctcatga atgactttta   540
   tccgggaatc ttgacggtga cctggaaggc agatggtacc cccatcaccc agggcgtgga   600
   gatgaccacg ccctccaaac agagcaacaa caagtacgcg gccagcagct acctgagcct   660
   gacgcccgag cagtggaggt cccgcagaag ctacagctgc caggtcatgc acgaagggag   720
   caccgtggag aagacggtgg cccctgcaga atgttcatag gttcccagcc ccgaccccac   780
   ccaaaggcct ggagctgcag gatcccaggg gaagggtctc tctctgcatc ccaagccatc   840
   cagccct                                                             847
<210> 7
   <211> 2489
   <212> DNA
   <213> Homo sapiens
<400> 7
   attaccaggc acgcgcagga aacatggcgg cggcgggtgt tgtgagcggg aagattatat      60
   atgaacaaga aggagtatat attcactcat cttgtggaaa gaccaatgac caagacggct     120
   tgatttcagg aatattacgt gttttagaaa aggatgccga agtaatagtg gactggggac     180
   cattggatga tgcattagat tcctctagta ttctctatgc tagaaaggac tccagttcag     240
   ttgtagaatg gactcaggcc ccaaaagaaa gaggtcatcg aggatcagaa catctgaaca     300
   gttacgaagc agaatgggac atggttaata cagtttcatt taaaaggaaa ccacatacca     360
   atggagatgc tccaagtcat agaaatggga aaagcaaatg gtcattcctg ttcagtttga     420
   cagacctgaa atcaatcaag caaaacaaag agggtatggg ctggtcctat ttggtattct     480
   gtctaaagga tgacgtcgtt ctccctgctc tacactttca tcaaggagat agcaaactac     540
   tgattgaatc tcttgaaaaa tatgtggtat tgtgtgaatc tccacaggat aaaagaacac     600
   ttcttgtgaa ttgtcagaat aagagtcttt cacagtcttt tgaaaatctt cttgatgagc     660
   cagcatatgg tttaatacaa aaaattaaaa aggaccctta tacggcaact atgataggat     720
   tttccaaagt cacaaactac atttttgaca gtttgagagg cagcgatccc tctacacatc     780
   aacgaccacc ttcagaaatg gcagattttc ttagtgatgc tattccaggt ctaaagataa     840
   atcaacaaga agaaccagga tttgaagtca tcacaagaat tgatttgggg gaacgccctg     900
   ttgttcaaag gagagaaccg gtatcactgg aagaatggac taagaacatt gattctgaag     960
   gaagaatttt aaatgtagat aatatgaagc agatgatatt tagaggggga cttagtcatg 1020
   cattgagaaa gcaagcatgg aaatttcttc tgggttattt tccctgggac agtaccaagg 1080
   aggaaagaac ccaattacaa aagcaaaaaa ctgatgaata cttcagaatg aaactgcagt 1140
   ggaaatccat cagccaggaa caagagaaaa gaaattcgag gttaagagat tatagaagtc 1200
   ttatcgaaaa agatgttaac agaacagatc gaacaaacaa gttttatgaa ggccaagata 1260
   atccagggtt gattttactt catgacattt tgatgaccta ctgtatgtat gattttgatt 1320
   taggatatgt tcagggaatg agtgatttac tttcccctct tttatatgtg atggaaaatg 1380
   aagtggatgc cttttggtgc tttgcctctt acatggacca aatgcatcag aattttgaag 1440
   aacaaatgca aggcatgaag acccagctaa ttcagctgag taccttactt cgattgttag 1500
   acagtggatt ttgcagttac ttagaatctc aggactctgg atacctttat ttttgcttca 1560
   ggtggctttt aatcagattc aaaagggaat ttagttttct agatattctt cgattatggg 1620
   aggtaatgtg gaccgaacta ccatgtacaa atttccatct tcttctctgt tgtgctattc 1680
   tggaatcaga aaagcagcaa ataatggaaa agcattatgg cttcaatgaa atacttaagc 1740
   atatcaatga attgtccatg aaaattgatg tggaagatat actctgcaag gcagaagcaa 1800
   tttctctaca gatggtaaaa tgcaaggaat tgccacaagc agtctgtgag atccttgggc 1860
   ttcaaggcgg tgaagttaca acaccagatt cagacgttgg tgaagacgaa aatgttgtca 1920
   tgactccttg tcctacatct gcatttcaaa gtaatgcctt gcctacactc tctgccagtg 1980
   gagccagaaa tgacagccca acacagatac cagtgtcctc agatgtctgc agattaacac 2040
   ctgcatgatc actgttcttg cttttttggg aagagacact ttgttgcaac cctttttcaa 2100
   gtacttgaaa gttgaaaatt tgaaatcttg gtattgatca tgctttaagg tttatgtaaa 2160
   gaaagtgtac tgatgttctt acattaaagc tttacaaaga tttaaactaa ttatttttgt 2220
   agttacttct accaaatagc ctttcctttt cgataacatt cctcagtatt tttatagcca 2280
   agtacatttt attttcttgc tgatgaactg gaattggata aatattgcaa gtggatgagt 2340
   tggaaattat gcactttgaa aaacattcac tttgtttaag cttattgggt ttcagatttg 2400
   attaaattaa atgtggaggc tttctatagc attctaagct gagaagtaga ttgttaccca 2460
   gtaatgaaat aaaaaataaa aataaaagg 2489
<210> 8
   <211> 1673
   <212> DNA
   <213> Homo sapiens
<400> 8
   agcccagcac tagaagtcgg cggtgtttcc attcggtgat cagcactgaa cacagaggac 60
   tcaccatgga gtttgggctg agctgggttt tcctcgttgc tcttttaaga ggtgtccagt 120
   gtcaggtgca gctggtggag tctgggggag gcgtggtcca gcctgggagg tccctgagac 180
   tctcctgtgc agcgtctgga ttcaccttca gtaattatgg catgcactgg gtccgccagg 240
   ctccaggcaa ggggctggag tgggtggcag ctatatggta tgatggaagt aataaatact 300
   atgcagactc cgtgaagggc cgattcacca tctccagaga caattccaag aacacgttgt 360
   atatgcaaat gaacagcctg agagccgagg acacggctgt gtattattgt gcgagagagg 420
   gtcggtgggt acgatatact acggtgacta ctatcggata ctactttgac tactggggcc 480
   agggaaccct ggtcaccgtc tcctcagcct ccaccaaggg cccatcggtc ttccccctgg 540
   caccctcctc caagagcacc tctgggggca cagcggccct gggctgcctg gtcaaggact 600
   acttccccga accggtgacg gtgtcgtgga actcaggcgc cctgaccagc ggcgtgcaca 660
   ccttcccggc tgtcctacag tcctcaggac tctactccct cagcagcgtg gtgaccgtgc 720
   cctccagcag cttgggcacc cagacctaca tctgcaacgt gaatcacaag cccagcaaca 780
   ccaaggtgga caagagagtt gagcccaaat cttgtgacaa aactcacaca tgcccaccgt 840
   gcccagcacc tgaactcctg gggggaccgt cagtcttcct cttcccccca aaacccaagg 900
   acaccctcat gatctcccgg acccctgagg tcacatgcgt ggtggtggac gtgagccacg 960
   aagaccctga ggtcaagttc aactggtacg tggacggcgt ggaggtgcat aatgccaaga 1020
   caaagccgcg ggaggagcag tacaacagca cgtaccgtgt ggtcagcgtc ctcaccgtcc 1080
   tgcaccagga ctggctgaat ggcaaggagt acaagtgcaa ggtctccaac aaagccctcc 1140
   cagcccccat cgagaaaacc atctccaaag ccaaagggca gccccgagaa ccacaggtgt 1200
   acaccctgcc cccatcccgg gaggagatga ccaagaacca ggtcagcctg acctgcctgg 1260
   tcaaaggctt ctatcccagc gacatcgccg tggagtggga gagcaatggg cagccggaga 1320
   acaactacaa gaccacgcct cccgtgctgg actccgacgg ctccttcttc ctctatagca 1380
   agctcaccgt ggacaagagc aggtggcagc aggggaacgt cttctcatgc tccgtgatgc 1440
   atgaggctct gcacaaccac tacacgcaga agagcctctc cctgtccccg ggtaaatgag 1500
   tgcgacggcc ggcaagcccc cgctccccgg gctctcgcgg tcgcacgagg atgcttggca 1560
   cgtaccccgt ctacatactt cccaggcacc cagcatggaa ataaagcacc caccactgcc 1620
   ctgggccctg caaaaaaaaa aaaaaaaaaa aaaaaaaaaa aaaaaaaaaa aaa 1673
<210> 9
   <211> 1264
   <212> DNA
   <213> Homo sapiens
<400> 9
   gtggtaccca gtcctcaggt gcaaccccct gcgtggtcct ctgtggcagc cttctctcat     60
   tcagagctgt tttccacaga ggtagtgaaa agaactggat tttcaagttc actttgcaag    120
   agaaaaagaa aactcagtag aagataatgg caagtccaga ctggggatat gatgacaaaa    180
   atggtcctga acaatggagc aagctgtatc ccattgccaa tggaaataac caatcccctg    240
   ttgatattaa aaccagtgaa accaaacatg acacctctct gaaacctatt agtgtctcct    300
   acaacccagc cacagccaaa gaaattatca atgtggggca ttctttccat gtaaattttg    360
   aggacaacga taaccgatca gtgctgaaag gtggtccttt ctctgacagc tacaggctct    420
   ttcagtttca ttttcactgg ggcagtacaa atgagcatgg ttcagaacat acagtggatg    480
   gagtcaaata ttctgccgag cttcacgtag ctcactggaa ttctgcaaag tactccagcc    540
   ttgctgaagc tgcctcaaag gctgatggtt tggcagttat tggtgttttg atgaaggttg    600
   gtgaggccaa cccaaagctg cagaaagtac ttgatgccct ccaagcaatt aaaaccaagg    660
   gcaaacgagc cccattcaca aattttgacc cctctactct ccttccttca tccctggatt    720
   tctggaccta ccctggctct ctgactcatc ctcctcttta tgagagtgta acttggatca    780
   tctgtaagga gagcatcagt gtcagctcag agcagctggc acaattccgc agccttctat    840
   caaatgttga aggtgataac gctgtcccca tgcagcacaa caaccgccca acccaacctc    900
   tgaagggcag aacagtgaga gcttcatttt gatgattctg agaagaaact tgtccttcct    960
   caagaacaca gccctgcttc tgacataatc cagttaaaat aataattttt aagaaataaa   1020
   tttatttcaa tattagcaag acagcatgcc ttcaaatcaa tctgtaaaac taagaaactt   1080
   aaattttagt tcttactgct taattcaaat aataattagt aagctagcaa atagtaatct   1140
   gtaagcataa gcttatctta aattcaagtt tagtttgagg aattctttaa aattacaact   1200
   aagtgatttg tatgtctatt tttttcagtt tatttgaacc aataaaataa ttttatctct   1260
   ttct                                                                1264
<210> 10
   <211> 2454
   <212> DNA
   <213> Homo sapiens
<400> 10
   ggaataggtt agtttcagac aagcctgctt gccggagctc agcagacacc aggccttccg     60
   ggcaggcctg gcccaccgtg ggcctcagag ctgctgctgg ggcattcaga accggctctc    120
   cattggcatt gggaccagag accccgcaag tggcctgttt gcctggacat ccacctgtac    180
   gtccccaggt ttcgggaggc ccaggggcga tgccagaccc cgcggcgcac ctgcccttct    240
   tctacggcag catctcgcgt gccgaggccg aggagcacct gaagctggcg ggcatggcgg    300
   acgggctctt cctgctgcgc cagtgcctgc gctcgctggg cggctatgtg ctgtcgctcg    360
   tgcacgatgt gcgcttccac cactttccca tcgagcgcca gctcaacggc acctacgcca    420
   ttgccggcgg caaagcgcac tgtggaccgg cagagctctg cgagttctac tcgcgcgacc    480
   ccgacgggct gccctgcaac ctgcgcaagc cgtgcaaccg gccgtcgggc ctcgagccgc 540
   agccgggggt cttcgactgc ctgcgagacg ccatggtgcg tgactacgtg cgccagacgt 600
   ggaagctgga gggcgaggcc ctggagcagg ccatcatcag ccaggccccg caggtggaga 660
   agctcattgc tacgacggcc cacgagcgga tgccctggta ccacagcagc ctgacgcgtg 720
   aggaggccga gcgcaaactt tactctgggg cgcagaccga cggcaagttc ctgctgaggc 780
   cgcggaagga gcagggcaca tacgccctgt ccctcatcta tgggaagacg gtgtaccact 840
   acctcatcag ccaagacaag gcgggcaagt actgcattcc cgagggcacc aagtttgaca 900
   cgctctggca gctggtggag tatctgaagc tgaaggcgga cgggctcatc tactgcctga 960
   aggaggcctg ccccaacagc agtgccagca acgcctcagg ggctgctgct cccacactcc 1020
   cagcccaccc atccacgttg actcatcctc agagacgaat cgacaccctc aactcagatg 1080
   gatacacccc tgagccagca cgcataacgt ccccagacaa accgcggccg atgcccatgg 1140
   acacgagcgt gtatgagagc ccctacagcg acccagagga gctcaaggac aagaagctct 1200
   tcctgaagcg cgataacctc ctcatagctg acattgaact tggctgcggc aactttggct 1260
   cagtgcgcca gggcgtgtac cgcatgcgca agaagcagat cgacgtggcc atcaaggtgc 1320
   tgaagcaggg cacggagaag gcagacacgg aagagatgat gcgcgaggcg cagatcatgc 1380
   accagctgga caacccctac atcgtgcggc tcattggcgt ctgccaggcc gaggccctca 1440
   tgctggtcat ggagatggct gggggcgggc cgctgcacaa gttcctggtc ggcaagaggg 1500
   aggagatccc tgtgagcaat gtggccgagc tgctgcacca ggtgtccatg gggatgaagt 1560
   acctggagga gaagaacttt gtgcaccgtg acctggcggc ccgcaacgtc ctgctggtta 1620
   accggcacta cgccaagatc agcgactttg gcctctccaa agcactgggt gccgacgaca 1680
   gctactacac tgcccgctca gcagggaagt ggccgctcaa gtggtacgca cccgaatgca 1740
   tcaacttccg caagttctcc agccgcagcg atgtctggag ctatggggtc accatgtggg 1800
   aggccttgtc ctacggccag aagccctaca agaagatgaa agggccggag gtcatggcct 1860
   tcatcgagca gggcaagcgg atggagtgcc caccagagtg tccacccgaa ctgtacgcac 1920
   tcatgagtga ctgctggatc tacaagtggg aggatcgccc cgacttcctg accgtggagc 1980
   agcgcatgcg agcctgttac tacagcctgg ccagcaaggt ggaagggccc ccaggcagca 2040
   cacagaaggc tgaggctgcc tgtgcctgag ctcccgctgc ccaggggagc cctccacgcc 2100
   ggctcttccc caccctcagc cccaccccag gtcctgcagt ctggctgagc cctgcttggt 2160
   tgtctccaca cacagctggg ctgtggtagg gggtgtctca ggccacaccg gccttgcatt 2220
   gcctgcctgg ccccctgtcc tctctggctg gggagcaggg aggtccggga gggtgcggct 2280
   gtgcagcctg tcctgggctg gtggctcccg gagggccctg agctgagggc attgcttaca 2340
   cggatgcctt cccctgggcc ctgacattgg agcctgggca tcctcaggtg gtcaggcgta 2400
   gatcaccaga ataaacccag cttccctctt gaaaaaaaaa aaaaaaaaaa aacc 2454
<210> 11
   <211> 2196
   <212> DNA
   <213> Homo sapiens
<400> 11
   agatctcctg aggtcaggag ttcaagacaa gcccagacaa cttggtgaat gaaaccccat     60
   ctctactaaa aacaaaaaca gaaacaacaa aaaagaaaga gccctctggt taaccttgta    120
   tgtgtgagac gattatgatg agatagatcc cagattgaac aactggtcac ccaggaattt    180
   taaatttgct gctggagggc acaaaatttt gtctctcttt cctttttctt acactgggct    240
   cttggctcta aatgtagagg ctcacatcat tctccctgtg aggcgcttgg acagagagct    300
   cttatgctgt tcactcacca ggtgccaagg cagagtagat tctaatattt gagttgaaca    360
   ttcttgaaca gttatcctgg gaaacagtag ataccagaca gcccttgaac tggctccagg    420
   ccgcttttta tttgcaggct ctcagttcag cagtgcttgt ggggatgggc ctgtttcata    480
   ctctagattg actgggaggg aatcaagcca gatggcattc acctcccaga gatgtatcct    540
   agacacacat ttccacattg tcagggttct ggtgctttct tacagtcatg ccctacacag    600
   tgtgtcccta caaaaggtcc gaactttcac cttcagatcc ttcttccctt gattgtgggc    660
   aaacttggct gaatctagtt ctgttttatt ccaaaggaca atttatatca cattgttcac    720
   agaagagaca ttccccctgc cccgtcaacc ttttccacac cactgcaccc accaggtgat    780
   ttgcatattg tcccctaggg tggacccttc cccttgtgag tctgagataa aaagctcagc    840
   tctatccttg ccttgactga tcaggactcc tcagttcacc ttctcaccat gaggctccct    900
   gctcagctcc tggggctgct aatgctctgg gtccctggta aggacagaaa gagatgaggg    960
   aggacaactg ggtgggaggt gagctctgtg ggctccacag cttcacatgt ttattccaat   1020
   aatgtgatag aggcacatgg tctatgctcc agggaatgga attcaggttt gtcttatgaa   1080
   taatcaggat tcacctccag ggaacgatga ccagtgctct gattaagaac ttgaaaaaaa   1140
   agagttccct tgtggctaat aaataatggg tctattttag aaagtctact tttcatgata   1200
   taaatcaaaa ctttaaaaat gtaactgtaa atttatatca caagagaaat tatgaaagtt   1260
   gctcataatg tatctatata aacttgcact tctctgttat tatttcagga tccagtgagg   1320
   atattgtgat gacccagact ccactctccc tgcccgtcac ccctggagag ccggcctcca   1380
   tctcctgcag gtctagtcag agcctcttgg atagtgatga tggaaacacc tatttggact   1440
   ggtacctgca gaagccaggg cagtctccac agctcctgat ctatacgctt tcctatcggg   1500
   cctctggagt cccagacagg ttcagtggca gtgggtcagg cactgatttc acactgaaaa   1560
   tcagcagggt ggaggctgag gatgttggag tttattactg catgcaacgt atagagtttc   1620
   cttccacagt ggtacagccc tgaacagaaa cctccctgct gtggtgcccc agctgctcac   1680
   atgcactgct tgtctgggga gcaggtcagc agcgtctctg agtctgcaaa agaggaggct   1740
   gttggagaat acagggcagg gtttgcttct gaggactctg cctgggacta caggtgcatg   1800
   ccactaaaca tggctaattt ttctattttt ttgtagagtc ggtgcttcac catgttgccc   1860
   agcctgttgt caaaatcatg ggctcaagcc acccacctga cttggcctcc caacgtgctg   1920
   gcagtacagt gtgagccact gcggcaggtc agcacccctg tttatgttcc tgtcacctgc   1980
   cacagccttg actctcataa ccaacaggaa aatgaggagg ttctagggcc ctgtgagtaa   2040
   aaaactggga tgatagggaa aggagaatgg aatctcatct gaatcctcct tccttgccta   2100
   catttgttta aatttattga gcaaaagggc cagactactg atcatttctg gcaaaacatg   2160
   ttgagtacat tttagggttt aacagttttg ggtacc                             2196
<210> 12
   <211> 972
   <212> DNA
   <213> Homo sapiens
<400> 12
   gatcaggact cctcagttca ccttctcaca atgaggctcc ctgctcagct cctggggctg     60
   ctaatgctct gggtctctgg atccagtggg gatattgtga tgactcagtc tccactctcc    120
   ctgcccgtca cccctggaga gccggcctcc atctcctgca ggtctagtca gagcctcctg    180
   catagtgatg gatacaacta tttggattgg tacctgcaga agccagggca gtctccacag    240
   ctcctgatct atttgggttc taatcgggcc tccggggtcc ctgacaggtt cagtggcagt    300
   ggatcaggca cagattttac actgaaaatc agcaaagtgg aggctgagga tgttgggatt    360
   tattactgca tgcaaggtct acaaactcct cagacgttcg gccaagggac caaggtggaa    420
   atcaaacgaa ctgtggctgc accatctgtc ttcatcttcc cgccatctga tgagcagttg    480
   aaatctggaa ctgcctctgt tgtgtgcctg ctgaataact tctatcccag agaggccaaa    540
   gtacagtgga aggtggataa caccctccaa tcgggtaact cccaggagag tgtcacagag    600
   caggacagca aggacagcac ctacagcctc agcagcaccc tgacgctgag caaagcagac    660
   tacgagaaac acaaagtcta cgcctgcgaa gtcacccatc agggcctgag ctcgcccgtc    720
   acaaagagct tcaacagggg agagtgttag agggagaagt gcccccacct gctcctcagt    780
   tccagcctga ccccctccca tcctttggcc tctgaccctt tttccacagg ggacctaccc    840
   ctattgcggt cctccagctc atctttcacc tcacccccct cctcctcctt ggctttaatt    900
   atgctaatgt tggaggagaa tgaataaata aagtgaatct ttgaaaaaaa aaaaaaaaaa    960
   aaaaaaaaaa aa                                                        972
<210> 13
   <211> 835
   <212> DNA
   <213> Homo sapiens
<400> 13
   ggcacgaggc tcaaccacag actacacttg ctgaactggc tcctggggcc atgaggctgt    60
   cactgccact gctgctgctg ctgctgggag cctgggccat cccagggggc ctcggggaca   120
   gggcgccact cacagccaca gccccacaac tggatgatga ggagatgtac tcagcccaca   180
   tgcccgctca cctgcgctgt gatgcctgca gagctgtggc ttaccagatg tggcaaaatc   240
   tggcaaaggc agagaccaaa cttcatacct caaactctgg ggggcggcgg gagctgagcg   300
   agttggtcta cacggatgtc ctggaccgga gctgctcccg gaactggcag gactacggag   360
   ttcgagaagt ggaccaagtg aaacgtctca caggcccagg acttagcgag gggccagagc   420
   caagcatcag cgtgatggtc acagggggcc cctggcctac caggctctcc aggacatgtt   480
   tgcactactt gggggagttt ggagaagacc agatctatga agcccaccaa caaggccgag   540
   gggctctgga ggcattgcta tgtgggggac cccagggggc ctgctcagag aaggtgtcag   600
   ccacaagaga agagctctag tcctggactc taccctcctc tgaaagaagc tggggcttgc   660
   tctgacggtc tccactcccg tctgcaggca gccaggaggg caggaagccc ttgctctgtg   720
   ctgccatcct gcctccctcc tccagcctca gggcactcgg gcctgggtgg gagtcaacgc   780
   cttcccctct ggactcaaat aaaacccagt gacctcaaaa aaaaaaaaaa aaaaa        835
<210> 14
   <211> 1436
   <212> DNA
   <213> Homo sapiens
<400> 14
   gtccgcggaa atttgaaatg gctgacgggt cgctgacggg cggcggtctg gaggcagcgg    60
   ccatggcgcc ggagcgcacg ggctgggcgg tggagcagga gctggcgtct ctggagaaag   120
   tttttcagaa gaagtgaagt caagatgaag aaccatttgc ttttctgggg agtcctggcg   180
   gtttttatta aggctgttca tgtgaaagcc caagaagatg aaaggattgt tcttgttgac   240
   aacaaatgta agtgtgcccg gattacttcc aggatcatcc gttcttccga agatcctaat   300
   gaggacattg tggagagaaa catccgaatt attgttcctc tgaacaacag ggagaatatc   360
   tctgatccca cctcaccatt gagaaccaga tttgtgtacc atttgtctga cctctgtaaa   420
   aaatgtgatc ctacagaagt ggagctggat aatcagatag ttactgctac ccagagcaat   480
   atctgtgatg aagacagtgc tacagagacc tgctacactt atgacagaaa caagtgctac   540
   acagctgtgg tcccactcgt atatggtggt gagaccaaaa tggtggaaac agccttaacc   600
   ccagatgcct gctatcctga ctaatttaag tcattgctga ctgcatagct ctttttcttg   660
   agaggctctc cattttgatt cagaaagtta gcatatttat taccaatgaa tttgaaacca 720
   gggctttttt ttttttttgg gtgatgtaaa accaactccc cgccaccaaa ataattaaaa 780
   tagtcacatt gttatcttta ttaggtaatc acttcttaat tatatgttca tactctaagt 840
   atcaaaatct tccaattatc atgctcacct gaaagaggta tgctctctta ggaatacagt 900
   ttctagcatt aaacaaataa acaaggggag aaaataaaac tcaaggagtg aaaatcagga 960
   ggtgtaataa aatgttcctc gcattccccc ccgctttttt ttttttttga ctttgccttg 1020
   gagagccaga gcttccgcat tttctttact attcttttta aaaaaagttt cactgtgtag 1080
   agaacatata tgcataaaca taggtcaatt atatgtctcc attagaaaaa taataattgg 1140
   aaaacatgtt ctagaactag ttacaaaaat aatttaaggt gaaatctcta atatttataa 1200
   aagtagcaaa ataaatgcat aattaaaata tatttggaca taacagactt ggaagcagat 1260
   gatacagact tctttttttc ataatcaggt tagtgtaaga aattgccatt tgaaacaatc 1320
   cattttgtaa ctgaacctta tgaaatatat gtatttcatg gtacgtattc tctagcacag 1380
   tctgagcaat taaatagatt cataagaaaa aaaaaaaaaa aaaaaaaaaa aaaaaa 1436
<210> 15
   <211> 660
   <212> DNA
   <213> Homo sapiens
<400> 15
   atgtccgaga ctgcgcctgc cgcgcccgct gctccggccc ctgccgagaa gactcccgtg 60
   aagaagaagg cccgcaagtc tgcaggtgcg gccaagcgca aagcgtctgg gcccccggtg 120
   tccgagctca ttactaaagc tgttgccgcc tccaaggagc gcagcggcgt atctttggcc 180
   gctctcaaga aagcgctggc agccgctggc tatgacgtgg agaaaaacaa cagccgcatc 240
   aagctgggtc tcaagagcct ggtgagcaag ggcaccctgg tgcagaccaa gggcaccggc 300
   gcgtcgggtt ccttcaaact caacaagaag gcggcctctg gggaagccaa gcctaaggct 360
   aaaaaggcag gcgcggccaa ggccaagaag ccagcaggag cggcgaagaa gcccaagaag 420
   gcgacggggg cggccacccc caagaagagc gccaagaaga ccccaaagaa ggcgaagaag 480
   ccggctgcag ctgctggagc caaaaaagcg aaaagcccga aaaaggcgaa agcagccaag 540
   ccaaaaaagg cgcccaagag cccagcgaag gccaaagcag ttaaacccaa ggcggctaaa 600
   ccaaagaccg ccaagcccaa ggcagccaag ccaaagaagg cggcagccaa gaaaaagtag 660
<210> 16
   <211> 750
   <212> DNA
   <213> Homo sapiens
<400> 16
   agcttccctc tcctcctcac cctcctcact cactgtgcag ggtcctgggc ccagtctgtg    60
   ctgactcagc caccctcagc gtctgggacc cccgggcaga gggtcaccat ctcttgttct   120
   ggaagcagct ccaacatcgg aagtaatact gtaaactggt accagcagct cccaggaacg   180
   gcccccaaac tcctcatcta tcgtaataat cagcggccct caggggtccc tgaccgattc   240
   tctggctcca agtctggcac ctcagcctcc ctggccatca gtgggctcca gtctgaggat   300
   gaggctgatt attactgtgc agcatgggat gacagcctga atggtgtggt attcggcgga   360
   gggaccaagc tgaccgtcct aggtcagccc aaggctgccc cctcggtcac tctgttcccg   420
   ccctcctctg aggagcttca agccaacaag gccacactgg tgtgtctcat aagtgacttc   480
   tacccgggag ccgtgacagt ggcctggaag gcagatagca gccccgtcaa ggcgggagtg   540
   gagaccacca caccctccaa acaaagcaac aacaagtacg cggccagcag ctatctgagc   600
   ctgacgcctg agcagtggaa gtcccacaga agctacagct gccaggtcac gcatgaaggg   660
   agcaccgtgg agaagacagt ggcccctaca gaatgttcat aggttctcaa ccctcacccc   720
   ccaccacggg agactagagc tgcaggatcc                                    750
<210> 17
   <211> 597
   <212> DNA
   <213> Homo sapiens
<400> 17
   atgcccctag gtctcctgtg gctgggccta gccctgttgg gggctctgca tgcccaggcc    60
   caggactcca cctcagacct gatcccagcc ccacctctga gcaaggtccc tctgcagcag   120
   aacttccagg acaaccaatt ccaggggaag tggtatgtgg taggcctggc agggaatgca   180
   attctcagag aagacaaaga cccgcaaaag atgtatgcca ccatctatga gctgaaagaa   240
   gacaagagct acaatgtcac ctccgtcctg tttaggaaaa agaagtgtga ctactggatc   300
   aggacttttg ttccaggttg ccagcccggc gagttcacgc tgggcaacat taagagttac   360
   cctggattaa cgagttacct cgtccgagtg gtgagcacca actacaacca gcatgctatg   420
   gtgttcttca agaaagtttc tcaaaacagg gagtacttca agatcaccct ctacgggaga   480
   accaaggagc tgacttcgga actaaaggag aacttcatcc gcttctccaa atatctgggc   540
   ctccctgaaa accacatcgt cttccctgtc ccaatcgacc agtgtatcga cggctga      597
<210> 18
   <211> 2112
   <212> DNA
   <213> Homo sapiens
<400> 18
   cgcgtcgctg cccagcccgg tccggcgcgc cacgcagtgg atctctggac aggacaagac 60
   tccgaagcta ctcccccagc acacagcccg ggacccacaa acccagcttg cccccagccc 120
   tcccacctgc cactccctgg cccctcccac cggccgcccc ccttggcgcg ggcgcatggt 180
   gtgaaaggcc aagtgctgag gcgggtatca tgggtgctgt gccctaggcc tgggtggcag 240
   ggggtgggtg gcctgtgggt gtgccggggg ggccagtgtg cccaccccag tctcttggcg 300
   tgctggaggg catcctggat ggaattgaag tgaatggaac agaagccaag caaggtggag 360
   tgtgggtcag acccagagga gaacagtgcc aggtcaccag atggaaagcg aaaaagaaag 420
   aacggccaat gttccctgaa aagcagcatg tcagggtata tccctagtta cctggacaaa 480
   gacgagcagt gtgtcgtgtg tggggacaag gcaactggtt atcactaccg ctgtatcact 540
   tgtgagggct gcaagggctt ctttcgccgc acaatccaga agaacctcca tcccacctat 600
   tcctgcaaat atgacagctg ctgtgtcatt gacaagatca cccgcaatca gtgccagctg 660
   tgccgcttca agaagtgcat cgccgtggcc atggccatgg acttggttct agatgactcg 720
   aagcgggtgg ccaagcgtaa gctgattgag cagaaccggg agcggcggcg gaaggaggag 780
   atgatccgat cactgcagca gcgaccagag cccactcctg aagagtggga tctgatccac 840
   attgccacag aggcccatcg cagcaccaat gcccagggca gccattggaa acagaggcgg 900
   aaattcctgc ccgatgacat tggccagtca cccattgtct ccatgccgga cggagacaag 960
   gtggacctgg aagccttcag cgagtttacc aagatcatca ccccggccat cacccgtgtg 1020
   gtggactttg ccaaaaaact gcccatgttc tccgagctgc cttgcgaaga ccagatcatc 1080
   ctcctgaagg ggtgctgcat ggagatcatg tccctgcggg cggctgtccg ctacgaccct 1140
   gagagcgaca ccctgacgct gagtggggag atggctgtca agcgggagca gctcaagaat 1200
   ggcggcctgg gcgtagtctc cgacgccatc ttcgaactgg gcaagtcact ctctgccttt 1260
   aacctggatg acacggaagt ggctctgctg caggctgtgc tgctaatgtc aacagaccgc 1320
   tcgggcctgc tgtgtgtgga caagatcgag aagagtcagg aggcgtacct gctggcgttc 1380
   gagcactacg tcaaccaccg caaacacaac attccgcact tctggcccaa gctgctgatg 1440
   aaggagagag aagtgcagag ttcgattctg tacaaggggg cagcggcaga aggccggccg 1500
   ggcgggtcac tgggcgtcca cccggaagga cagcagcttc tcggaatgca tgttgttcag 1560
   ggtccgcagg tccggcagct tgagcagcag cttggtgaag cgggaagtct ccaagggccg 1620
   gttcttcagc accagagccc gaagagcccg cagcagcgtc tcctggagct gctccaccga 1680
   agcggaattc tccatgcccg agcggtctgt ggggaagacg acagcagtga ggcggactcc 1740
   ccgagctcct ctgaggagga accggaggtc tgcgaggacc tggcaggcaa tgcagcctct 1800
   ccctgaagcc ccccagaagg ccgatgggga aggagaagga gtgccatacc ttctcccagg 1860
   cctctgcccc aagagcagga ggtgcctgaa agctgggagc gtgggctcag cagggctggt 1920
   cacctcccat cccgtaagac caccttccct tcctcagcag ccaaacatgg ccagactccc 1980
   ttgctttttg ctgtgtagtt ccctctgcct gggatgccct tccccctttc tctgcctggc 2040
   aacatcttac ttgtcctttg aggccccaac tcaagtgtca cctccttccc cagctccccc 2100
   aggcagaaat ag 2112
<210> 19
   <211> 975
   <212> DNA
   <213> Homo sapiens
<400> 19
   atgagccgcc cgtcctccac cggccccagc gctaataaac cctgcagcaa gcagccgccg 60
   ccgcagcccc agcacactcc gtccccggct gcgcccccgg ccgccgccac catctcggct 120
   gcgggccccg gctcgtccgc ggtgcccgcc gcggcggcgg tgatctcggg ccccggcggc 180
   ggcggcgggg ccggcccggt gtccccgcag caccacgagc tgacctcgct cttcgagtgt 240
   ccggtctgct ttgactatgt cctgcctcct attctgcagt gccaggccgg gcacctggtg 300
   tgtaaccaat gccgccagaa gttgagctgc tgcccgacgt gcaggggcgc cctgacgccc 360
   agcatcagga acctggctat ggagaaggtg gcctcggcag tcctgtttcc ctgtaagtat 420
   gccaccacgg gctgttccct gaccctgcac catacggaga aaccagaaca tgaagacata 480
   tgtgaatacc gtccctactc ctgcccatgt cctggtgctt cctgcaagtg gcaggggtcc 540
   ctggaagctg tgatgtccca tctcatgcac gcccacaaga gcattaccac ccttcaggga 600
   gaagacatcg tctttctagc tacagacatt aacttgccag gggctgtcga ctgggtgatg 660
   atgcagtcat gttttggcca tcacttcatg ctggtgctgg agaaacaaga gaagtacgaa 720
   ggccaccagc agttttttgc catcgtcctg ctcattggca cccgcaagca agccgagaac 780
   tttgcctaca gactggagtt gaatgggaac cggcggagat tgacctggga ggccacgccc 840
   cgttcgattc atgacggtgt ggctgcggcc atcatgaaca gcgactgcct tgttttcgac 900
   acagccatag cacatctttt tgcagataat gggaaccttg gaatcaatgt tactatttct 960
   acatgttgtc catga 975
<210> 20
   <211> 650
   <212> DNA
   <213> Homo sapiens
<400> 20
   gtctcagtca ggacacagca tggacatgag ggtccccgct cagctcctgg ggctcctgct 60
   acttcggctc cgaggtgcca gatgtgacat ccagatgacc cagtctccat cctccctgtc 120
   tgcgtctgta ggagacagag tcaccatcac ttgccgggca agtcagagca ttagcagcta 180
   tttaaattgg tatcagcaga aaccagggaa agcccctaag ctcctgatct atgctgcatc 240
   cagtttgcaa agtggggtcc catcaaggtt cagtggcagt ggatctggga cagatttcac 300
   tctcaccatc agcagtctgc aacctgaaga ttttgcaagt tactactgtc aacagagtta 360
   caggaccccc gcgtggacgt tcggccaagg gaccaaggtg gaaatcaaac gaactgtggc 420
   tgcaccatct gtcttcatct tcccgccatc tgatgagcag ttgaaatctg gaactgcctc 480
   tgttgtgtgc ctgctgaata acttctatcc cagagaggcc aaagtacagt ggaaggtgga 540
   taacgccctc caatcgggta actcccagga gagtgtcaca gagcaggaca gcaaggacag 600
   cacctacagc ctcagcagca ccctgacgct gagcaaagca gactacgaga 650
<210> 21
   <211> 851
   <212> DNA
   <213> Homo sapiens
<400> 21
   cccgcaagtg tacctcaatg gcgagtttgt agggggctgt gacattcttc tgcagatgca 60
   ccagaatggg gacttggtgg aagaactgaa aaagctgggg atccactccg cccttttaga 120
   tgaaaagaaa gaccaagact ccaagtgagg gcggccaagt cctcgctgag cagagaggga 180
   gccgttcatg tcagagactc actgccagaa aagccttacc cattttggtt ttcactattg 240
   agaccgcaac tgcttgcact gatcattttg gttcatgagc agttggtgat tttagttggt 300
   ctggtgttcg ggctaagaat attttattgt ggacttaatt acaaccactg cactgtaatg 360
   attcaatgct gtattatgat attgctgtaa acaaaattca ttcttatatt gtcacttatt 420
   ctttgcctga ttcagaagtt aaataggagc tttggaatca ttattcatga cccctctgca 480
   aatgtgtcag tctccaaaga gagtatctcc ccccaaattt tgtgtagctt cttttgttat 540
   ggaaaatggt ggacaaaaaa agaaactgtg ataactgggg cgttgttttt taaaataaac 600
   tccagcacag ggatgctgtg catgcctgag ttgattccga aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa a 851
<210> 22
   <211> 927
   <212> DNA
   <213> Homo sapiens
<400> 22
   ggaagtttag gttaactgtc ttaaatttcc aaagctgtaa tcattatttt cattctcaaa 60
   gtgatggcct tgtgttttgc tcctctcctc cagggccaga ctgagcccag gttgatttca 120
   ggcggacacc aatagactcc acagcagctc caggagccca gacaccggcg gccagaagca 180
   aggctaggag ctgctgcagc catgtcggcc ctcagcctcc tcattctggg cctgctcacg 240
   gcagtgccac ctgccagctg tcagcaaggc ctggggaacc ttcagccctg gatgcagggc 300
   cttatcgcgg tggccgtgtt cctggtcctc gttgcaatcg cctttgcagt caaccacttc 360
   tggtgccagg aggagccgga gcctgcacac atgatcctga ccgtcggaaa caaggcagat 420
   ggagtcctgg tgggaacaga tggaaggtac tcttcgatgg cggccagttt caggtccagt 480
   gagcatgaga atgcctatga gaatgtgccc gaggaggaag gcaaggtccg cagcaccccg 540
   atgtaacctt ctctgtggct ccaaccccaa gactcccagg cacatgggat ggatgtccag 600
   tgctaccacc caagccccct ccttctttgt gtggaatctg caatagtggg ctgactccct 660
   ccagccccat gccggcccta cccgcccttg aagtatagcc agccaaggtt ggagctcaga 720
   ccgtgtctag gttggggctc ggctgtggcc ctggggtctc ctgctcagct cagaagagcc 780
   ttctggagag gacagtcagc tgagcacctc ccatcctgct cacacgtcct tccccataac 840
   tatggaaatg gccctaattt ctgtgaaata aagacttttt gtatttctgg ggctgaggct 900
   cagcaacagc ccctcaggct tccaaaa 927
<210> 23
   <211> 897
   <212> DNA
   <213> Homo sapiens
<400> 23
   ctcgcttttc ggttgccgtt gtcttttttc cttgactcgg aaatgtccgg tcgtggtaag      60
   cagggtggca aggcgcgcgc caaggctaag tcgcgctcgt cgcgcgcggg gctgcagttc     120
   cccgtgggcc gcgtgcaccg gttgctccgc aagggcaact attcggagcg cgtgggcgcc     180
   ggcgccccgg tctatctggc cgcggtgctc gagtacttga ctgccgagat cctggagctt     240
   gccggcaacg cggcgcgcga caacaagaag acgcgcatca tcccgcgcca cctgcagctg     300
   gccatccgca acgacgagga gctcaacaag ctgctgggcc gcgtgaccat cgcgcagggt     360
   ggcgtcctgc ccaacatcca ggccgtactg ctgcccaaga agacggagag ccaccacaag     420
   gccaagggca agtgaggccg cccgccgccc ccggggcccc tttgatggac ataaaggctc     480
   ttttcagagc cacctaccat ctcgagaaaa gagccgcact gatcctgcag ttctttatag     540
   gccggaggcc tgatcaccct aggctcatga atgagcgcag tggccatggg gaagggcgca     600
   acgggaaccg agaccctggg gactgattgg gctgcatact tgcgaggtgg gcaacgtgtt     660
   ctgttaacaa cagggaaccc tcgtccacag gtggccaccc cttgctcttg agtcccaccc     720
   aaaacctcta gtagggtttt aataacgctc accgtaaagg tgtcttcata attactagtg     780
   acaagttctc ttgactctag caaggttccc gtgtggtcat caagtacaga atgcaatttc     840
   ttaatgattt atctgatatt aaaagtattt atgatctcta aaaaaaaaaa aaaaaaa        897
<210> 24
   <211> 2533
   <212> DNA
   <213> Homo sapiens
<400> 24
   ggagctcaag ctcctctaca aagaggtgga cagagaagac agcagagacc atgggacccc 60
   cctcagcccc tccctgcaga ttgcatgtcc cctggaagga ggtcctgctc acagcctcac 120
   ttctaacctt ctggaaccca cccaccactg ccaagctcac tattgaatcc acgccattca 180
   atgtcgcaga ggggaaggag gttcttctac tcgcccacaa cctgccccag aatcgtattg 240
   gttacagctg gtacaaaggc gaaagagtgg atggcaacag tctaattgta ggatatgtaa 300
   taggaactca acaagctacc ccagggcccg catacagtgg tcgagagaca atatacccca 360
   atgcatccct gctgatccag aacgtcaccc agaatgacac aggattctat accctacaag 420
   tcataaagtc agatcttgtg aatgaagaag caaccggaca gttccatgta tacccggagc 480
   tgcccaagcc ctccatctcc agcaacaact ccaaccccgt ggaggacaag gatgctgtgg 540
   ccttcacctg tgaacctgag gttcagaaca caacctacct gtggtgggta aatggtcaga 600
   gcctcccggt cagtcccagg ctgcagctgt ccaatggcaa catgaccctc actctactca 660
   gcgtcaaaag gaacgatgca ggatcctatg aatgtgaaat acagaaccca gcgagtgcca 720
   accgcagtga cccagtcacc ctgaatgtcc tctatggccc agatgtcccc accatttccc 780
   cctcaaaggc caattaccgt ccaggggaaa atctgaacct ctcctgccac gcagcctcta 840
   acccacctgc acagtactct tggtttatca atgggacgtt ccagcaatcc acacaagagc 900
   tctttatccc caacatcact gtgaataata gcggatccta tatgtgccaa gcccataact 960
   cagccactgg cctcaatagg accacagtca cgatgatcac agtctctgga agtgctcctg 1020
   tcctctcagc tgtggccacc gtcggcatca cgattggagt gctggccagg gtggctctga 1080
   tatagcagcc ctggtgtatt ttcgatattt caggaagact ggcagattgg accagaccct 1140
   gaattcttct agctcctcca atcccatttt atcccatgga accactaaaa acaaggtctg 1200
   ctctgctcct gaagccctat atgctggaga tggacaactc aatgaaaatt taaagggaaa 1260
   accctcaggc ctgaggtgtg tgccactcag agacttcacc taactagaga cagtcaaact 1320
   gcaaaccatg gtgagaaatt gacgacttca cactatggac agcttttccc aagatgtcaa 1380
   aacaagactc ctcatcatga taaggctctt accccctttt aatttgtcct tgcttatgcc 1440
   tgcctctttc gcttggcagg atgatgctgt cattagtatt tcacaagaag tagcttcaga 1500
   gggtaactta acagagtgtc agatctatct tgtcaatccc aacgttttac ataaaataag 1560
   agatccttta gtgcacccag tgactgacat tagcagcatc tttaacacag ccgtgtgttc 1620
   aaatgtacag tggtcctttt cagagttgga cttctagact cacctgttct cactccctgt 1680
   tttaattcaa cccagccatg caatgccaaa taatagaatt gctccctacc agctgaacag   1740
   ggaggagtct gtgcagtttc tgacacttgt tgttgaacat ggctaaatac aatgggtatc   1800
   gctgagacta agttgtagaa attaacaaat gtgctgcttg gttaaaatgg ctacactcat   1860
   ctgactcatt ctttattcta ttttagttgg tttgtatctt gcctaaggtg cgtagtccaa   1920
   ctcttggtat taccctccta atagtcatac tagtagtcat actccctggt gtagtgtatt   1980
   ctctaaaagc tttaaatgtc tgcatgcagc cagccatcaa atagtgaatg gtctctcttt   2040
   ggctggaatt acaaaactca gagaaatgtg tcatcaggag aacatcataa cccatgaagg   2100
   ataaaagccc caaatggtgg taactgataa tagcactaat gctttaagat ttggtcacac   2160
   tctcacctag gtgagcgcat tgagccagtg gtgctaaatg ctacatactc caactgaaat   2220
   gttaaggaag aagatagatc caattaaaaa aaattaaaac caatttaaaa aaaaaaaaga   2280
   acacaggaga ttccagtcta cttgagttag cataatacag aagtcccctc tactttaact   2340
   tttacaaaaa agtaacctga actaatctga tgttaaccaa tgtatttatt tctgtggttc   2400
   tgtttccttg ttccaatttg acaaaaccca ctgttcttgt attgtattgc ccagggggag   2460
   ctatcactgt acttgtagag tggtgctgct ttaattcata aatcacaaat aaaagccaat   2520
   tagctctata act                                                      2533
<210> 25
   <211> 1020
   <212> DNA
   <213> Homo sapiens
<400> 25
   gaggaactgc tcagttagga cccagacgga accatggaag ccccagcgca gcttctcttc 60
   ctcctgctac tctggctccc agataccact ggagaaatag tgatgacgca gtctccagcc 120
   accctgtctg tgtctccagg ggaaagagcc accctctcct gcagggccag tcagagtgtt 180
   accagcaact tagcctggta ccagcagaca cctgggcagt ctcccaggct cgtcatctat 240
   ggtgcatcca gcagggccag tggtgtccca gccaggttca gtggcagtgg gtctgggaca 300
   gagttcactc tcaccatcag cagcctgcag tctgaagatt ttgcagttta ttactgtcag 360
   cagtataata agtggccgca cacttttggc caggggacca agctggacat caaacgaact 420
   gtggctgcac catctgtctt catcttcccg ccatctgatg agcagttgaa atctggaact 480
   gcctctgttg tgtgcctgct gaataacttc tatcccaggg aggccaaagt acagtggaag 540
   gtggataacg ccctccaatc gggtaactcc caggagagtg tcacagagca ggacagcaag 600
   gacagcacct acagcctcag cagcaccctg acgctgagca aagcagacta cgagaaacac 660
   aaagtctacg cctgcgaagt cacccatcag ggcctgagct cgcccgtcac aaagagcttc 720
   aacaggggag agtgttagag ggagaagtgc ccccacctgc tcctcagttc cagcctgacc 780
   ccctcccatc ctttggcctc tgaccctttt tccacagggg acctacccct attgcggtcc 840
   tccagctcat ctttcacctc acccccctcc tcctccttgg ctttaattat gctaatgttg 900
   gaggagaatg aataaataaa gtgaatcttt gcaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
<210> 26
   <211> 1020
   <212> DNA
   <213> Homo sapiens
<400> 26
   gaggaactgc tcagttagga cccagacgga accatggaag ccccagcgca gcttctcttc    60
   ctcctgctac tctggctccc agataccact ggagaaatag tgatgacgca gtctccagcc   120
   accctgtctg tgtctccagg ggaaagagcc accctctcct gcagggccag tcagagtgtt   180
   accagcaact tagcctggta ccagcagaca cctgggcagt ctcccaggct cgtcatctat   240
   ggtgcatcca gcagggccag tggtgtccca gccaggttca gtggcagtgg gtctgggaca   300
   gagttcactc tcaccatcag cagcctgcag tctgaagatt ttgcagttta ttactgtcag   360
   cagtataata agtggccgca cacttttggc caggggacca agctggacat caaacgaact   420
   gtggctgcac catctgtctt catcttcccg ccatctgatg agcagttgaa atctggaact   480
   gcctctgttg tgtgcctgct gaataacttc tatcccaggg aggccaaagt acagtggaag   540
   gtggataacg ccctccaatc gggtaactcc caggagagtg tcacagagca ggacagcaag   600
   gacagcacct acagcctcag cagcaccctg acgctgagca aagcagacta cgagaaacac   660
   aaagtctacg cctgcgaagt cacccatcag ggcctgagct cgcccgtcac aaagagcttc   720
   aacaggggag agtgttagag ggagaagtgc ccccacctgc tcctcagttc cagcctgacc   780
   ccctcccatc ctttggcctc tgaccctttt tccacagggg acctacccct attgcggtcc   840
   tccagctcat ctttcacctc acccccctcc tcctccttgg ctttaattat gctaatgttg   900
   gaggagaatg aataaataaa gtgaatcttt gcaaaaaaaa aaaaaaaaaa aaaaaaaaaa   960
   aaaaaaaaaa aaaaaaaaaa aaaaaaaaaa aaaaaaaaaa aaaaaaaaaa aaaaaaaaaa  1020
<210> 27
   <211> 564
   <212> DNA
   <213> Homo sapiens
<400> 27
   cgactttccc gatcgccagg caggagtttc tctcggtgac tactatcgct gtcatgtctg    60
   gtcgtggcaa gcaaggaggc aaggcccgcg ccaaggccaa gtcgcgctcg tcccgcgctg   120
   gccttcagtt cccggtaggg cgagtgcatc gcttgctgcg caaaggcaac tacgcggagc   180
   gagtgggggc cggcgcgccc gtctacatgg ctgcggtcct cgagtatctg accgccgaga   240
   tcctggagct ggcgggcaac gcggctcggg acaacaagaa gacgcgcatc atccctcgtc    300
   acctccagct ggccatccgc aacgacgagg aactgaacaa gctgctgggc aaagtcacca    360
   tcgcccaggg cggcgtcttg cctaacatcc aggccgtact gctccctaag aagacggaga    420
   gtcaccacaa ggcaaagggc aagtgaggct gacgtccggc ccaagtgggc ccagcccggc    480
   ccgcgtctcg aaggggcacc tgtgaactca aaaggctctt ttcagagcca cccacgtttt    540
   caaataaaag agttgttaat gctg                                           564
<210> 28
   <211> 2470
   <212> DNA
   <213> Homo sapiens
<400> 28
   acgaggcctg gccggggcgg gcggcgcggg ggcggcatga gggcccgcgg cccggggggc      60
   tgaggcgccc gccgcctgcc gcgggggccg ctcgcgtcct ccatggaggc cggagaggaa    120
   ccgctgctgc tggccgaact caagcccggg cgcccccacc agtttgattg gaagtccagc    180
   tgtgaaacct ggagcgtcgc cttctcccca gatggctcct ggtttgcttg gtctcaagga    240
   cactgcatcg tcaaactgat cccctggccg ttggaggagc agttcatccc taaagggttt    300
   gaagccaaaa gccgaagtag caaaaatgag acgaaagggc ggggcagccc aaaagagaag    360
   acgctggact gtggtcagat tgtctggggg ctggccttca gcccgtggcc ttccccaccc    420
   agcaggaagc tctgggcacg ccaccacccc caagtgcccg atgtctcttg cctggttctt    480
   gctacgggac tcaacgatgg gcagatcaag atctgggagg tgcagacagg gctcctgctt    540
   ttgaatcttt ccggccacca agatgtcgtg agagatctga gcttcacacc cagtggcagt    600
   ttgattttgg tctccgcgtc acgggataag actcttcgca tctgggacct gaataaacac    660
   ggtaaacaga ttcaagtgtt atcgggccac ctgcagtggg tttactgctg ttccatctcc    720
   ccagactgca gcatgctgtg ctctgcagct ggagagaagt cggtctttct atggagcatg    780
   aggtcctaca cgttaattcg gaagctagag ggccatcaaa gcagtgttgt ctcttgtgac    840
   ttctcccccg actctgccct gcttgtcacg gcttcttacg ataccaatgt gattatgtgg    900
   gacccctaca ccggcgaaag gctgaggtca ctccaccaca cccaggttga ccccgccatg    960
   gatgacagtg acgtccacat tagctcactg agatctgtgt gcttctctcc agaaggcttg   1020
   taccttgcca cggtggcaga tgacagactc ctcaggatct gggccctgga actgaaaact   1080
   cccattgcat ttgctcctat gaccaatggg ctttgctgca cattttttcc acatggtgga   1140
   gtcattgcca cagggacaag agatggccac gtccagttct ggacagctcc tagggtcctg   1200
   tcctcactga agcacttatg ccggaaagcc cttcgaagtt tcctaacaac ttaccaagtc   1260
   ctagcactgc caatccccaa gaaaatgaaa gagttcctca catacaggac tttttaagca   1320
   acaccacatc ttgtgcttct ttgtagcagg gtaaatcgtc ctgtcaaagg gagttgctgg 1380
   aataatgggc caaacatctg gtcttgcatt gaaatagcat ttctttggga ttgtgaatag 1440
   aatgtagcaa aaccagattc cagtgtacta gtcatggatc tttctctccc tggcatgtga 1500
   aagtcagtct tagaggaaga gattccactt gcacggcaac agagccttac gttaaatttt 1560
   cagtccagtt atgaacagca agtgttgaac tctttctgct tgttttgatt caaagtgcag 1620
   ttactgatgt tgttttgatt atgcaactaa gtaggcctcc agagcctctc tagtggcaga 1680
   gcagctcaca ctccctccgc tgggaacgat ggcttctgcc tagtacttat ccttgtgttt 1740
   ctgatgcagt ggtagcattg gttcaagttc tctcctgctg tggtcagagt tgcttcgatg 1800
   ttggccaagt gcttttcttc ttgggctccc ttctgacctg caggacagtt ttcctggagc 1860
   catttggtat gaggtattaa tttagcttaa ctaaattaca ggggactcag aggccgtgct 1920
   cctgaccgat ccagacacta ttactggctt tttttttttt tttttaacaa tggtgtgcat 1980
   gtgcaggaaa tgacaaattt gtatgtcaga ttatacaagg atgtattctt aaaccgcatg 2040
   actattcaga tggctactga gttatcagtg gccatttatt agcatcatat ttatttgtat 2100
   tttctcaaca gatgttaagg tacaactgtg tttttctcga ttatctaaaa accatagtac 2160
   ttaaattgaa cagttgcaaa gatgtcttaa ttgtgtaaag aattggtgta gtcatgactt 2220
   tagctgatac tcttatgtac gagatctgtc tctgctgttt aacttcattg gattaatcag 2280
   ctggtttcaa ctctactgcg aaacaaaaat agctccttaa aagtactgtt ctccttcagt 2340
   ggcatgtagt tatctaatca agacacctca ttcaaacaaa acctgcctta ggaaaattta 2400
   atatatttta aattatttta aaagaaatac aacatcttat tctttagctt tcaaaaaaaa 2460
   aaaaaaaaaa 2470
<210> 29
   <211> 2374
   <212> DNA
   <213> Homo sapiens
<400> 29
   gggcgatgag agcgggtact gcgaactgcc gggcgatgct gtcgctgccg ccgtgatacg      60
   gagagcaaca gttccccagc aacacccctc cccgacacag gcacacaccc cccgacaggc     120
   acgcacaccc accccacagt gcccggctcg gctgcgcctc ctctattggc ccaggaagcc     180
   cacccagccc cgccacgcag agcccagaag gaaagaaagc ctcatgcctg agccgagggg     240
   agcaccatgg atctgacaaa aatgggcatg atccagctgc agaaccctag ccaccccacg      300
   gggctactgt gcaaggccaa ccagatgcgg ctggccggga ctttgtgcga tgtggtcatc     360
   atggtggaca gccaggagtt ccacgcccac cggacggtgc tggcctgcac cagcaagatg     420
   tttgagatcc tcttccaccg caatagtcaa cactatactt tggacttcct ctcgccaaag     480
   accttccagc agattctgga gtatgcatat acagccacgc tgcaagccaa ggcggaggac 540
   ctggatgacc tgctgtatgc ggccgagatc ctggagatcg agtacctgga ggaacagtgc 600
   ctgaagatgc tggagaccat ccaggcctca gacgacaatg acacggaggc caccatggcc 660
   gatggcgggg ccgaggaaga agaggaccgc aaggctcggt acctcaagaa catcttcatc 720
   tcgaagcatt ccagcgagga gagtgggtat gccagtgtgg ctggacagag cctccctggg 780
   cccatggtgg accagagccc ttcagtctcc acttcatttg gtctttcagc catgagtccc 840
   accaaggctg cagtggacag tttgatgacc ataggacagt ctctcctgca gggaactctt 900
   cagccacctg cagggcccga ggagccaact ctggctgggg gtgggcggca ccctggggtg 960
   gctgaggtga agacggagat gatgcaggtg gatgaggtgc ccagccagga cagccctggg 1020
   gcagccgagt ccagcatctc aggagggatg gggacaaggg ttgaggaaag aggcaaagag 1080
   gggcctggga ccccgactcg aagcagcgtc atcaccagtg ctagggagct acactatggg 1140
   cgagaggaga gtgccgagca ggtgccaccc ccagctgagg ctggccaggc ccccactggc 1200
   cgacctgagc acccagcacc cccgcctgag aagcatctgg gcatctactc cgtgttgccc 1260
   aaccacaagg ctgacgctgt attgagcatg ccgtcttccg tgacctctgg cctccacgtg 1320
   cagcctgccc tggctgtctc catggacttc agcacctatg gggggctgct gccccagggc 1380
   ttcatccaga gggagctgtt cagcaagctg ggggagctgg ctgtgggcat gaagtcagag 1440
   agccggacca tcggagagca gtgcagcgtg tgtggggtcg agcttcctga taacgaggct 1500
   gtggagcagc acaggaagct gcacagtggg atgaagacgt acgggtgcga gctctgcggg 1560
   aagcggttcc tggatagttt gcggctgaga atgcacttac tggctcattc agcgggtgcc 1620
   aaagcctttg tctgtgatca gtgcggtgca cagttttcga aggaggatgc cctggagaca 1680
   cacaggcaga cccatactgg cactgacatg gccgtcttct gtctgctgtg tgggaagcgc 1740
   ttccaggcgc agagcgcact gcagcagcac atggaggtcc acgcgggcgt gcgcagctac 1800
   atctgcagtg agtgcaaccg caccttcccc agccacacgg ctctcaaacg ccacctgcgc 1860
   tcacatacag gcgaccaccc ctacgagtgt gagttctgtg gcagctgctt ccgggatgag 1920
   agcacactca agagccacaa acgcatccac acgggtgaga aaccctacga gtgcaatggc 1980
   tgtggcaaga agttcagcct caagcatcag ctggagacgc actatagggt gcacacaggt 2040
   gagaagccct ttgagtgtaa gctctgccac cagcgctccc gggactactc ggccatgatc 2100
   aagcacctga gaacgcacaa cggcgcctcg ccctaccagt gcaccatctg cacagagtac 2160
   tgccccagcc tctcctccat gcagaagcac atgaagggcc acaagcccga ggagatcccg 2220
   cccgactgga ggatagagaa gacgtacctc tacctgtgct atgtgtgaag ggaggcccgc 2280
   ggcggtggag ccgagcgggg agccaggaaa gaagagttgg agtgagatga aggaaggact 2340
   atgacaaata aaaaaaaaaa aaaaaaaaaa aaaa 2374
<210> 30
   <211> 393
   <212> DNA
   <213> Homo sapiens
<400> 30
   atgtctggac gtggaaagca aggcggcaaa gctcgggcaa aagctaaaac gcgttcttcc     60
   agggccggtc ttcagtttcc agttggccgt gtgcaccgcc tcctccgcaa aggcaactac    120
   tccgaacgag tcggggccgg cgctccagtg tacctggcag cggtgctgga atatctgacg    180
   gccgagatct tagagctagc tggcaacgcg gctcgcgaca ataagaagac ccgcatcatc    240
   ccgcgccacc tgcagctagc catccgcaac gacgaggagc taaataagct tctaggtcgc    300
   gtgaccatcg cgcagggcgg tgtcctgccc aacatccagg ccgtattgct gcctaagaag    360
   acggagagcc accataaggc caagggcaag tga                                 393
<210> 31
   <211> 857
   <212> DNA
   <213> Homo sapiens
<400> 31
   caggaaagat gcagccactc ctgcttctgc tggcctttct cctacccact ggggctgagg      60
   caggggagat catcggaggc cgggagagca ggccccactc ccgcccctac atggcgtatc     120
   ttcagatcca gagtccagca ggtcagagca gatgtggagg gttcctggtg cgagaagact     180
   ttgtgctgac agcagctcat tgctggggaa gcaatataaa tgtcaccctg ggcgcccaca     240
   atatccagag acgggaaaac acccagcaac acatcactgc gcgcagagcc atccgccacc     300
   ctcaatataa tcagcggacc atccagaatg acatcatgtt attgcagctg agcagaagag     360
   tcagacggaa tcgaaacgtg aacccagtgg ctctgcctag agcccaggag ggactgagac     420
   ccgggacgct gtgcactgtg gccggctggg gcagggtcag catgaggagg ggaacagata     480
   cactccgaga ggtgcagctg agagtgcaga gggataggca gtgcctccgc atcttcggtt     540
   cctacgaccc ccgaaggcag atttgtgtgg gggaccggcg ggaacggaag gctgccttca     600
   agggggattc cggaggcccc ctgctgtgta acaatgtggc ccacggcatc gtctcctatg     660
   gaaagtcgtc aggggttcct ccagaagtct tcaccagggt ctcaagtttc ctgccctgga     720
   taaggacaac aatgagaagc ttcaaactgc tggatcagat ggagaccccc ctgtgactga     780
   ctcttcttct cggggacaca ggccagctcc acagtgttgc cagagcctta ataaacgtcc     840
   acagagtata aataacc                                                    857
<210> 32
   <211> 3250
   <212> DNA
   <213> Homo sapiens
<400> 32
   ccaacttatt taaaacaaaa caattttgta ggtattatta tacccatttc acagatgatg 60
   ataaatgaga ccaatagaag ttaaataact tgccaaaggc cacacagctg gtgagtgatg 120
   gagaacgaat taaaactcaa gtgagcataa ttctaaaagc catcttctcg ttagtgtttc 180
   tcactatcca ggtctgcctt tgccttattt aactgaagtt aagccatcct tacctgtgat 240
   cacctagcct ctcagtttgg ggggatcatt acagcgggtt tttaactccc aatgttctgg 300
   tccagtttgc tttacatgtt cttatttata cattgtcaag gatgacctca ggacagtaca 360
   gcaaggacac agtggcactt cacattttgt tcccacgaaa tgactggggc ataatctcag 420
   atcatcttcc tttagaatgt ggaaacatca gcagaagaat attagtcttt atacaagtca 480
   aatccaaaat gacacatgtg aaaactaata gagctgactt tcagccatga tagctttggc 540
   acacctcaca tccctttgtt caacctctct tccctcaacg gagagctgca ttcctgggaa 600
   tttctgttgt gcacttttcc cacttgccct gctgtcattt aaaggtgaac attctagttt 660
   tgctaagaaa accctttcct tcatttggaa tgaacagcaa ttttattact tttgacctta 720
   aaatgagttt gctgccttca aatcttttca gcgccttcat cacgctctgc ttcggggcga 780
   tcttcttcct gccagactcc tccaagctgc tcagcggggt cctgttccac tccagccccg 840
   ccttgcagcc ggccgccgac cacaagcccg ggcccggggc gcgcgccgag gacgcggccg 900
   aggggcgagc ccggcgccgc gaggaggggg cacccgggga cccggaggcc gccctggagg 960
   acaacttggc caggatccgc gaaaaccacg agcgggctct cagggaagcc aaggagaccc 1020
   tgcagaagct gcccgaggag atccaaagag acatcctact ggagaagaag aaggtggccc 1080
   aggaccagct gcgtgacaag gcgccgttca gaggcctgcc cccggtggac ttcgtgcccc 1140
   caatcggggt ggagagccgg gagcccgccg acgccgccat ccgcgagaaa agggcaaaga 1200
   tcaaagagat gatgaaacat gcttggaata attataaagg ttatgcctgg ggattaaatg 1260
   aactcaaacc tatatcaaaa ggaggccatt caagcagttt gtttggtaac atcaaaggag 1320
   caactatagt agatgccctg gatacacttt ttattatgga aatgaaacat gaatttgaag 1380
   aagcaaaatc atgggttgaa gaaaatttag attttaatgt gaatgctgaa atttctgtct 1440
   ttgaagtaaa tatacgcttt gttggtggac tactctcagc ctactatctg tctggagaag 1500
   agatttttcg aaagaaagca gtggaacttg gggtaaaatt gctacctgca tttcatactc 1560
   cctctggaat accttgggca ttgctgaata tgaaaagtgg tattggaagg aactggccct 1620
   gggcctctgg aggcagcagt attctggcag aatttggaac cctgcatttg gagtttatgc 1680
   acttgagcca cttatcagga aaccccatct ttgctgaaaa ggtaatgaat attcgaacag 1740
   tactgaacaa actggaaaaa ccacaaggcc tttatcctaa ctatctgaat cccagtagtg 1800
   gacagtgggg tcaacatcat gtatcagttg gaggacttgg agacagcttc tatgagtatt 1860
   tgctgaaggc ctggttaatg tctgacaaga cagatctgga agctaagaag atgtattttg 1920
   atgctgttca ggctatcgag actcatttga tccgcaagtc tagcagcgga ctaacttata 1980
   tcgcagagtg gaaaaggggc ctcctggagc acaagatggg ccacctgacc tgcttcgcgg 2040
   ggggcatgtt cgcactcggg gctgatgcag ctcccgaagg catggcccaa cactaccttg 2100
   aactcggggc tgaaattgcc cgtacttgtc atgaatcata taatcgaaca tttatgaaac 2160
   tgggaccaga agctttcaga tttgatggtg gtgttgaagc catcgctaca agacaaaatg 2220
   aaaaatacta catcttacgg ccagaagtta tggagactta catgtatatg tggagactga 2280
   ctcatgatcc aaagtacagg aaatgggcct gggaagccgt agaggccttg gaaaaccatt 2340
   gcagagtgaa tggaggctat tcaggcctaa gggatgttta ccttcttcat gagagttatg 2400
   atgatgtgca gcagagtttc ttcctggcag agacattgaa atatttgtac ctaatatttt 2460
   ctgacgacga tcttcttcca ctggagcatt ggatcttcaa tagcgaggca catcttctcc 2520
   ctatcctccc taaagataaa aaggaagttg aaatcagaga ggaataaaaa agacatttat 2580
   attttattct gctccattcc cttcactgta taccttaata attccttttc tggtaatcag 2640
   gcacatgatg aactttgatt agtaggtctg tgattaagtt cttaaattgt tttgcagtct 2700
   tttatgttta ttatcatagg tataggtgga cctaaattcc ttatcatatc tttattaatt 2760
   cagccagtgt atccaccagt tttttgttta tgtttttaag taacctatta tctctggatt 2820
   tcatgaaggt gtaatatcgt ttttgttaaa ctgaatagaa ttgtatagcg atgacctctt 2880
   aattataatt tgatttgact gcaaaacttt ttcctcctct aagaggagat gatgtctgct 2940
   ttaagctgta atgttttgcc atgttgcaaa aagccataat aataagtata aaaaagcttt 3000
   ttcctttaca atttcatgtt aatctggttt gtctgtccac cagagacaga tcttctgtga 3060
   cagcctcctt atgcaggtct atcattattt gatagaatgt cttctaaaat acttcactca 3120
   cattgtaatt caaattagaa agtcattcca aaaggtcatg tcatgttgac ctcatttcat 3180
   cggaactgca gtatattttt gttggttaat tatattagtg ttttctattt tgaaaaaaaa 3240
   aaaaaaaaaa 3250
<210> 33
   <211> 381
   <212> DNA
   <213> Homo sapiens
<400> 33
   atgcctgagc cagcgaaatc cgctcccgcc ccgaagaagg gctccaagaa ggccgtgacc    60
   aaggcgcaga agaaggacag caagaagcgc aagcgcagcc gcaaggagag ctactccgta   120
   tacgtgtaca aggtgctgaa acaggtccac cccgacaccg gcatctcctc taaagccatg   180
   gggatcatga attcctttgt caacgacatc ttcgagcgca tcgccggcga ggcttcccgc   240
   ctggcgcatt acaacaagcg ctcgaccatc acctccaggg agatccagac ggccgtgcgc   300
   ctgctgcttc ccggggagct ggccaagcac gctgtgtcag agggcaccaa ggccgttacc   360
   aagtacacca gctccaagta a                                             381
<210> 34
   <211> 1113
   <212> DNA
   <213> Homo sapiens
<400> 34
   ggggcgacgt ttagcgacta ttgcgcctgc gccagcgccg gctgcgagac tggggccgtg 60
   gctgctggtc ccgggtgatg ctaggcggct ccctgggctc caggctgttg cggggtgtag 120
   gtgggagtca cggacggttc ggggcccgag gtgtccgcga aggtggcgca gccatggcgg 180
   caggggagag catggctcag cggatggtct gggtggacct ggagatgaca ggattggaca 240
   ttgagaagga ccagattatt gagatggcct gtctgataac tgactctgat ctcaacattt 300
   tggctgaagg tcctaacctg attataaaac aaccagatga gttgctggac agcatgtcag 360
   attggtgtaa ggagcatcac gggaggtctg gccttaccaa ggcagtgaag gagagtacaa 420
   ttacattgca gcaggcagag tatgaatttc tgtcctttgt acgacagcag actcctccag 480
   ggctctgtcc acttgcagga aattcagttc atgaagataa gaagtttctt gacaaataca 540
   tgccccagtt catgaaacat cttcattata gaataattga tgtgagcact gttaaagaac 600
   tgtgcagacg ctggtatcca gaagaatatg aatttgcacc aaagaaggct gcttctcata 660
   gggcacttga tgacattagt gaaagcatca aagagcttca gttttaccga aataacatct 720
   tcaagaaaaa aatagatgaa aagaagagga aaattataga aaatggggaa aatgagaaga 780
   ccgtgagttg atgccagtta tcatgctgcc actacatcgt tatctggagg caacttctgg 840
   tggttttttt ttctcacgct gatggcttgg cagagcacct tcggttaact tgcatctcca 900
   gattgattac tcaagcagac agcacacgaa atactatttt tctcctaata tgctgtttcc 960
   attatgacac agcagctcct ttgtaagtac caggtcatgt ccatcccttg gtacatatat 1020
   gcatttgctt ttaaaccatt tcttttgttt aaataaataa ataagtaaat aaagctagtt 1080
   ctattgaaat gcaaaaaaaa aaaaaaaaaa aaa 1113
<210> 35
   <211> 467
   <212> DNA
   <213> Homo sapiens
<400> 35
   attcttgtta tttgagtgct ctttcactct cctccgccat gcccgacccg gctaaatctg 60
   ctcctgcccc caaaaagggc tccaagaaag ccgtaaccaa ggcccagaaa aaggacggca     120
   agaagcgcaa gcgcagccgc aaagagagtt actctatcta cgtgtacaag gtgctgaagc     180
   aagtccaccc cgacaccggc atctcatcga aggccatggg catcatgaac tccttcgtca     240
   atgacatctt tgagcgcatc gctggcgagg cttcccgcct ggcgcattac aacaagcgct     300
   cgaccatcac ctccagggag atccagacgg ccgtgcgcct gctgctgccc ggggagctgg     360
   ccaagcacgc cgtgtccgag ggcacaaagg ccgtcaccaa gtacaccagc tccaagtgag     420
   ctctcgcagc tgccagcaat ccaaaggctc ttttcagagc cactcac                  467
<210> 36
   <211> 3272
   <212> DNA
   <213> Homo sapiens
<400> 36
   gggcactgct ttaaaactgg gaaggaggaa gacgaggcca gggagccgga gggtcaccaa 60
   ggtagatttc cagcagcgct agtccagctg aacactttcc agccttgttt ttcagcagct 120
   ttgaggaaaa gtatagtgat ccgtatgtga aactttcatt gtacgtagcg gatgagaata 180
   gagaacttgc tttggtccag acaaaaacaa ttaaaaagac actgaaccca aaatggaatg 240
   aagaatttta tttcagggta aacccatcta atcacagact cctatttgaa gtatttgacg 300
   aaaatagact gacacgagac gacttcctgg gccaggtgga cgtgcccctt agtcaccttc 360
   cgacagaaga tccaaccatg gagcgaccct atacatttaa ggactttctc ctcagaccaa 420
   gaagtcataa gtctcgagtt aagggatttt tgcgattgaa aatggcctat atgccaaaaa 480
   atggaggtca agatgaagaa aacagtgacc agagggatga catggagcat ggatgggaag 540
   ttgttgactc aaatgactcg gcttctcagc accaagagga acttcctcct cctcctctgc 600
   ctcccgggtg ggaagaaaaa gtggacaatt taggccgaac ttactatgtc aaccacaaca 660
   accggaccac tcagtggcac agaccaagcc tgatggacgt gtcctcggag tcggacaata 720
   acatcagaca gatcaaccag gaggcagcac accggcgctt ccgctcccgc aggcacatca 780
   gcgaagactt ggagcccgag ccctcggagg gcggggatgt ccccgagcct tgggagacca 840
   tttcagagga agtgaatatc gctggagact ctctcggtct ggctctgccc ccaccaccgg 900
   cctccccagg atctcggacc agccctcagg agctgtcaga ggaactaagc agaaggcttc 960
   agatcactcc agactccaat ggggaacagt tcagctcttt gattcaaaga gaaccctcct 1020
   caaggttgag gtcatgcagt gtcaccgacg cagttgcaga acagggccat ctaccaccgc 1080
   ccagtgcccc agctgggaga gcgcgttcat caactgtcac gggtggtgag gaaccaacgc 1140
   catcagtggc ctatgtacat accacgccgg gtctgccttc aggctgggaa gaaagaaaag 1200
   atgctaaggg gcgcacatac tatgtcaatc ataacaatcg aaccacaact tggactcgac 1260
   ctatcatgca gcttgcagaa gatggtgcgt ccggatcagc cacaaacagt aacaaccatc 1320
   taatcgagcc tcagatccgc cggcctcgta gcctcagctc gccaacagta actttatctg 1380
   ccccgctgga gggtgccaag gactcacccg tacgtcgggc tgtgaaagac accctttcca 1440
   acccacagtc cccacagcca tcaccttaca actcccccaa accacaacac aaagtcacac 1500
   agagcttctt gccacccggc tgggaaatga ggatagcgcc aaacggccgg cccttcttca 1560
   ttgatcataa cacaaagact acaacctggg aagatccacg tttgaaattt ccagtacata 1620
   tgcggtcaaa gacatcttta aaccccaatg accttggccc ccttcctcct ggctgggaag 1680
   aaagaattca cttggatggc cgaacgtttt atattgatca taatagcaaa attactcagt 1740
   gggaagaccc aagactgcag aacccagcta ttactggtcc ggctgtccct tactccagag 1800
   aatttaagca gaaatatgac tacttcagga agaaattaaa gaaacctgct gatatcccca 1860
   ataggtttga aatgaaactt cacagaaata acatatttga agagtcctat cggagaatta 1920
   tgtccgtgaa aagaccagat gtcctaaaag ctagactgtg gattgagttt gaatcagaga 1980
   aaggtcttga ctatgggggt gtggccagag aatggttctt cttactgtcc aaagagatgt 2040
   tcaaccccta ctacggcctc tttgagtact ctgccacgga caactacacc cttcagatca 2100
   accctaattc aggcctctgt aatgaggatc atttgtccta cttcactttt attggaagag 2160
   ttgctggtct ggccgtattt catgggaagc tcttagatgg tttcttcatt agaccatttt 2220
   acaagatgat gttgggaaag cagataaccc tgaatgacat ggaatctgtg gatagtgaat 2280
   attacaactc tttgaaatgg atcctggaga atgaccctac tgagctggac ctcatgttct 2340
   gcatagacga agaaaacttt ggacagacat atcaagtgga tttgaagccc aatgggtcag 2400
   aaataatggt cacaaatgaa aacaaaaggg aatatatcga cttagtcatc cagtggagat 2460
   ttgtgaacag ggtccagaag cagatgaacg cattcttgga gggattcaca gaactacttc 2520
   ctattgattt gattaaaatt tttgatgaaa atgagctgga gttgctcatg tgcggcctcg 2580
   gtgatgtgga tgtgaatgac tggagacagc attctattta caagaacggc tactgcccaa 2640
   accaccccgt cattcagtgg ttctggaagg ctgtgctact catggacgcc gaaaagcgta 2700
   tccggttact gcagtttgtc acagggacat cgcgagtacc tatgaatgga tttgccgaac 2760
   tttatggttc caatggtcct cagctgttta caatagagca atggggcagt cctgagaaac 2820
   tgcccagagc tcacacatgc tttaatcgcc ttgacttacc tccatatgaa acctttgaag 2880
   atttacgaga gaaacttctc atggccgtgg aaaatgctca aggatttgaa ggggtggatt 2940
   aagcaccctg tacctcgggg gtggttgttc ttcaagcaag ttctgcttgc acttttgcat 3000
   ttgcctaaca gacttttgca gaggcgatgg cagagagcag ctgcaggcat ggtccctgga 3060
   gccgagcctt caccacgcac tcgtccaagt tcggatgcgg gaacctggtc ccagcttgag 3120
   ttcctgcctt tcccaccaca aattatcaac tggttgatgt gtacactaat tacatttcag 3180
   gaggacttaa tgctatttat gttgtgcctc tgcaggcaaa gcccttaata aatattttac 3240
   atccttaaaa aaaaaaaaaa aaaaaaaaaa aa 3272
<210> 37
   <211> 3215
   <212> DNA
   <213> Homo sapiens
<400> 37
   gacaatatca ggtgagctgt ggaggtgggg tccttggaag ctggatgaca gcagctggca     60
   aggggataag agagcagtga gcccctccct caaggaggtc tggctttatc catagacagg    120
   gccctctgag gtggggctga ggtacaaagg gggattgagc agcccaggag aagagagatg    180
   ggggttccct tcttctcttc tctcagatgc atggtggact taggaccttg ctgggctggg    240
   ggtctcactg cagagatgaa gctgcttctg gccctagcag ggctcctggc cattctggcc    300
   acgccccagc cctctgaagg tgctgctcca gctgtcctgg gggaggtgga cacctcgttg    360
   gtgctgagct ccatggagga ggccaagcag ctggtggaca aggcctacaa ggagcggcgg    420
   gaaagcatca agcagcggct tcgcagcggc tcagccagcc ccatggaact cctatcctac    480
   ttcaagcagc cggtggcagc caccaggacg gcggtgaggg ccgctgacta cctgcacgtg    540
   gctctagacc tgctggagag gaagctgcgg tccctgtggc gaaggccatt caatgtcact    600
   gatgtgctga cgcccgccca gctgaatgtg ttgtccaagt caagcggctg cgcctaccag    660
   gacgtggggg tgacttgccc ggagcaggac aaataccgca ccatcaccgg gatgtgcaac    720
   aacagacgca gccccacgct gggggcctcc aaccgtgcct ttgtgcgctg gctgccggcg    780
   gagtatgagg acggcttctc tcttccctac ggctggacgc ccggggtcaa gcgcaacggc    840
   ttcccggtgg ctctggctcg cgcggtctcc aacgagatcg tgcgcttccc cactgatcag    900
   ctgactccgg accaggagcg ctcactcatg ttcatgcaat ggggccagct gttggaccac    960
   gacctcgact tcacccctga gccggccgcc cggcctccct tcgtcactgg cgtcaactgc    1020
   gagaccagct gcgttcagca gccgccctgc ttcccgctca agatcccgcc caatgacccc    1080
   cgcatcaaga accaagccga ctgcatcccg ttcttccgct cctgcccggc ttgccccggg    1140
   agcaacatca ccatccgcaa ccagatcaac gcgctcactt ccttcgtgga cgccagcatg    1200
   gtgtacggca gcgaggagcc cctggccagg aacctgcgca acatgtccaa ccagctgggg    1260
   ctgctggccg tcaaccagcg cttccaagac aacggccggg ccctgctgcc ctttgacaac    1320
   ctgcacgatg acccctgtct cctcaccaac cgctcagcgc gcatcccctg cttcctggca    1380
   ggggacaccc gttccagtga gatgcccgag ctcacctcca tgcacaccct cttacttcgg    1440
   gagcacaacc ggctggccac agagctcaag agcctgaacc ctaggtggga tggggagagg    1500
   ctctaccagg aagcccggaa gatcgtgggg gccatggtcc agatcatcac ttaccgggac    1560
   tacctgcccc tggtgctggg gccaacggcc atgaggaagt acctgcccac gtaccgttcc 1620
   tacaatgact cagtggaccc acgcatcgcc aacgtcttca ccaatgcctt ccgctacggc 1680
   cacaccctca tccaaccctt catgttccgc ctggacaatc ggtaccagcc catggaaccc 1740
   aacccccgtg tccccctcag cagggtcttt tttgcctcct ggagggtcgt gctggaaggt 1800
   ggcattgacc ccatcctccg gggcctcatg gccacccctg ccaagctgaa tcgtcagaac 1860
   caaattgcag tggatgagat ccgggagcga ttgtttgagc aggtcatgag gattgggctg 1920
   gacctgcctg ctctgaacat gcagcgcagc agggaccacg gcctcccagg atacaatgcc 1980
   tggaggcgct tctgtgggct cccgcagcct gaaactgtgg gccagctggg cacggtgctg 2040
   aggaacctga aattggcgag gaaactgatg gagcagtatg gcacgcccaa caacatcgac 2100
   atctggatgg gcggcgtgtc cgagcctctg aagcgcaaag gccgcgtggg cccactcctc 2160
   gcctgcatca tcggtaccca gttcaggaag ctccgggatg gtgatcggtt ttggtgggag 2220
   aacgagggtg tgttcagcat gcagcagcga caggccctgg cccagatctc attgccccgg 2280
   atcatctgcg acaacacagg catcaccacc gtgtctaaga acaacatctt catgtccaac 2340
   tcatatcccc gggactttgt caactgcagt acacttcctg cattgaacct ggcttcctgg 2400
   agggaagcct cctagaggcc aggtaagggg gtgcagcagt gaggggtata tctgggctgg 2460
   ccagttggaa ccacggagat ctccttgccc tagatgagcc cagccctgtt ctgggtgcag 2520
   ctgagaaaat gagtgactag acgttcattt gtgtgctcat gtatgtgcga agtatataaa 2580
   ttggcttttc atgcgtgtgt gttgtctgaa catggggagt gtttcatggg ttatgtgtat 2640
   gtgccattta tgtgagtgtg tgtttgtgct gatgagaata ctgagtatgt ggaaggcagc 2700
   agagcggact ggtgaggagc acagctcagg aactagactg cctgggttcc aatcctggct 2760
   ctgtggcttg ctagctatgt gaccttgagc aaattaccct ccttaaacaa gagttttctt 2820
   ccttgtaaat tacatctgtc atggtttctt ggagggccca cttgtatcct ctggttcttc 2880
   atttattgag cacctactac atgcaaggca ctgtactagg cgtgagaagc atatagaggc 2940
   aagaaagaga taccaagatg ccatctgtgt cctggttagc agagctggac cagtggtgcc 3000
   ttggagggat aagccagctg cagctgggct gtgtggttga cttatgggcc cagccagcca 3060
   ggctcaggcc atggctcccc tttttcttcc tcaccctgat ttcttgctta ttcactgaag 3120
   ttctcctgaa gaggaactgg gcctgttgcc ctttctgtac catttatttg ctcccaatgt 3180
   ttatgataat aaaggcaccg ctgatgggga cctcc 3215
<210> 38
   <211> 726
   <212> DNA
   <213> Homo sapiens
<400> 38
   gccttccttc ctgcttcgcc tccgcgcctc gcgctatggg acagagcccc cgatccgcca 60
   gcaccacctg aggatccaga aaccgcccca gcgatggaag aggatcagga gctggagaga 120
   aaaatatctg gattgaagac ctcaatggct gaaggcgaga ggaagacagc cctggaaatg 180
   gtccaggcag ctggaacaga tagacactgt gtgacatttg tattgcacga ggaagaccat 240
   accctaggaa attctctacg ttacatgatc atgaagaacc cggaagtgga attttgtggt 300
   tacactacga cccatccttc agagagcaaa attaatttac gcattcagac tcgaggtacc 360
   cttccagctg ttgagccatt tcagagaggc ctgaatgagc tcatgaatgt ctgccaacat 420
   gtgcttgaca agtttgaggc cagcataaag gactataagg atcaaaaagc aagcagaaat 480
   gaatccacat tctagtcctt tatgcagtat acaaggagaa ctgtcctgta ggatattctc 540
   ttcctgatgg tgcagaaccc agaattagaa gtttgtggtt acagcatact ctgtccttca 600
   gaaaggcgtg attctagctg ttgacccctt gcagctgttg gaatctctgc aagaacctct 660
   gtattcttct aataaattcc ctcttttatt taaaaaaaaa aaaaaaaaaa aaaaaaaaaa 720
   aaaaaa 726
<210> 39
   <211> 381
   <212> DNA
   <213> Homo sapiens
<400> 39
   atgcctgaac ctaccaagtc tgctcctgcc ccaaagaagg gctccaagaa ggcggtgact 60
   aaggctcaga agaaggacgg gaagaagcgc aagcgcagcc gcaaggagag ctattcagtg 120
   tatgtgtaca aggtgctgaa gcaggtccat cccgacaccg gcatctcttc caaggcaatg 180
   gggatcatga attccttcgt caacgacatc ttcgagcgca tcgcaggcga ggcttcccgc 240
   ctggcgcatt acaacaagcg ctcgaccatc acctccaggg agatccagac ggccgtgcgc 300
   ctgctgcttc cgggggagct ggccaagcac gccgtgtcgg agggcaccaa ggccgtcacc 360
   aagtacacca gttccaagta a 381
<210> 40
   <211> 1922
   <212> DNA
   <213> Homo sapiens
<400> 40
   agacacgtgg tccgggtgga agtgtccctg ctgcgagcag gagctcacgc tgggagggca     60
   gacacatggt cccgtggaag tgtccctgct gcaagcagga gcgctagtgc tgggagggcg    120
   gacacgtggc tccgggcaga agtgtccgcc agcaggagcg ctcgtgcttg gaaggtagac    180
   acgtggcccg ggcggaagta tccttgcagc gagcaggagc tggcgctggg agggcagaca    240
   cgtggtccgg gcggaagtgt ctgtgcagcc agcgggagct cgcgctggga gcggagacag     300
   gccctgccct gggagaagcc ctgccacacg tcgtgcccac gctgagggcc tgtctgcagc     360
   cctcccaaga cccgcagatg cgcctgaagc tgttctccat cctgtccacc gtgctgctca     420
   gagccacgga caccatcaac tcccaggggc agtttcccag ctacctcgag acggtgacaa     480
   aggacatcct ggcccccaat ctgcagtggc atgcggggag gacagccgcg gccatccgca     540
   cggctgccgt gtcctgcctc tgggcgctca ccagcagcga ggtcctgtcg gcagagcaga     600
   tacgggacgt gcaggaaaca ctgatgcccc aggtcctgac caccctggag gaggattcga     660
   agatgacgcg actgatctca tgccgtatta tcaacacgtt cttaaaaacc tcgggcggca     720
   tgacggatcc agagaaactc atcaagattt atcctgaact cttaaaacgc ctagatgacg     780
   tgtccaacga tgtgaggatg gcagccgcct ccaccttggt cacctggctg cagtgtgtca     840
   agggtgccaa cgcaaaatcc tactatcaga gcagtgtcca gtacctgtac cgagagttgc     900
   tggttcacct tgacgatcca gagagggcca tccaggatgc aattttagag gtcctcaaag     960
   agggcagcgg gctgttccca gatctcctgg tgagggagac ggaggccgtc atccacaagc    1020
   accgctcggc cacctactgc gagcagctcc tgcagcatgt gcaggccgtg ccagccacac    1080
   agtgaccacg ctggtttcag ccacggcaca cccttgtccc cacctgagcc agagtttgtg    1140
   gcctttaaat ctcataaaca aggcacctct gtgccagcag tgagactgtg acagcaagaa    1200
   tgtactcctc aggacacctg cccgctcttt ccctggaata acagcctctg agtggattct    1260
   gcatgttatg tgatttgttc tgttcatcaa gagggctccc aaacatctgc agctgatttg    1320
   aaattaaaag taagtcgcag ccgctcctcc cgcagccact tcagcagcat cttagatttt    1380
   aagcctcacg tgcgcagctg gttcatgaac tattggctgc atcctgctta ggtgcccacc    1440
   aagaaggttt ttacctactt aacaaaaaag aaagaagcca aagtgattag aaagaaatga    1500
   aatctctttt tgggttctgt ctactgaaat ttaatatctc agtgaacaga ctaaaaggaa    1560
   tttagaatcc taacaactta ccagatttct cctgttttaa atatactggg actttaaagg    1620
   ttatatgtcc ggtcaccgta tgttttaagt cggtgttaat gctaacagtg ttgaaaacaa    1680
   tatttcatga gatctaattg tggttgcccc tataggtagc aggaaagtaa agttgcattt    1740
   ccctctcgca cattctacac ccaagtgcct aaaagatctc attgtaagtg ggtagtgtta    1800
   ccggaagcca ttgtgttcac acgggggaaa tgccgtatat atttttcaac aaatattaac    1860
   gtttatactt tcatgtttga aaatttaatt aaaaatattt gttttaaaaa aaaaaaaaaa    1920
   aa                                                                   1922
<210> 41
   <211> 1421
   <212> DNA
   <213> Homo sapiens
<400> 41
   acttactgcg ggacggcctt ggagagtact cgggttcgtg aacttcccgg aggcgcaatg     60
   agctgcatta acctgcccac tgtgctgccc ggctccccca gcaagacccg ggggcagatc    120
   caggtgattc tcgggccgat gttctcagga aaaagcacag agttgatgag acgcgtccgt    180
   cgcttccaga ttgctcagta caagtgcctg gtgatcaagt atgccaaaga cactcgctac    240
   agcagcagct tctgcacaca tgaccggaac accatggagg cgctgcccgc ctgcctgctc    300
   cgagacgtgg cccaggaggc cctgggcgtg gctgtcatag gcatcgacga ggggcagttt    360
   ttccctgaca tcatggagtt ctgcgaggcc atggccaacg ccgggaagac cgtaattgtg    420
   gctgcactgg atgggacctt ccagaggaag ccatttgggg ccatcctgaa cctggtgccg    480
   ctggccgaga gcgtggtgaa gctgacggcg gtgtgcatgg agtgcttccg ggaagccgcc    540
   tataccaaga ggctcggcac agagaaggag gtcgaggtga ttgggggagc agacaagtac    600
   cactccgtgt gtcggctctg ctacttcaag aaggcctcag gccagcctgc cgggccggac    660
   aacaaagaga actgcccagt gccaggaaag ccaggggaag ccgtggctgc caggaagctc    720
   tttgccccac agcagattct gcaatgcagc cctgccaact gagggacctg caagggccgc    780
   ccgctccctt cctgccactg ccgcctactg gacgctgccc tgcatgctgc ccagccactc    840
   caggaggaag tcgggaggcg tggagggtga ccacaccttg gccttctggg aactctcctt    900
   tgtgtggctg ccccacctgc cgcatgctcc ctcctctcct acccactggt ctgcttaaag    960
   cttccctctc agctgctggg acgatcgccc aggctggagc tggccccgct tggtggcctg   1020
   ggatctggca cactccctct ccttggggtg agggacagag ccccacgctg ttgacatcag   1080
   cctgcttctt cccctctgcg gctttcactg ctgagtttct gttctccctg ggaagcctgt   1140
   gccagcacct ttgagccttg gcccacactg aggcttaggc ctctctgcct gggatgggct   1200
   cccaccctcc cctgaggatg gcctggattc acgccctctt gtttcctttt gggctcaaag   1260
   cccttcctac ctctggtgat ggtttccaca ggaacaacag catctttcac caagatgggt   1320
   ggcaccaacc ttgctgggac ttggatccca ggggcttatc tcttcaagtg tggagagggc   1380
   agggtccacg cctctgctgt agcttatgaa attaactaat t                        1421
<210> 42
   <211> 999
   <212> DNA
   <213> Homo sapiens
<400> 42
   ggcacgaggg gcgcaagccg gcaagatggc ggcggctggg gctggccgtc tgaggcgggt      60
   ggcatcggct ctgctgctgc ggagcccccg cctgcccgcc cgggagctgt cggccccggc     120
   ccgactctat cacaagaagg ttgttgatca ttatgaaaat cctagaaacg tggggtccct     180
   tgacaagaca tctaaaaatg ttggaactgg actggtgggg gctccagcat gtggtgacgt     240
   aatgaaatta cagattcaag tggatgaaaa ggggaagatt gtggatgcta ggtttaaaac     300
   atttggctgt ggttccgcaa ttgcctccag ctcattagcc actgaatggg tgaaaggaaa     360
   gacggtggag gaagccttga ctatcaaaaa cacagatatc gccaaggagc tctgccttcc     420
   tcccgtgaaa ctgcactgct ccatgctggc tgaagatgca atcaaggccg ccctggctga     480
   ttacaaattg aaacaagaac ccaaaaaagg agaggcagag aagaaatgag ccctccctcg     540
   gcgaagcctc cagcaggcca caccagctgt ttcccacctg ctgtgcagtc accttagatg     600
   ttcagaagcc gcttcctctc cactgaagag ctatgagata cgcacaatac ttgctgttca     660
   cgttatgact ctcatgcaag caaaatacac agtttcattg ttctgaatcc tgtggtttct     720
   ttcagcccac ttttatcgcc ttaacctagt taatgtatat tttgaattgt gtgtatgacc     780
   tcagaactga aattgataat gaagttgcaa gttttgatag cccgtgaagt gcataagtat     840
   ctaattttac ctgaattgat ttggggggaa attaccagta gaatgccttg gtctgaatat     900
   ttgatagaac caattgttgt acataaaaca gatctgcgca tatatatata tgtataaaaa     960
   ataataaaat aatggaagat gaaaaaaaaa aaaaaaaaa                            999
<210> 43
   <211> 487
   <212> DNA
   <213> Homo sapiens
<400> 43
   actcactttc tgacttaggc cacaggtcgt tttaccatgt ctggacgtgg caagcagggc 60
   ggcaaggctc gcgccaaggc caaaacccgc tcctctagag ctgggctcca atttcctgta 120
   ggacgagtgc accgcctgct ccgcaagggc aactacgctg agcgggtcgg ggccggcgcg 180
   ccggtttacc tggcggcggt gctggagtac ctaactgccg agatcctgga gctggcgggc 240
   aacgcagccc gcgacaacaa aaagacccgc atcatcccgc gccacttgca gctggccatc 300
   cgcaacgacg aggagctcaa caagctgctt ggtaaagtta ccatcgctca gggcggtgtt 360
   ctgcctaaca tccaggccgt actgctcccc aagaagactg agagccacca caaagctaag 420
   ggcaagtaag ggctgaactt taaaaatgta aacttacaag acaaaaggct cttttcagag 480
   ccaccca 487
<210> 44
   <211> 833
   <212> DNA
   <213> Homo sapiens
<400> 44
   ggccacccgc ctttcactat ccgccattct tgtcacctca gctgctgccc tcgctaccgc 60
   accgacttcg cccgtgtgct cgcctgcact tgcgctgccc gccatggcca ccgcccagcc 120
   gtcgcaggtg cgccagaagt acgacaccaa ctgcgacgcc gccatcaaca gccacatcac 180
   gctggagctc tacacctcct acctgtacct gtctatggcc ttctacttca accgggacga 240
   cgtggccctg gagaacttct tccgctactt cctgcgcctg tcggacgaca aaatggagca 300
   tgcccagaag ctgatgaggc tgcagaacct gcgcggtggc cacatctgcc ttcacgatat 360
   caggaagcca gagtgccaag gctgggagag cgggctcgtg gccatggagt ccgccttcca 420
   cctggagaag aacgtcaacc agagcctgct ggatctgtac cagctggccg tggagaaggg 480
   cgacccccag ctgtgccact tcctggagag ccactacctg cacgagcaag tcaagaccat 540
   caaagagctg ggtggctacg tgagcaacct gcgcaagatt tgttccccgg aagccggcct 600
   ggctgagtac ctgttcgaca agctcaccct gggcggccgc gtcaaagaga cttgagccca 660
   gatgggcccc acagccacgg ggtcccttcc ctgggtcagg ccactaggcg gggcgtgcat 720
   gttgcccttt cagaacgttc tcttcagttt tatctttcag ttttaccatt gttagcaaaa 780
   aagttatctg gttctcaaag caataaaggt gtccataaaa aaaaaaaaaa aaa 833
<210> 45
   <211> 7149
   <212> DNA
   <213> Homo sapiens
<400> 45
   atgtctggcg gcgccgcaga gaagcagagc agcactcccg gttccctgtt cctctcgccg 60
   ccggctcctg cccccaagaa tggctccagc tccgattcct ccgtggggga gaaactggga 120
   gccgcggccg ccgacgctgt gaccggcagg accgaggagt acaggcgccg ccgccacact 180
   atggacaagg acagccgtgg ggcggccgcg accactacca ccactgagca ccgcttcttc 240
   cgccggagcg tcatctgcga ctccaatgcc actgcgctgg agcttcccgg ccttcctctt 300
   tccctgcccc agcccagcat ccccgcggct gtcccgcaga gtgctccacc ggagccccac 360
   cgggaagaga ccgtgaccgc caccgccact tcccaggtag cccagcagcc tccagccgct 420
   gccgcccctg gggaacaggc cgtcgcgggc cctgccccct cgactgtccc cagcagtacc 480
   agcaaagacc gcccagtgtc ccagcctagc cttgtgggga gcaaagagga gccgccgccg 540
   gcgagaagtg gcagcggcgg cggcagcgcc aaggagccac aggaggaacg gagccagcag 600
   caggatgata tcgaagagct ggagaccaag gccgtgggaa tgtctaacga tggccgcttt 660
   ctcaagtttg acatcgaaat cggcagaggc tcctttaaga cggtctacaa aggtctggac 720
   actgaaacca ccgtggaagt cgcctggtgt gaactgcagg atcgaaaatt aacaaagtct 780
   gagaggcaga gatttaaaga agaagctgaa atgttaaaag gtcttcagca tcccaatatt 840
   gttagatttt atgattcctg ggaatccaca gtaaaaggaa agaagtgcat tgttttggtg 900
   actgaactta tgacgtctgg aacacttaaa acgtatctga aaaggtttaa agtgatgaag 960
   atcaaagttc taagaagctg gtgccgtcag atccttaaag gtcttcagtt tcttcatact 1020
   cgaactccac ctatcattca ccgcgatctt aaatgtgaca acatctttat caccggccct 1080
   actggctcag tcaagattgg agacctcggt ctggcaaccc tgaagcgggc ttcttttgcc 1140
   aagagtgtga taggtacccc agagttcatg gcccctgaga tgtatgagga gaaatatgat 1200
   gaatccgttg acgtttatgc ctttgggatg tgcatgcttg agatggctac atctgaatat 1260
   ccttactcgg agtgccaaaa tgctgcgcag atctaccgtc gcgtgaccag tggggtgaag 1320
   ccagccagtt ttgacaaagt agcaattcct gaagtgaagg aaattattga aggatgcata 1380
   cgacaaaaca aagatgaaag atattccatc aaagaccttt tgaaccatgc cttcttccaa 1440
   gaggaaacag gagtacgggt agaattagca gaggaagatg atggagaaaa aatagccata 1500
   aaattatggc tacgtattga agatattaag aaattaaagg gaaaatacaa agataatgaa 1560
   gctattgagt tttcttttga tttagagaga gatgtcccag aagatgttgc acaagaaatg 1620
   gtagagtctg ggtatgtctg tgaaggtgat cacaagacca tggctaaagc tatcaaagac 1680
   agagtatcat taattaagag gaaacgagag cagcggcagt tggtacggga ggagcaagaa 1740
   aaaaaaaagc aggaagagag cagtctcaaa cagcaggtag aacaatccag tgcttcccag 1800
   acaggaatca agcagctccc ttctgctagc accggcatac ctactgcttc taccacttca 1860
   gcttcagttt ctacacaagt agaacctgaa gaacctgagg cagatcaaca tcaacaacta 1920
   cagtaccagc aacccagtat atctgtgtta tctgatggga cggttgacag tggtcaggga 1980
   tcctctgtct tcacagaatc tcgagtgagc agccaacaga cagtttcata tggttcccaa 2040
   catgaacagg cacattctac aggcacagtc ccagggcata taccttctac tgtccaagca 2100
   cagtctcagc cccatggggt atatccaccc tcaagtgtgg cacaggggca gagccagggt 2160
   cagccatcct caagtagctt aacaggggtt tcatcttccc aacccataca acatcctcag 2220
   cagcagcagg gaatacagca gacagcccct cctcaacaga cagtgcagta ttcactttca 2280
   cagacatcaa cctccagtga ggccactact gcacagccag tgagtcagcc tcaagctcca 2340
   caagtcttgc ctcaagtatc agctggaaaa cagcttccag tttcccagcc agtaccaact 2400
   atccaaggcg aacctcagat cccagttgcg acacaaccct cggttgttcc agtccactct 2460
   ggtgctcatt tccttccagt gggacagccg ctccctactc ccttgctccc tcagtaccct 2520
   gtctctcaga ttcccatatc aactcctcat gtgtctacgg ctcagacagg tttctcatcc 2580
   cttcccatca caatggcagc tggcattact cagcctctgc tcacgttggc ttcatctgct 2640
   acaacagctg cgatcccggg ggtatcaact gtggttccta gtcagcttcc aacccttctg 2700
   cagcctgtga ctcagctgcc aagtcaggtt cacccacagc tcctacaacc agcagttcag 2760
   tccatgggaa taccagctaa ccttggacaa gctgctgagg ttccactttc ctctggagat 2820
   gttctgtacc agggcttccc acctcgactg ccaccacagt acccaggaga ttcaaatatt 2880
   gctccctctt ccaacgtggc ttctgtttgc atccattcta cagtcctatc ccctcccatg 2940
   ccgacagaag tactggctac acctgggtac tttcccacag tggtgcagcc ttatgtggaa 3000
   tcaaatcttt tagttcctat gggtggtgta ggaggacagg ttcaagtgtc ccagccagga 3060
   gggagtttag cacaagcccc cactacatcc tcccagcaag cagttttgga gagtactcag 3120
   ggagtctctc aggttgctcc tgcagagcca gttgcagtag cacagcccca agctacccag 3180
   ccgaccactt tggcttcctc tgtagacagt gcacattcag atgttgcttc aggtatgagt 3240
   gatggcaatg agaacgtccc atcttccagt ggaaggcatg aaggaagaac tacaaaacgg 3300
   cattaccgaa aatctgtaag gagtcgctct cgacatgaaa aaacttcacg cccaaaatta 3360
   agaattttga atgtttcaaa taaaggagac cgagtagtag aatgtcaatt agagactcat 3420
   aataggaaaa tggttacatt caaatttgac ctagatggtg acaaccccga ggagatagca 3480
   acaattatgg tgaacaatga ctttattcta gcaatagaga gagagtcgtt tgtggatcaa 3540
   gtgcgagaaa ttattgaaaa agctgatgaa atgctcagtg aggatgtcag tgtggaacca 3600
   gagggtgatc agggattgga gagtctacaa ggaaaggatg actatggctt ttcaggttct 3660
   cagaaattgg aaggagagtt caaacaacca attcctgcgt cttccatgcc acagcaaata 3720
   ggcattccta ccagttcttt aactcaagtt gttcattctg cgggaaggcg gtttatagtg 3780
   agtcctgtgc cagaaagccg attacgagaa tcaaaagttt tccccagtga aataacagat 3840
   acagttgctg cctctacagc tcagagccct ggaatgaact tgtctcactc tgcatcatcc 3900
   cttagtctac aacaggcctt ttctgaactt agacgtgccc aaatgacaga aggacccaac 3960
   acagcacctc caaactttag tcatacagga ccaacatttc cagtagtacc tcctttctta 4020
   agtagcattg ctggagtccc aaccacagca gcagccacag caccagtccc tgcaacaagc 4080
   agccctccta atgacatttc cacatcagta attcagtctg aggttacagt gcccactgaa 4140
   gaggggattg ctggagttgc caccagcaca ggtgtggtaa cttcaggtgg tctccccata 4200
   ccacctgtgt ctgaatcacc agtactttcc agcgtagttt caagtatcac aatacctgca 4260
   gttgtctcaa tatctactac atccccgtca cttcaagtcc ccacatccac atctgagatc 4320
   gttgtttcta gtacagcact gtatccttca gtaacagttt cagcaacttc agcctctgca 4380
   gggggcagta ctgctacccc aggtcctaag cctccagctg tagtatctca gcaggcagca 4440
   ggcagcacta ctgtgggagc cacattaaca tcagtttcta ccaccacttc attcccaagc 4500
   acagcttcac agctgtccat tcagcttagc agcagtactt ctactcctac tttagctgaa 4560
   accgtggtag ttagcgcaca ctcactagat aagacatctc atagcagtac aactggattg 4620
   gctttctccc tctctgcacc atcttcctct tcctctcctg gagcaggagt gtctagttat 4680
   atttctcagc ctggtgggct gcatcctttg gtcattccat cagtgatagc ttctactcct 4740
   attcttcccc aagcagcagg acctacttct acacctttat taccccaagt acctagtatc 4800
   ccacccttgg tacagcctgt tgccaatgtg cctgctgtac agcagacact aattcatagt 4860
   cagcctcaac cagctttgct tcccaaccag ccccatactc attgtcctga agtagattct 4920
   gatacacaac ccaaagctcc tggaattgat gacataaaga ctctagaaga aaagctgcgg 4980
   tctctgttca gtgaacacag ctcatctgga gctcagcatg cctctgtctc actggagacc 5040
   tcactagtca tagagagcac tgtcacacca ggcatcccaa ctactgctgt tgcaccaagc 5100
   aaactcctga cttctaccac aagtacttgc ttaccaccaa ccaatttacc actaggaaca 5160
   gttgctttgc cagttacacc agtggtcaca cctgggcaag tttctacccc agtcagcact 5220
   actacatcag gagtgaaacc tggaactgct ccctccaagc cacctctaac taaggctccg 5280
   gtgctgccag tgggtactga acttccagca ggtactctac ccagcgagca gctgccacct 5340
   tttccaggac cttctctaac ccagtcccag caacctctag aggatcttga tgctcaattg 5400
   agaagaacac ttagtccaga gattatcaca gtgacttctg cggttggtcc tgtgtccatg 5460
   gcggctccaa cagcaatcac agaagcagga acacagcctc agaagggtgt ttctcaagtc 5520
   aaagaaggcc ctgtcctagc aactagttca ggagctggtg tttttaagat gggacgattt 5580
   caggtttctg ttgcagcaga cggtgcccag aaagagggta aaaataagtc agaagatgca 5640
   aagtctgttc attttgaatc cagcacctca gagtcctcag tgctatcaag tagtagtcca 5700
   gagagtacct tggtgaaacc agagccgaat ggcataacca tccctggtat ctcttcagat 5760
   gtgccagaga gtgcccacaa aactactgcc tcagaggcaa agtcagacac tgggcagcct 5820
   accaaggttg gacgttttca ggtgacaact acagcaaaca aagtgggtcg tttctctgta 5880
   tcaaaaactg aggacaagat cactgacaca aagaaagaag gaccagtggc atctcctcct 5940
   tttatggatt tggaacaagc tgttcttcct gctgtgatac caaagaaaga gaagcctgaa 6000
   ctgtcagagc cttcacatct aaatgggccg tcttctgacc cggaggccgc ttttttaagt 6060
   agggatgtgg atgatggttc cggtagtcca cactcgcccc atcagctgag ctcaaagagc 6120
   cttcctagcc agaatctaag tcaaagcctt agtaattcat ttaactcctc ttacatgagt 6180
   agcgacaatg agtcagatat cgaagatgaa gacttaaagt tagagctgcg acgactacga 6240
   gataaacatc tcaaagagat tcaggacctg cagagtcgcc agaagcatga aattgaatct 6300
   ttgtatacca aactgggcaa ggtgccccct gctgttatta ttcccccagc tgctcccctt 6360
   tcagggagaa gacgacgacc cactaaaagc aaaggcagca aatctagtcg aagcagttcc 6420
   ttggggaata aaagccccca gctttcaggt aacctgtctg gtcagagtgc agcttcagtc 6480
   ttgcaccccc agcagaccct ccaccctcct ggcaacatcc cagagtccgg gcagaatcag 6540
   ctgttacagc cccttaagcc atctccctcc agtgacaacc tctattcagc cttcaccagt 6600
   gatggtgcca tttcagtacc aagcctttct gctccaggtc aaggaaccag cagcacaaac 6660
   actgttgggg caacagtgaa cagccaagcc gcccaagctc agcctcctgc catgacgtcc 6720
   agcaggaagg gcacattcac agatgacttg cacaagttgg tagacaattg ggcccgagat 6780
   gccatgaatc tctcaggcag gagaggaagc aaagggcaca tgaattacga gggccctgga 6840
   atggcaagga agttctctgc acctgggcaa ctgtgcatct ccatgacctc gaacctgggt 6900
   ggctctgccc ccatctctgc agcatcagct acctctctag gtcacttcac caagtctatg 6960
   tgccccccac agcagtatgg ctttccagct accccatttg gcgctcaatg gagtgggacg 7020
   ggtggcccag caccacagcc acttggccag ttccaacctg tgggaactgc ctccttgcag 7080
   aatttcaaca tcagcaattt gcagaaatcc atcagcaacc ccccaggctc caacctgcgg 7140
   accacttag 7149
<210> 46
   <211> 2168
   <212> DNA
   <213> Homo sapiens
<400> 46
   ggcgcgcgtg aacgcggtcc ccgggaccat gctgcggcca cagcggcccg gagacttgca 60
   gctcggggcc tccctctacg agctggtggg ctacaggcag ccgccctcct cctcctcctc 120
   ctccacctcc tccacctcct ccacttcctc ctcctccacg acggcccccc tcctccccaa 180
   ggctgcgcgc gagaagccgg aggcgccggc cgagcctcca ggccccgggc ccgggtcagg 240
   cgcgcacccg ggcggcagcg cccggccgga cgccaaggag gagcagcagc agcagctgcg 300
   gcgcaagatc aacagccgcg agcggaagcg catgcaggac ctgaacctgg ccatggacgc 360
   cctgcgcgag gtcatcctgc cctactcagc ggcgcactgc cagggcgcgc ccggccgcaa 420
   gctctccaag atagccacgc tgctgctcgc ccgcaactac atcctactgc tgggcagctc 480
   gctgcaggag ctgcgccgcg cgctgggcga gggcgccggg cccgccgcgc cgcgcctgct 540
   gctggccggg ctgcccctgc tcgccgccgc gcccggctcc gtgttgctgg cgcccggcgc 600
   cgtaggaccc cccgacgcgc tgcgccccgc caagtacctg tcgctggcgc tggacgagcc 660
   gccgtgcggc cagttcgctc tccccggcgg cggcgcaggc ggccccggcc tctgcacctg 720
   cgccgtgtgc aagttcccgc acctggtccc ggccagcctg ggcctggccg ccgtgcaggc 780
   gcaattctcc aagtgagggc gggcctgggc ctggggcgcg acctcggccc ggcctccctt 840
   cgctcagctt ctccgcgccc ctgctccctg cgtctgggag agcgaggccg agcaaggaaa 900
   gcatttcgaa ccttccagtc cagaggaagg gactgtcggg cacccccttc cccgccccca 960
   cccctgggac gttaaagtga ccagagcgga tgttcgatgg cgcctcgggg cagtttgggg 1020
   ttctgggtcg gttccagcgg ctttaggcag aaagtgctcg ctctcaccca gcacatctct 1080
   ctccttgtcc ctggagttgc gcgcttcgcg gggccgatgt agaacttagg gcgccttgcc 1140
   gtggttggcg cgccccgggt gcagcgagag gccatccccg agcgctatct ccccggagcg 1200
   gagcacgccg gctcccagta ctaggggctg cgctcgagca gtggcggggg cggaggggtg 1260
   gttcttttcc ttctcctccg ccagaggcca cgggcgccct tgttcccgcc ggccaggtcc 1320
   tatcaaagga ggctgccgga actcaagagg cagaaaaaga ccagttaggc ggtgcagacg 1380
   gtctgggacg tggcagacgg acggaccctc ggcggacagg tggtcggcgt cggggtgcgg 1440
   tgggtagggg cgaggacaac gcagggtgcg ctgggttggg acgtgggtcc acttttgtag 1500
   accagctgtt tggagagctg tatttaagac tcgcgtatcc agtgttttgt cgcagagagt 1560
   tttcgctctt aaatcctggg ggtttcttag aaagcaactt agaactcgag attcaccttt 1620
   cgtttccctt tccccaaaag tagcgtaacc aacatttaag cttgcttaaa aacgaaaacc 1680
   aaccgccttg catccagtgt tcccgattta ctaaaatagg taaccaggcg tctcacagtc 1740
   gccgtcctgt caagagcgct aatgaacgtt ctcattaaca cgcaggagta ccgggagccc 1800
   tgaaccgccc gctgctcggc ggatcccagc tgcggtggcg acggcgggaa ggcgctttcc 1860
   gctgttcctc agcgggccgg gcccttgacc agcgcggccc gcaggtcttc cttctcgccg 1920
   tcttgcagtt gaagagctac atacgtagtc agtttcgatt tgttacagac gttaacaaat 1980
   tcctttaccc aaggttatgc tatgaccttt ccgcagttta ctttgatttt ctatgtttaa 2040
   ggttttggtt gttggtagta gccgaattta actggcactt tattttactt ctaaccttgt 2100
   ttcctgacgg tgtacagaat caacaaaata aaacatttaa agtctgattt tttaaaaaaa 2160
   aaaaaaaa 2168
<210> 47
   <211> 1936
   <212> DNA
   <213> Homo sapiens
<400> 47
   gcagaggcgc aggtagatgg agttggggag ttgcctggag ggcgggaggg aggcggcgga     60
   ggaagagggc gagcctgagg tgaaaaagcg gcgacttctg tgtgtgaggt ttgcctcggt    120
   cgcaagctgc gatgccgcag tggctcagtg cttcctggcc gagaacgact gggagatgga    180
   aagggctctg aactcctact tcgagcctcc ggtggaggag agcgccttgg aacgccgacc    240
   tgaaaccatc tctgagccca agacctatgt tgacctaacc aatgaagaaa caactgattc    300
   caccacttct aaaatcagcc catctgaaga tactcagcaa gaaaatggca gcatgttctc    360
   tctcattacc tggaatattg atggattaga tctaaacaat ctgtcagaga gggctcgagg    420
   ggtgtgttcc tacttagctt tgtacagccc agatgtgata tttctacagg aagttattcc    480
   cccatattat agctacctaa agaagagatc aagtaattat gagattatta caggtcatga    540
   agaaggatat ttcacagcta taatgttgaa gaaatcaaga gtgaaattaa aaagccaaga 600
   gattattcct tttccaagta ccaaaatgat gagaaacctt ttatgtgtgc atgtgaacgt 660
   gtcaggaaat gagctttgcc ttatgacatc ccatttggag agcaccagag ggcatgctgc 720
   ggaacgaatg aatcagttaa aaatggtttt aaagaaaatg caagaggctc cagagtcagc 780
   tacagttata tttgcaggag atacaaatct aagggatcga gaggttacca gatgtggtgg 840
   tttacccaac aacattgtgg atgtctggga gtttttgggc aaacctaaac attgccagta 900
   tacatgggat acacaaatga actctaatct tggaataact gctgcttgta aacttcgttt 960
   tgatcgaata tttttcagag cagcagcaga agagggacac attattcccc gaagtttgga 1020
   ccttcttgga ttagaaaaac tggactgtgg tagatttcct agtgatcact ggggtcttct 1080
   gtgcaactta gatataatat tgtaaaatgc ttttcaagtg tgggttttgc cctgattgtt 1140
   gcaaatacaa tttccacctt ctggaaaggt aggtttgctg tggaggaaat aatgtactag 1200
   atcattgtca cagaaaaacc aactatgatt tatggttgtg ttttcagaat tcaacattaa 1260
   agattaatgt ttatttaaac gaacacattc ctgcattcag gatgtgaggc catttaataa 1320
   aaagggcaca aagcctgtca gagttttcaa cggtgcttat agctgccagc tggattccaa 1380
   acaggtaccc cattgtctct gagctaatgt ttatattttt ccattcaggc accgaaatag 1440
   ttaatattta aaataagtct tcaaaagaaa acataagaga ttattgagtt cttgggactg 1500
   gatcctttat ttcataagtt cagatcatct taaatgaaaa tgccatgatt atctgcagtt 1560
   aagtagatga cagctattct acatcagact tgatttttgt cagctaatta cataattggt 1620
   aagctataat tgaaacctta tggcttaaaa ttccttaact cctttttgat tcatgtttgt 1680
   agtcatgttg tcaacagagg caaagttaag cttgatgatg gttaaaatcg gtttgatagc 1740
   accatgggac atttttctaa caaaaataaa tgcatgaaga gacatagcct tttagttttg 1800
   ctaattgtga aatggaaatg ctttacagga agtaaatgca aattactttt aagtgtgctt 1860
   taaagaaaaa tattttcccc acaagagaaa tttaaataaa gaattttatt tgtttaaaaa 1920
   aaaaaaaaaa aaaaaa 1936
<210> 48
   <211> 494
   <212> DNA
   <213> Homo sapiens
<400> 48
   tgtggttgct cgtagtgagt tgcgctcgct atgtctggac gtggcaagca gggaggcaaa 60
   gcccgcgcta aggccaagac tcgctcttct agggccggtc tccagttccc cgtgggccga 120
   gtgcaccgcc tgctccgcaa aggcaactat gccgagcggg tcggggccgg cgcgccggtg 180
   tatctggcag cggtgctgga gtacctgacc gccgagatcc tggaactggc gggcaacgcg 240
   gcccgcgaca acaagaagac ccgcatcatc ccgcgtcatc tccaactggc catccgcaac 300
   gacgaggagc tcaacaagct gctgggcaaa gtcaccatcg cacagggcgg tgtcctgccc 360
   aacattcagg ccgtgctact gcccaaaaag actgagagcc accacaaggc gaagggcaag 420
   taactatctg tactagtttg tggcagctca agtaaaatcg agtccaaacc aacggctctt 480
   ttcagggcca ccca 494
<210> 49
   <211> 1152
   <212> DNA
   <213> Homo sapiens
<400> 49
   tcagagttca cgaggcagcc gaggaagagg aggcttgagg cccagggtgg gcaccagcca      60
   gccatggcca cagccgagac cgccttgccc tccatcagca cactgaccgc cctgggcccc     120
   ttcccggaca cacaggatga cttcctcaag tggtggcgct ccgaagaggc gcaggacatg     180
   ggcccgggtc ctcctgaccc cacggagccg cccctccacg tgaagtctga ggaccagccc     240
   ggggaggaag aggacgatga gaggggcgcg gacgccacct gggacctgga tctcctcctc     300
   accaacttct cgggcccgga gcccggtggc gcgccccaga cctgcgctct ggcgcccagc     360
   gaggcctccg gggcgcaata tccgccgccg cccgagactc tgggcgcata tgctggcggc      420
   ccggggctgg tggctgggct tttgggttcg gaggatcact cgggttgggt gcgccctgcc     480
   ctgcgagccc gggctcccga cgccttcgtg ggcccagccc tggctccagc cccggccccc     540
   gagcccaagg cgctggcgct gcaaccggtg tacccggggc ccggcgccgg ctcctcgggt     600
   ggctacttcc cgcggaccgg gctttcagtg cctgcggcgt cgggcgcccc ctacgggcta     660
   ctgtccgggt accccgcgat gtacccggcg cctcagtacc aagggcactt ccagctcttc     720
   cgcgggctcc agggacccgc gcccggtccc gccacgtccc cctccttcct gagttgtttg     780
   ggacccggga cggtgggcac tggactcggg gggactgcag aggatccagg tgtgatagcc     840
   gagaccgcgc catccaagcg aggccgacgt tcgtgggcgc gcaagaggca ggcagcgcac     900
   acgtgcgcgc acccgggttg cggcaagagc tacaccaaga gctcccacct gaaggcgcat     960
   ctgcgcacgc acacagggga gaagccatac gcctgcacgt gggaaggctg cggctggaga    1020
   ttcgcgcgct cggacgagct gacccgccac taccggaaac acacggggca gcgccccttc    1080
   cgctgccagc tctgcccacg tgctttttcg cgctctgacc acctggcctt gcacatgaag    1140
   cgccaccttt ga                                                        1152
<210> 50
   <211> 1362
   <212> DNA
   <213> Homo sapiens
<400> 50
   agcaactcca aggacacagt tcacagaaat ttggttctca gccccaaaat actgattgaa     60
   ttggagacaa ttacaaggac tctctggcca aaaacccttg aagaggcccc gtgaaggagg    120
   cagtgaggag cttttgattg ctgacctgtg tcgtaccacc ccagaatgtg cactgggggc    180
   tgtgccagat gcctgggggg gaccctcatt ccccttgctt tttttggctt cctggctaac    240
   atcctgttat tttttcctgg aggaaaagtg atagatgaca acgaccacct ttcccaagag    300
   atctggtttt tcggaggaat attaggaagc ggtgtcttga tgatcttccc tgcgctggtg    360
   ttcttgggcc tgaagaacaa tgactgctgt gggtgctgcg gcaacgaggg ctgtgggaag    420
   cgatttgcga tgttcacctc cacgatattt gctgtggttg gattcttggg agctggatac    480
   tcgtttatca tctcagccat ttcaatcaac aagggtccta aatgcctcat ggccaatagt    540
   acatggggct accccttcca cgacggggat tatctcaatg atgaggcctt atggaacaag    600
   tgccgagagc ctctcaatgt ggttccctgg aatctgaccc tcttctccat cctgctggtc    660
   gtaggaggaa tccagatggt tctctgcgcc atccaggtgg tcaatggcct cctggggacc    720
   ctctgtgggg actgccagtg ttgtggctgc tgtgggggag atggacccgt ttaaacctcc    780
   gagatgagct gctcagactc tacagcatga cgactacaat ttcttttcat aaaacttctt    840
   ctcttcttgg aattattaat tcctatctgc ttcctagctg ataaagctta gaaaaggcag    900
   ttattccttc tttccaacca gctttgctcg agttagaatt ttgttatttt caaataaaaa    960
   atagtttggc cacttaacaa atttgattta taaatctttc aaattagttc ctttttagaa   1020
   tttaccaaca ggttcaaagc atacttttca tgattttttt attacaaatg taaaatgtat   1080
   aaagtcacat gtactgccat actacttctt tgtatataaa gatgtttata tctttggaag   1140
   ttttacataa atcaaaggaa gaaagcacat ttaaaatgag aaactaagac caatttctgt   1200
   ttttaagagg aaaaagaatg attgatgtat cctaagtatt gttatttgtt gtcttttttt   1260
   gctgccttgc ttgagttgct tgtgactgat cttttgaggc tgtcatcatg gctagggttc   1320
   ttttatgtat gttaaattaa aacctgaatt cagaggtaac gt                      1362
<210> 51
   <211> 2088
   <212> DNA
   <213> Homo sapiens
<400> 51
   gaattcggca cgagcgcgcg gcgaatctca acgctgcgcc gtctgcgggc gcttccgggc     60
   caccagtttc tctgctttcc accctggcgc cccccagccc tggctcccca gctgcgctgc    120
   cccgggcgtc cacgccctgc gggcttagcg ggttcagtgg gctcaatctg cgcagcgcca    180
   cctccatgtt gaccaagcct ctacaggggc ctcccgcgcc ccccgggacc cccacgccgc    240
   cgccaggagg caaggatcgg gaagcgttcg aggccgagta tcgactcggc cccctcctgg    300
   gtaagggggg ctttggcacc gtcttcgcag gacaccgcct cacagatcga ctccaggtgg     360
   ccatcaaagt gattccccgg aatcgtgtgc tgggctggtc ccccttgtca gactcagtca     420
   catgcccact cgaagtcgca ctgctatgga aagtgggtgc aggtggtggg caccctggcg    480
   tgatccgcct gcttgactgg tttgagacac aggaaggctt catgctggtc ctcgagcggc    540
   ctttgcccgc ccaggatctc tttgactata tcacagagaa gggcccactg ggtgaaggcc    600
   caagccgctg cttctttggc caagtagtgg cagccatcca gcactgccat tcccgtggag    660
   ttgtccatcg tgacatcaag gatgagaaca tcctgataga cctacgccgt ggctgtgcca    720
   aactcattga ttttggttct ggtgccctgc ttcatgatga accctacact gactttgatg    780
   ggacaagggt gtacagcccc ccagagtgga tctctcgaca ccagtaccat gcactcccgg    840
   ccactgtctg gtcactgggc atcctcctct atgacatggt gtgtggggac attccctttg    900
   agagggacca ggagattctg gaagctgagc tccacttccc agcccatgtc tccccagact    960
   gctgtgccct aatccgccgg tgcctggccc ccaaaccttc ttcccgaccc tcactggaag   1020
   agatcctgct ggacccctgg atgcaaacac cagccgagga tgttacccct caacccctcc   1080
   aaaggaggcc ctgccccttt ggcctggtcc ttgctaccct aagcctggcc tggcctggcc   1140
   tggcccccaa tggtcagaag agccatccca tggccatgtc acagggatag atggacattt   1200
   gttgacttgg ttttacaggt cattaccagt cattaaagtc cagtattact aaggtaaggg   1260
   attgaggatc aggggttaga agacataaac caagtttgcc cagttccctt cccaatccta   1320
   caaaggagcc ttcctcccag aacctgtggt ccctgatttt ggagggggaa cttcttgctt   1380
   ctcattttgc taaggaagtt tattttggtg aagttgttcc cattttgagc cccgggactc   1440
   ttattttgat gatgtgtcac cccacattgg cacctcctac taccaccaca caaacttagt   1500
   tcatatgctt ttacttgggc aagggtgctt tccttccaat accccagtag cttttatttt   1560
   agtaaaggga ccctttcccc tagcctaggg tcccatattg ggtcaagctg cttacctgcc   1620
   tcagcccagg attttttatt ttgggggagg taatgccctg ttgttacccc aaggcttctt   1680
   tttttttttt tttttttttg ggtgagggga ccctactttg ttatcccaag tgctcttatt   1740
   ctggtgagaa gaaccttaat tccataattt gggaaggaat ggaagatgga caccaccgga   1800
   caccaccaga caataggatg ggatggatgg ttttttgggg gatgggctag gggaaataag   1860
   gcttgctgtt tgttttcctg gggcgctccc tccaattttg cagatttttg caacctcctc   1920
   ctgagccggg attgtccaat tactaaaatg taaataatca cgtattgtgg ggaggggagt   1980
   tccaagtgtg ccctcctttt ttttcctgcc tggattattt aaaaagccat gtgtggaaac   2040
   ccactattta ataaaagtaa tagaatcaga aaaaaaaaaa aaaaaaaa                  2088
<210> 52
   <211> 735
   <212> DNA
   <213> Homo sapiens
<400> 52
   agtggttctc cgcccctgcc actgggccat ggagactgtg gcacagtaga ctgtagtgtg      60
   aggctcgcgg gggcagtggc catggaggcc gtgctgaacg agctggtgtc tgtggaggac     120
   ctgctgaagt ttgaaaagaa atttcagtct gagaaggcag caggctcggt gtccaagagc     180
   acgcagtttg agtacgcctg gtgcctggtg cggacaaggt acaatgatga catccgtaaa     240
   ggcatcgtgc tgctcgagga gctgctgccc aaagggagca aggaggaaca gcgggattac     300
   gtcttctacc tggccgtggg gaactaccgg ctcaaggaat acgagaaggc cttaaagtac     360
   gtccgcgggt tgctgcagac agagccccag aacaaccagg ccaaggaact ggagcggctc     420
   attgacaagg ccatgaagaa agatggactc gtgggcatgg ccatcgtggg aggcatggcc     480
   ctgggtgtgg cgggactggc cggactcatc ggacttgctg tgtccaagtc caaatcctga     540
   aggagacgcg ggagcccacg gagaacgctc caggagggcc tgtccatcct cgctgtcctt     600
   tccctgttct ccccctgccc cccgtctcta tcctctgtgg ccttcagcta atttctgctc     660
   ccctgagatt cgtccttcag ccccatcatg tgctttggga tgagtgtaaa taaaacgggg     720
   ctgtggcttg ggaaa                                                       735
<210> 53
   <211> 2627
   <212> DNA
   <213> Homo sapiens
<400> 53
   gctgacgcct tcgagcgcgg cccggggccc ggagcggccg gagcagcccg ggtcctgacc     60
   ccggcccggc tcccgctccg ggctctgccg gcgggcgggc gagcgcggcg cggtccgggc    120
   cggggggatg tctcggcgga cgcgctgcga ggatctggat gagctgcact accaggacac    180
   agattcagat gtgccggagc agagggatag caagtgcaag gtcaaatgga cccatgagga    240
   ggacgagcag ctgagggccc tggtgaggca gtttggacag caggactgga agttcctggc    300
   cagccacttc cctaaccgca ctgaccagca atgccagtac aggtggctga gagttttgaa    360
   tccagacctt gtcaaggggc catggaccaa agaggaagac caaaaagtca tcgagctggt    420
   taagaagtat ggcacaaagc agtggacact gattgccaag cacctgaagg gccggctggg    480
   gaagcagtgc cgtgaacgct ggcacaacca cctcaaccct gaggtgaaga agtcttgctg    540
   gaccgaggag gaggaccgca tcatctgcga ggcccacaag gtgctgggca accgctgggc    600
   cgagatcgcc aagatgttgc cagggaggac agacaatgct gtgaagaatc actggaactc    660
   taccatcaaa aggaaggtgg acacaggagg cttcttgagc gagtccaaag actgcaagcc    720
   cccagtgtac ttgctgctgg agctcgagga caaggacggc ctccagagtg cccagcccac    780
   ggaaggccag ggaagtcttc tgaccaactg gccctccgtc cctcctacca taaaggagga 840
   ggaaaacagt gaggaggaac ttgcagcagc caccacatcg aaggaacagg agcccatcgg 900
   tacagatctg gacgcagtgc gaacaccaga gcccttggag gaattcccga agcgtgagga 960
   ccaggaaggc tccccaccag aaacgagcct gccttacaag tgggtggtgg aggcagctaa 1020
   cctcctcatc cccgctgtgg gttctagcct ctctgaagcc ctggacttga tcgagtcgga 1080
   ccctgatgct tggtgtgacc tgagtaaatt tgacctccct gaggaaccat ctgcagagga 1140
   cagtatcaac aacagcctag tgcagctgca agcgtcacat cagcagcaag tcctgccacc 1200
   ccgccagcct tccgccctgg tgcccagtgt gaccgagtac cgcctggatg gccacaccat 1260
   ctcagacctg agccggagca gccggggcga gctgatcccc atctccccca gcactgaagt 1320
   cgggggctct ggcattggca caccgccctc tgtgctcaag cggcagagga agaggcgtgt 1380
   ggctctgtcc cctgtcactg agaatagcac cagtctgtcc ttcctggatt cctgtaacag 1440
   cctcacgccc aagagcacac ctgttaagac cctgcccttc tcgccctccc agtttctgaa 1500
   cttctggaac aaacaggaca cattggagct ggagagcccc tcgctgacat ccaccccagt 1560
   gtgcagccag aaggtggtgg tcaccacacc actgcaccgg gacaagacac ccctgcacca 1620
   gaaacatgct gcgtttgtaa ccccagatca gaagtactcc atggacaaca ctccccacac 1680
   gccaaccccg ttcaagaacg ccctggagaa gtacggaccc ctgaagcccc tgccacagac 1740
   cccgcacctg gaggaggact tgaaggaggt gctgcgttct gaggctggca tcgaactcat 1800
   catcgaggac gacatcaggc ccgagaagca gaagaggaag cctgggctgc ggcggagccc 1860
   catcaagaaa gtccggaagt ctctggctct tgacattgtg gatgaggatg tgaagctgat 1920
   gatgtccaca ctgcccaagt ctctatcctt gccgacaact gccccttcaa actcttccag 1980
   cctcaccctg tcaggtatca aagaagacaa cagcttgctc aaccagggct tcttgcaggc 2040
   caagcccgag aaggcagcag tggcccagaa gccccgaagc cacttcacga cacctgcccc 2100
   tatgtccagt gcctggaaga cggtggcctg cggggggacc agggaccagc ttttcatgca 2160
   ggagaaagcc cggcagctcc tgggccgcct gaagcccagc cacacatctc ggaccctcat 2220
   cttgtcctga ggtgttgagg gtgtcacgag cccattctca tgtttacagg ggttgtgggg 2280
   gcagaggggg tctgtgaatc tgagagtcat tcaggtgacc tcctgcaggg agccttctgc 2340
   caccagcccc tccccagact ctcaggtgga ggcaacaggg ccatgtgctg ccctgttgcc 2400
   gagcccagct gtgggcggct cctggtgcta acaacaaagt tccacttcca ggtctgcctg 2460
   gttccctccc caaggccaca gggagctccg tcagcttctc ccaagcccac gtcaggcctg 2520
   gcctcatctc agaccctgct taggatgggg gatgtggcca ggggtgctcc tgtgctcacc 2580
   ctctcttggt gcattttttt ggaagaataa aattgcctct ctctttg 2627
<210> 54
   <211> 1249
   <212> DNA
   <213> Homo sapiens
<400> 54
   ctgattttct ctttggattc ttccaaaatc agagtcagac tactccctgt gccatgaacg     60
   gagatgacac ctttgcaagg agacccacgg ttggtgctca aataccagag aagatacaaa    120
   aggccttcga tgatattgcc aaatacttct ctaaggaaga gtgggaaaag atgaaagtct    180
   cggagaaaat cgtctatgtg tatatgaaga gaaagtatga ggccatgact aaactaggtt    240
   tcaaggccat cctcccatct ttcatgcgta ataaacgggt cacagacttc caggggaatg    300
   attttgataa tgaccctaac cgtgggaatc aggttcaacg tcctcagatg actttcggca    360
   ggctccaggg aatcttcccg aagatcatgc ccaagaagcc agcagaggaa ggaaatgttt    420
   cgaaggaagt gccagaagca tctggcccac aaaacgatgg gaaacagctg tgccccccgg    480
   gaaaaccaac tacctctgag aagattaaca tgatatctgg acccaaaagg ggggaacatg    540
   cctggaccca cagactgcgt gagagaaagc agctggtgat ttatgaagag atcagcgatc    600
   ctgaggaaga tgatgagtaa ctccccttgg ggatatgaca catgcccatg atgagaagca    660
   gaacgtggtg acctttcacg aacatgggca tggctgtgga cccctcgtca tcaggtgcat    720
   agcaagtgaa agcaagtgtt cacaacagtg aaaagttgag cgtcattttt cttagtgtgc    780
   caagagtacg atattagcgt ttccattgta ttttcttgaa gtgtgtcatt ctgttagata    840
   tgaacatttt cactgatgag caagacatac ttaatgcata ttttggtttg tgtatccatg    900
   cacctacctt agaaaacaag tattgtcagt tacctctgca tggaacagca ttaccctcct    960
   ctctccctag atgtgactac tgagggcagt tctgagtgtt taatttcaga ttttttcctc    1020
   tgcatttaca cacacacaca aaccacacca cacacacaca cacacacaca cacacacaca   1080
   ccaagtacca gtataagcat ctcccatctg cttttcccat tgccatgcgt cctggtcagg   1140
   cttccctcac tctgtttcct ggtcagcatg tactcccctc atccgattcc cctgtagcag   1200
   tcactgacag taaataaacc tttgcaaacg ttaaaaaaaa aaaaaaaaa               1249
<210> 55
   <211> 1949
   <212> DNA
   <213> Homo sapiens
<400> 55
   atgacgcgag accccgcccc cgcagcgccc gcttccaaga tggcggcagc gatgcctgcc 60
   cggctgttgg ggtggcggtg acgacaggca gcaaaagacc agctggtccc agattcgctg 120
   ctggagtgct ggatggagcc tttctctgcc ctctgtgaca tttccaattt tagataatgc 180
   ctcacatctc tgtccccccg ggaccccctg gagcccccat gatccctaag aagacagctt 240
   gaacctagat ctcaccccca ggatgttgcg gaggctgctg gagcggcctt gcacgctggc 300
   cctgcttgtg ggctcccagc tggctgtcat gatgtacctg tcactggggg gcttccgaag 360
   tctcagtgcc ctatttggcc gagatcaggg accgacattt gactattctc accctcgtga 420
   tgtctacagt aacctcagtc acctgcctgg ggccccaggg ggtcctccag ctcctcaagg 480
   tctgccctac tgtccagaac gatctcctct cttagtgggt cctgtgtcgg tgtcctttag 540
   cccagtgcca tcactggcag agattgtgga gcggaatccc cgggtagaac cagggggccg 600
   gtaccgccct gcaggttgtg agccccgctc ccgaacagcc atcattgtgc ctcatcgtgc 660
   ccgggagcac cacctgcgcc tgctgctcta ccacctgcac cccttcttgc agcgccagca 720
   gcttgcttat ggcatctatg tcatccacca ggctggaaat ggaacattta acagggcaaa 780
   actgttgaac gttggggtgc gagaggccct gcgtgatgaa gagtgggact gcctgttctt 840
   gcacgatgtg gacctcttgc cagaaaatga ccacaatctg tatgtgtgtg acccccgggg 900
   accccgccat gttgccgttg ctatgaacaa gtttggatac agcctcccgt acccccagta 960
   cttcggagga gtctcagcac ttactcctga ccagtacctg aagatgaatg gcttccccaa 1020
   tgaatactgg ggctggggtg gtgaggatga cgacattgct accagggtgc gcctggctgg 1080
   gatgaagatc tctcggcccc ccacatctgt aggacactat aagatggtga agcaccgagg 1140
   agataagggc aatgaggaaa atccccacag atttgacctc ctggtccgta cccagaattc 1200
   ctggacgcaa gatgggatga actcactgac ataccagttg ctggctcgag agctggggcc 1260
   tctttatacc aacatcacag cagacattgg gactgaccct cggggtcctc gggctccttc 1320
   tgggccacgt tacccacctg gttcctccca agccttccgt caagagatgc tgcaacgccg 1380
   gcccccagcc aggcctgggc ctctatctac tgccaaccac acagccctcc gaggttcaca 1440
   ctgactcctc cttcctgtct accttaatca tgaaaccgaa ttcatggggt tgtattctcc 1500
   ccaccctcag ctcctcactg ttctcagagg gatgtgaggg aactgaactc tggtgccgtg 1560
   ctagggggta ggggcctctc cctcactgct ggactggagc tgggctcctg tagacctgag 1620
   gggtccctct ctctagggtc tcctgtaggg cttatgactg tgaatccttg atgtcatgat 1680
   tttatgtgac gattcctagg agtccctgcc cctagagtag gagcagggct ggaccccaag 1740
   cccctccctc ttccatggag agaagagtga tctggcttct cctcggacct ctgtgaatat 1800
   ttattctatt tatggttccc gggaagttgt ttggtgaagg aagcccctcc ctgggcattt 1860
   tctgcctatg ctggaatagc tccctcttct ggtcctggct cagggggctg ggattttgat 1920
   atattttcta ataaaggact ttgtctcgc 1949
<210> 56
   <211> 470
   <212> DNA
   <213> Homo sapiens
<400> 56
   gttcctccat ttatcgtttc ttcgtcatgt cgggacgcgg caagcaggga ggcaaagctc      60
   gcgccaaagc caagacccgc tcttctcgtg ccggtctcca gttccccgtg ggccgagtgc     120
   accgactgct ccgcaagggc aactatgctg agcgggtcgg ggccggcgcg ccggtgtacc     180
   tggcggcggt gctggagtac ctgactgccg agatcctgga gctggcgggc aacgccgccc     240
   gcgacaacaa gaagacccgc attatcccgc gccacttgca gctggccatc cgcaacgacg     300
   aggagctcaa caagctgctg ggcaaagtaa ccatcgctca gggtggtgtc ctgcccaaca     360
   tccaggctgt gctactgccc aagaagaccg agagtcacca caaggccaaa ggcaaataat     420
   gtctccatag aatcactttc caatacaacg gctcttttca gagccaccta                470
<210> 57
   <211> 1120
   <212> DNA
   <213> Homo sapiens
<400> 57
   acttcttcgc accagggaag ccccacccac cagaacgcca agatgtccag caagcgggcc      60
   aaagccaagg ccaccaagaa gcggccacag cgggccacat ccaatgtctt cgcaatgttt     120
   gaccagtccc agatccagga gtttaaggag gctttcaaca tgattgacca gaaccgtgat     180
   ggcttcattg acaaggagga cctgcacgac atgctggcct cgctggggaa gaaccccaca     240
   gacgaatacc tggagggcat gatgagcgag gccccggggc catacaactt caccatgttc     300
   ctcaccatgt ttggggagaa gctgaacggc acggaccccg aggatgtgat tcgcaacgcc     360
   tttgcctgct tcgacgagga atcctcaggt ttcatccatg aggaccacct ccggaagctg     420
   ctcaccacca tgggtgaccg cttcacagat gaggaagtgg acgagatgta ccgggaggca     480
   cccgttgata agaaaggcaa cttcaactac gtggagttca cccgcatcct caaacatggc     540
   gccaaggata aacacgacta ggccatcccc agccccctga cacccagccc ccgccagtca     600
   cccctccccg cacacacccg tccataccag ctccctgccc atgaccctcg ctcagggatc     660
   cccctttgag ggttagggtc ccagttccca gtggaagaaa caggccagga gagtgcgtgc     720
   cgagctgagg cagatgttcc cacagtgacc ccagagccct gggctatagt ctctgacccc     780
   tccaaggaaa gaccaccttc tggggacatg ggctggaggg caggacctag aggcaccaag     840
   ggaaccgcat tccggggctg ttccccgagg aggaagggaa gcctctgtgt gccccccagg     900
   aggaagaggc cctgagtcct gggatcagac accccttcac gtgtatccca cacaaatgca     960
   agctcaccaa ggtcccctct cagtcccctt ccctacaccc tgacgccaga tgccgcacac    1020
   ccaacgccac cagccatggg agtgtgctca ggagtcgcgg ggcagacgtg acatctgtcc    1080
   agagggggca gaatctccaa tagaggactg agacaacatg                           1120
<210> 58
   <211> 1497
   <212> DNA
   <213> Homo sapiens
<400> 58
   accaacctct tcgaggcaca aggcacaaca ggctgctctg ggattctctt cagccaatct 60
   tcattgctca agtgtctgaa gcagccatgg cagaagtacc tgagctcgcc agtgaaatga 120
   tggcttatta cagtggcaat gaggatgact tgttctttga agctgatggc cctaaacaga 180
   tgaagtgctc cttccaggac ctggacctct gccctctgga tggcggcatc cagctacgaa 240
   tctccgacca ccactacagc aagggcttca ggcaggccgc gtcagttgtt gtggccatgg 300
   acaagctgag gaagatgctg gttccctgcc cacagacctt ccaggagaat gacctgagca 360
   ccttctttcc cttcatcttt gaagaagaac ctatcttctt cgacacatgg gataacgagg 420
   cttatgtgca cgatgcacct gtacgatcac tgaactgcac gctccgggac tcacagcaaa 480
   aaagcttggt gatgtctggt ccatatgaac tgaaagctct ccacctccag ggacaggata 540
   tggagcaaca agtggtgttc tccatgtcct ttgtacaagg agaagaaagt aatgacaaaa 600
   tacctgtggc cttgggcctc aaggaaaaga atctgtacct gtcctgcgtg ttgaaagatg 660
   ataagcccac tctacagctg gagagtgtag atcccaaaaa ttacccaaag aagaagatgg 720
   aaaagcgatt tgtcttcaac aagatagaaa tcaataacaa gctggaattt gagtctgccc 780
   agttccccaa ctggtacatc agcacctctc aagcagaaaa catgcccgtc ttcctgggag 840
   ggaccaaagg cggccaggat ataactgact tcaccatgca atttgtgtct tcctaaagag 900
   agctgtaccc agagagtcct gtgctgaatg tggactcaat ccctagggct ggcagaaagg 960
   gaacagaaag gtttttgagt acggctatag cctggacttt cctgttgtct acaccaatgc 1020
   ccaactgcct gccttagggt agtgctaaga ggatctcctg tccatcagcc aggacagtca 1080
   gctctctcct ttcagggcca atccccagcc cttttgttga gccaggcctc tctcacctct 1140
   cctactcact taaagcccgc ctgacagaaa ccacggccac atttggttct aagaaaccct 1200
   ctgtcattcg ctcccacatt ctgatgagca accgcttccc tatttattta tttatttgtt 1260
   tgtttgtttt attcattggt ctaatttatt caaagggggc aagaagtagc agtgtctgta 1320
   aaagagccta gtttttaata gctatggaat caattcaatt tggactggtg tgctctcttt 1380
   aaatcaagtc ctttaattaa gactgaaaat atataagctc agattattta aatgggaata 1440
   tttataaatg agcaaatatc atactgttca atggttctga aataaacttc tctgaag 1497
<210> 59
   <211> 1237
   <212> DNA
   <213> Homo sapiens
<400> 59
   agcgtgggta aaagcaaaag caacagctca agcagcctcc ttggagaaaa cctgaaaatt      60
   caacttgttc aagagaaggt cttgtacgtg cctaagttct agagcctcct gacgtgagca     120
   tggctgagag tgaggaccgc tccctgagga tcgttctggt agggaaaact ggaagtggga     180
   aaagtgcaac agcgaacacc atccttggag aggaaatctt tgattctaga attgctgccc     240
   aagctgttac caagaactgt caaaaagcat cccgggaatg gcaggggaga gaccttcttg     300
   ttgtagacac tccagggctc tttgacacca aggagagcct ggacaccacc tgcaaggaaa     360
   tcagccgctg catcatctcc tcctgcccag ggccccatgc tattgtccta gttctgctgc     420
   tgggccgcta cacagaggag gagcagaaaa ccgttgcatt gatcaaggct gtctttggga     480
   agtcagccat gaagcacatg gtcatcttgt tcactcgcaa agaagagttg gagggccaga     540
   gcttccatga cttcatagca gatgcggatg tgggcctaaa aagcatcgtc aaggagtgcg     600
   ggaaccgctg ctgtgccttt agcaacagca agaaaaccag taaggcagag aaggaaagtc     660
   aagtgcagga gttggtggag ctgatagaga aaatggtgca gtgcaacgaa ggggcttact     720
   tttctgatga catatacaag gacacagagg aaaggctgaa acaacgggaa gaggttttga     780
   ggaaaatcta cactgaccaa ttaaatgaag aaattaaact agtagaagag gataagcata     840
   aatcagagga agaaaaggag aaagaaatta aattactaaa attaaaatat gatgaaaaaa     900
   taaaaaatat aagggaagaa gctgagagaa atatatttaa agatgttttt aataggattt     960
   ggaagatgct ttcagaaata tggcataggt ttttgtcgaa atgtaagttt tattcttcct    1020
   aatttactgt gatttgttaa tggatgaatt gtattttgca aagatagtta gagaaatacc    1080
   tccttcccct tagctttatt aaggtatcat tgataaataa aaataaaata tgtttaatgt    1140
   atataatgtg atttttaaat atatatatat atatacacac attgtgaaat aatgaaataa    1200
   aggtaattaa cacatctaaa acaaaaaaaa aaaaaaa                             1237
<210> 60
   <211> 2397
   <212> DNA
   <213> Homo sapiens
<400> 60
   tttttagttc tgacttaggc caaaatagaa aaaaagaaag tatgttcaga aggcaaatgg     60
   tcatgagatc aaaggccaag ggaccccgac agggcaggcg cagagctcct gcttggggct   120
   tgggtggggt gtttgtgggg gttattctgc tccgcccccc ggaaaggcca ggagcccttc    180
   ggattggcgt cttgctgagc tcctgctgcc ccctgctggt ttcgcggcac tccctggtcc    240
   tcagaaatgt agacaggatg gtcaaatgga atcccatctc ccctctctct cttcattcac    300
   ttaaaattac ctctcccata cggactgaaa gtggcttgag tgataataga gaagttgaag    360
   ctgcttttca gcctaaatta tctccagaac ggcttcttgt tcttcattag aagagatgcg 420
   cttctcaggt ttccaggtga gccggatagc cctggctgta ggagtccaga gagaatagtt 480
   ccttctctgg tgtctctctc ttcacgaagc caagagggga tctcatgtag ggacccttga 540
   ataaaccatg cccgctggtt aattccacat gcttttcatg tcttgcagtt cagtgaattc 600
   tacagtcttg gtgaagaaca cgaagaagac taatccagag ataaaagaaa aaccctgcca 660
   ttttgaaaga tgtgaagggg aggtgaacac acgcttcagc ctaaaacact aagtagatgc 720
   aggcctgggc cgttctcata cccccgggaa ccatatctta cccattgtat gtcgcagctt 780
   gcaggccagt gcttggcaca gagcagggac tcaggaagcc tttgtcacta aagtaagagc 840
   ctctgcggag tacagtgcat ggggtcggct gggccagccc caggcagcag atcctggtat 900
   tgggctgagg aaagagcact gcgcttggag tcagtaagat ctgccacctc cctgagtctc 960
   atcagcaaaa tgaggataaa gataaagata ctatagttgc ccagcctgct tgacagggtt 1020
   gttgtaaggt tcacataaga tgatgatatg caaatgcttt gtaatctagg aggtgctatt 1080
   tgtctaaagt ctaatggaga attataatac atccaggagt taaggagttc taatgcttaa 1140
   aatgaaatag tctaagatct tagcaagaaa ggattaagaa ggacttttct ctccatattg 1200
   attttgtaat ggagttataa ataattgctt ctagagactg agaaattgat tggttttctt 1260
   taactcctat tctttctttt ctttctttaa tttttaaaaa actctttgaa tagttacctt 1320
   tctctatttt gggctgtttt tgtcccaaga gtaggatttt ttcccagtag agtgcagtgg 1380
   tccaagaatg ggccactgga tgatactgct ttaccaacga gtgacaggac catgaacctc 1440
   acagttgtga ggttcaatga gggctggccc tgccacataa atcctctgag ggagatgatg 1500
   acaattcact gctgattaat gccattctgc ctttactgta attagaagga aataacccca 1560
   gaatacaagg aatttagcaa gataaggaac ccctgctgct acctaaacat ccatctaaac 1620
   aaagatgttt ggcttttgaa gcaaagagtt tggttctcaa gactgtgttc tttgacagtt 1680
   aattttcaag aagactgaag actgaattat cattgttgag aattctctag gtctcagtaa 1740
   ccctctgaac cagcagtttg ggtggtcgat gcccagcaaa taggagtggg tggccttttc 1800
   tctggtgtat aagattcatc taatttttag gaatttttgt accattttcc ccctctagaa 1860
   acacatttac tccccaataa ttgtacggga ggtgatcgag gaagaagaac caagtgaaaa 1920
   atcagaggcc acctacatga ccatgcaccc agtttggcct tctctgaggt cagatcggaa 1980
   caactcactt gaaaaaaagt caggtggggg aatgccaaaa acacagcaag ccttttgaga 2040
   agaatggaga gtcccttcat ctcagcagcg gtggagactc tctcctgtgt gtgtcctggg 2100
   ccactctacc agtgatttca gactcccgct ctcccagctg tcctcctgtc tcattgtttg 2160
   gtcaatacac tgaagatgga gaatttggag cctggcagag agactggaca gctctggagg 2220
   aacgggcctg ctgaggggag gggagcatgg acttggcctc tggagtggga cactggccct 2280
   gggaaccagg ctgagctgag tggcctcaaa ccccccgttg gatcagaccc tcctgtgggc 2340
   agggttctta gtggatgagt tactgggaag aatcagagat aaaaaccaac ccaaatc 2397
<210> 61
   <211> 1763
   <212> DNA
   <213> Homo sapiens
<400> 61
   tagctggatt ccagccattg ctgcagctgc tccacagccc ttttcaggac ccaaacaacc 60
   gcagccgctg ttcccaggat ggtgatccgt gtatatattg catcttcctc tggctctaca 120
   gcgattaaga agaaacaaca agatgtgctt ggtttcctag aagccaacaa aataggattt 180
   gaagaaaaag atattgcagc caatgaagag aatcggaagt ggatgagaga aaatgtacct 240
   gaaaatagtc gaccagccac aggttacccc ctgccacctc agattttcaa tgaaagccag 300
   tatcgcgggg actatgatgc cttctttgaa gccagagaaa ataatgcagt gtatgccttc 360
   ttaggcttga cagccccacc tggttcaaag gaagcagaag tgcaagcaaa gcagcaagca 420
   tgaaccttaa gcactgtgct ttaagcatcc tgaaaaatga gtctccattg cttttataaa 480
   atagcagaat tagctttgct tcaaaagaaa taggcttaat gttgaaataa tagattagtt 540
   gggttttcac atgcaaacat tcaaaatgaa tacaaaatta aaatttgaac attatggtga 600
   ttatggtgag gagaatggga tattaacata aaattatatt aataagtaga tatcgtagaa 660
   atagtgttgt tacctgccaa gccatcctgt atacaccaat gattttacaa agaaaacacc 720
   cttccctcct tctgccatta ctatggcaac ttaagtgtat ctgcagctct acattaaaaa 780
   ggagaaagag aaataacctg tctctcattc ctaagttgcc tcattaattt tcatgaacaa 840
   gaatatgtac ctttttgatg ctatattact gcgattaaaa aagttcttgc aggtaatgtt 900
   tatgtatagt taaacgttgt aatttcttat cgtaattata acattcccat tctttgtaga 960
   tgaaactcta catatgaacc acagattttc tgagcttcta aatgtagcct ttcattgcac 1020
   atttcagtga tcagaataga tatcctttta cacgcacaaa agcaatagat tcattcagtg 1080
   gacaagttcc ttgtttaact acacagctat gatggaatca tatatccaag ttccttgcct 1140
   cagtgaaata tgcatatgta tatcatgaag tgggatgcca agtaagctta aaatgcattc 1200
   tctagcaaag agattagact tttaaataac tcttataaaa caggttggcg atcatttccc 1260
   aagattggtt tcccttgagt ttttgttaaa acaaatctta gtagttttgc ccgtttaaaa 1320
   caactcacaa tcgtaaatgc tactattcct aagatatctt acctttttat ttcagtttag 1380
   ccatgtattg tatgagtgta ttagtctaag cagtgagaat cttttctatg cctctattcc 1440
   agcaaaaagt agaagtatca aataaaaagg gcaactttta aaatattaag cctgaagact 1500
   tctaaaaaga caagaaacat ggcctaaata accaacatag atttacatag taagtttcac 1560
   actaccttat taccaaaagc aaacacctct tactttaaac tacattatca tgtatatcta 1620
   ttgtatgctg gtctttactt tttgccaaaa tcaacatata atgaagagat gcctttgttt 1680
   gatgagattc aaacttgatg ctatgcttta aaataaactc agtactttta gaaacataaa 1740
   aaaaaaaaaa aaaaaaaaaa aaa 1763
<210> 62
   <211> 1134
   <212> DNA
   <213> Homo sapiens
<400> 62
   cgacccctcg aggggcccag ccttggaagg gtaactggac cgctgccgcc tggttgcctg      60
   ggccagacca gacatgcctg ctgctccttc cggcttagga ggagcacgcg tcccgctcgg     120
   gcgcactctc cagccttttc ctggctgagg aggggccgag cctccggtag ggcgggggcc     180
   ggatgaggcg ggacctcagg cccggaaaac tgcctgtgcc acgtgacccg ccgccggcca     240
   gttaaaagga ggcgcctgct ggcctcccct tacagtgctt gttcggggcg ctccgctggc     300
   ttcttggaca attgcgccat gtgtgctgct cggctagcgg cggcggcggc ccagtcggtg     360
   tatgccttct cggcgcgccc gttggccggc ggggagcctg tgagcctggg ctccctgcgg     420
   ggcaaggtac tacttatcga gaatgtggcg tccctctgag gcaccacggt ccgggactac     480
   acccagatga acgagctgca gcggcgcctc ggaccccggg gcctggtggt gctcggcttc     540
   ccgtgcaacc agtttgggca tcaggagaac gccaagaacg aagagattct gaattccctc     600
   aagtacgtcc ggcctggtgg tgggttcgag cccaacttca tgctcttcga gaagtgcgag     660
   gtgaacggtg cgggggcgca ccctctcttc gccttcctgc gggaggccct gccagctccc     720
   agcgacgacg ccaccgcgct tatgaccgac cccaagctca tcacctggtc tccggtgtgt     780
   cgcaacgatg ttgcctggaa ctttgagaag ttcctggtgg gccctgacgg tgtgccccta     840
   cgcaggtaca gccgccgctt ccagaccatt gacatcgagc ctgacatcga agccctgctg     900
   tctcaagggc ccagctgtgc ctagggcgcc cctcctaccc cggctgcttg gcagttgcag     960
   tgctgctgtc tcgggggggt tttcatctat gagggtgttt cctctaaacc tacgagggag    1020
   gaacaccttg atcttacaga aaataccacc tcgagatggg tgctggtcct gttgatccca    1080
   gtctctgcca gaccaaggcg agtttcccca ctaataaagt gccgggtgtc agca          1134
<210> 63
   <211> 1233
   <212> DNA
   <213> Homo sapiens
<400> 63
   gaattccgcc aagcggggac ctcaggatgg aaaccagcag cctgcaccgc ccgagaaggt      60
   cggctgggtc cggaaattct gcgggaaagg gattttcagg gagatttgga aaaaccgcta    120
   tgtggtgctg aaaggggacc agctctacat ctctgagaag gaggtaaaag atgagaaaaa    180
   tattcaagag gtatttgacc tgagtgacta tgagaagtgt gaagagctcc ggaagtccaa    240
   gagcaggagc aagaaaaatc atagcaagtt tactcttgcc cactccaaac agcccggtaa    300
   cacggcaccc aacctgatct tcctggcagt gagtccagaa gagaaggaat cgtggatcaa    360
   tgccctcaac tctgccatca cccgagccaa gaaccgtatc ttggatgagg tcaccgttga    420
   ggaggacagc tatcttgccc atcccactcg agacagggca aaaatccagc actcccgccg    480
   ccccccaaca aggggacacc taatggctgt ggcttccacc tctacctcgg atgggatgct    540
   gaccttggac ttgatccaag aggaagaccc ttcccctgag gaaccaacct cttgtgctga    600
   gagctttcgg gttgacctgg acaagtctgt ggcccagctg gcagggagcc ggcggagagc    660
   ggactcagac cgcatccagc cctccgcaga ccgggcaagc agtctctccc gaccttggga    720
   aaaaacagac aaaggggcca cctacacccc ccaggcaccc aagaagttga cgcccacaga    780
   gaaaggccgc tgcgcctccc tggaggagat cctatctcag cgggatgctg cctctgcccg    840
   caccctccag ctgcgggctg aggaaccccc aacccctgcc ctccccaacc cggggcagct    900
   gtcccggatc caggacctgg tagcaaggaa actggaggag actcaggagc ttctggcaga    960
   ggttcaggga ctgggagatg ggaagcgaaa ggccaaggac ccccctcggt ctccgccgga   1020
   ttctgagtca gagcagctgc tgctggagac ggaacggctg ctgggagagg catcatcgaa   1080
   ttggagccag gcaaagaggg tgctgcagga ggtcagggag ctgagagacc tgtacagaca   1140
   gatggacctg cagaccccgg actcccacct cagacagacc accccgcaca gtcagtaccg   1200
   gaagagcctg atgtgagggc agggtggggt ctg                                1233
<210> 64
   <211> 2396
   <212> DNA
   <213> Homo sapiens
<400> 64
   ggcacgaggg ctgtgcgggt ggcggccggc gcgcggtggg gcatggcggg ttcgcggggt      60
   gcggggcgca cggcggcgcc gagcgtgcgg ccggagaagc ggcggtctga gcccgaactg     120
   gagcctgagc ccgagccgga gccccccctc ctctgcacct ctcctctcag ccacagcacc    180
   ggcagcgatt ctggcgtctc cgacagcgag gagagtgtgt tctcaggcct ggaagattcc     240
   ggcagtgaca gcagtgagga tgatgacgaa ggcgacgagg agggagagga cggagccctt    300
   gatgacgagg gccacagtgg gattaaaaag accactgagg agcaggtgca ggccagcact     360
   ccttgcccga ggacagagat ggcgagcgcc cggattgggg atgagtatgc ggaggacagc     420
   tctgatgagg aggacatccg gaacacggtg ggcaacgtgc ccttggagtg gtacgatgac     480
   ttcccccacg tgggctacga cctggatggc aggcgcatct acaagcccct gcggacccgg 540
   gatgagctgg accagttcct ggacaagatg gacgatcctg actactggcg caccgtgcag 600
   gacccgatga cagggcggga cctgagactg acggatgagc aggtggccct ggtgcggcgg 660
   ctgcagagtg gccagtttgg ggatgtgggc ttcaacccct atgagccggc tgtcgacttc 720
   ttcagcgggg acgtcatgat ccacccggtg accaaccgcc cggccgacaa gcgcagcttc 780
   atcccctccc tggtggagaa ggagaaggtc tctcgcatgg tgcacgccat caagatgggc 840
   tggatccagc ctcgccggcc ccgagacccc acccccagct tctatgacct gtgggcccag 900
   gaggacccca acgccgtgct cgggcgccac aagatgcacg tacctgctcc caagctggcc 960
   ctgccaggcc acgccgagtc gtacaaccca ccccctgaat acctgctcag cgaggaggag 1020
   cgcttggcgt gggaacagca ggagccaggc gagaggaagc tgagcttttt gccacgcaag 1080
   ttcccgagcc tgcgggccgt gcctgcctac ggacgcttca tccaggaacg cttcgagcgc 1140
   tgccttgacc tgtacctgtg cccacggcag cgcaagatga gggtgaatgt agaccctgag 1200
   gacctcatcc ccaagctgcc tcggccgagg gacctgcagc ccttccccac gtgccaggcc 1260
   ctggtctaca ggggccacag tgaccttgtc cggtgcctca gtgtctctcc tgggggccag 1320
   tggctggttt caggctctga cgacggctcc ctgcggctct gggaggtggc cactgcccgc 1380
   tgtgtgagga ctgttcccgt ggggggcgtg gtgaagagtg tggcctggaa ccccagcccc 1440
   gctgtctgcc tggtggctgc agccgtggag gactcggtgc tgctgctgaa cccagctctg 1500
   ggggaccggc tggtggcggg cagcacagat cagctgttga gcgccttcgt cccgcctgag 1560
   gagcccccct tgcagccggc ccgctggctg gaggcctcag aggaggagcg ccaagtgggc 1620
   ctgcggctgc gcatctgcca cgggaagcca gtgacgcagg tgacctggca cgggcgtggg 1680
   gactacctgg ccgtggtgct ggccacccaa ggccacaccc aggtgctgat tcaccagctg 1740
   agccgtcgcc gcagccagag tccgttccgc cgcagccacg gacaggtgca gcgagtggcc 1800
   ttccaccctg cccggccctt cctgttggtg gcgtcccagc gcagcgtccg cctctaccac 1860
   ctgctgcgcc aggagctcac caagaagctg atgcccaact gcaagtgggt gtccagcctg 1920
   gcggtgcacc ctgcaggtga caacgtcatc tgtgggagct acgatagcaa gctggtgtgg 1980
   tttgacctgg atctttccac caagccatac aggatgctga gacaccacaa gaaggctctg 2040
   cgggctgtgg ccttccaccc gcggtaccca ctctttgcgt caggctcgga cgacggcagt 2100
   gtcatcgtct gccatggcat ggtgtacaat gaccttctgc agaacccctt gctggtgccc 2160
   gtcaaggtgc tgaagggaca cgtgctgacc cgagatctgg gagtgctgga cgtcatcttc 2220
   caccccaccc agccgtgggt cttctcctcg gggcagacgg ggactgtccg cctcttcacc 2280
   tagctgttct gcctgcctgg ggctggggtg gtcgtgctga agtcaacaga gcctttaccc 2340
   tgtgcaaaaa aaaaaaaaaa aaaaaaaaaa aaaaaaaaaa aaaaaaaaaa aaaaaa 2396
<210> 65
   <211> 1048
   <212> DNA
   <213> Homo sapiens
<400> 65
   aggagctggg gccatcaagg cggaccatgt gtcaacttat gccgcgtttg tacagacgca      60
   tagaccaaca ggggagttta tgtttgaatt tgatgaagat gagatgttct atgtggatct     120
   ggacaagaag gagaccgtct ggcatctgga ggagtttggc caagcctttt cctttgaggc     180
   tcagggcggg ctggctaaca ttgctatatt gaacaacaac ttgaatacct tgatccagcg     240
   ttccaaccac actcaggcca ccaacgatcc ccctgaggtg accgtgtttc ccaaggagcc     300
   tgtggagctg ggccagccca acaccctcat ctgccacatt gacaagttct tcccaccagt     360
   gctcaacgtc acgtggctgt gcaacgggga gctggtcact gagggtgtcg ctgagagcct     420
   cttcctgccc agaacagatt acagcttcca caagttccat tacctgacct ttgtgccctc     480
   agcagaggac ttctatgact gcagggtgga gcactggggc ttggaccagc cgctcctcaa     540
   gcactgggag gcccaagagc caatccagat gcctgagaca acggagactg tgctctgtgc     600
   cctgggcctg gtgctgggcc tagtcggctt catcgtgggc accgtcctca tcataaagtc     660
   tctgcgttct ggccatgacc cccgggccca ggggaccctg tgaaatactg taaaggtgac     720
   aaaatatctg aacagaagag gacttaggag agatctgaac tccagctgcc ctacaaactc     780
   catctcagct tttcttctca cttcatgtga aaactactcc agtggctgac tgaattgctg     840
   acccttcaag ctctgtcctt atccattacc tcaaagcagt cattccttag taaagtttcc     900
   aacaaataga aattaatgac actttggtag cactaatatg gagattatcc tttcattgag     960
   ccttttatcc tctgttctcc tttgaagagc ccctcactgt caccttcccg agaataccct    1020
   aagaccaata aatacttcag tatttcag                                       1048
<210> 66
   <211> 1285
   <212> DNA
   <213> Homo sapiens
<400> 66
   ggggcccagg gccctcctat ggaccctgcc cgctcccctc ccattgtcca cggctgtccg 60
   cccaccccca ttctccaagc ttcagccccc tccttagttc ggcatctgca cagcactgaa 120
   gaacctggga atcagaccct gagaccctga gcaatcccag gtccagcgcc agccctatca 180
   tgaccaagga gtatcaagac cttcagcatc tggacaatga ggagagtgac caccatcagc 240
   tcagaaaagg gccacctcct ccccagcccc tcctgcagcg tctctgctcc ggacctcgcc 300
   tcctcctgct ctccctgggc ctcagcctcc tgctgcttgt ggttgtctgt gtgatcggat 360
   cccaaaactc ccagctgcag gaggagctgc ggggcctgag agagacgttc agcaacttca 420
   cagcgagcac ggaggcccag gtcaagggct tgagcaccca gggaggcaat gtgggaagaa 480
   agatgaagtc gctagagtcc cagctggaga aacagcagaa ggacctgagt gaagatcact 540
   ccagcctgct gctccacgtg aagcagttcg tgtctgacct gcggagcctg agctgtcaga 600
   tggcggcgct ccagggcaat ggctcagaaa ggacctgctg cccggtcaac tgggtggagc 660
   acgagcgcag ctgctactgg ttctctcgct ccgggaaggc ctgggctgac gccgacaact 720
   actgccggct ggaggacgcg cacctggtgg tggtcacgtc ctgggaggag cagaaatttg 780
   tccagcacca cataggccct gtgaacacct ggatgggcct ccacgaccaa aacgggccct 840
   ggaagtgggt ggacgggacg gactacgaga cgggcttcaa gaactggagg ccggagcagc 900
   cggacgactg gtacggccac gggctcggag gaggcgagga ctgtgcccac ttcaccgacg 960
   acggccgctg gaacgacgac gtctgccaga ggccctaccg ctgggtctgc gagacagagc 1020
   tggacaaggc cagccaggag ccacctctcc tttaatttat ttcttcaatg cctcgacctg 1080
   ccgcaggggt ccgggattgg gaatccgccc atctgggggc ctcttctgct ttctcgggaa 1140
   ttttcatcta ggattttaag ggaaggggaa ggatagggtg atgttccgaa ggtgaggagc 1200
   ttgaaacccg tggcgctttc tgcagtttgc aggttatcat tgtgaacttt tttttttttt 1260
   aagagtaaaa agaaatatac ctaaa 1285
<210> 67
   <211> 1820
   <212> DNA
   <213> Homo sapiens
<400> 67
   ggggatgcaa ctaagttgct gagacaaggg aagagagatg aggaaccaga gcttgtagaa 60
   accactttaa tcatatccag gagtttgcaa gaaacaggtg cttaacacta attcacctcc 120
   tgaacaagaa aaatgggctg tgaccggaac tgtgggctca tcgctggggc tgtcattggt 180
   gctgtcctgg ctgtgtttgg aggtattcta atgccagttg gagacctgct tatccagaag 240
   acaattaaaa agcaagttgt cctcgaagaa ggtacaattg cttttaaaaa ttgggttaaa 300
   acaggcacag aagtttacag acagttttgg atctttgatg tgcaaaatcc acaggaagtg 360
   atgatgaaca gcagcaacat tcaagttaag caaagaggtc cttatacgta cagagttcgt 420
   tttctagcca aggaaaatgt aacccaggac gctgaggaca acacagtctc tttcctgcag 480
   cccaatggtg ccatcttcga accttcacta tcagttggaa cagaggctga caacttcaca 540
   gttctcaatc tggctgtggc agctgcatcc catatctatc aaaatcaatt tgttcaaatg 600
   atcctcaatt cacttattaa caagtcaaaa tcttctatgt tccaagtcag aactttgaga 660
   gaactgttat ggggctatag ggatccattt ttgagtttgg ttccgtaccc tgttactact 720
   acagttggtc tgttttatcc ttacaacaat actgcagatg gagtttataa agttttcaat 780
   ggaaaagata acataagtaa agttgccata atcgacacat ataaaggtaa aaggaatctg 840
   tcctattggg aaagtcactg cgacatgatt aatggtacag atgcagcctc atttccacct 900
   tttgttgaga aaagccaggt attgcagttc ttttcttctg atatttgcag gtcaatctat 960
   gctgtatttg aatccgacgt taatctgaaa ggaatccctg tgtatagatt cgttcttcca 1020
   tccaaggcct ttgcctctcc agttgaaaac ccagacaact attgtttctg cacagaaaaa 1080
   attatctcaa aaaattgtac atcatatggt gtgctagaca tcagcaaatg caaagaaggg 1140
   agacctgtgt acatttcact tcctcatttt ctgtatgcaa gtcctgatgt ttcagaacct 1200
   attgatggat taaacccaaa tgaagaagaa cataggacat acttggatat tcaacctata 1260
   actggattca ctttacaatt tgcaaaacgg ctgcaggtca acctattggt caagccatca 1320
   gaaaaaattc aagtattaaa gaatctgaag aggaactata ttgtgcctat tctttggctt 1380
   aatgagactg ggaccattgg tgatgagaag gcaaacatgt tcagaagtca agtaactgga 1440
   aaaataaacc tccttggcct gatagaaatg atcttactca gtgttggtgt ggtgatgttt 1500
   gttgctttta tgatttcata ttgtgcatgc agatcgaaaa caataaaata agtatgtacc 1560
   aaaaaatatt gcttcaataa tattagctta tatattactt gttttcactt tatcaaagag 1620
   aagttacata ttaggccata tatatttcta gacatgtcta gccactgatc atttttaaat 1680
   ataggtaaat aaacctataa atattatcac gcagatcact aaagtatatc tttaattctg 1740
   ggagaaatga gataaaagat gtacttgtga ccattgtaac aatagcacaa taaagcactg 1800
   tgccaaagtt gtccaaaaaa 1820
<210> 68
   <211> 1314
   <212> DNA
   <213> Homo sapiens
<400> 68
   aggctcgcgg cgggcgctgg gcgcgggatc cgactctagt cgtaatggag gcgggcggct 60
   ttctggactc gctcatttac ggagcatgcg tggtcttcac ccttggcatg ttctccgccg 120
   gcctctcgga cctcaggcac atgcgaatga cccggagtgt ggacaacgtc cagttcctgc 180
   cctttctcac cacggaagtc aacaacctgg gctggctgag ttatggggct ttgaagggag 240
   acgggatcct catcgtcgtc aacacagtgg gtgctgcgct tcagaccctg tatatcttgg 300
   catatctgca ttactgccct cggaagcgtg ttgtgctcct acagactgca accctgctag 360
   gggtccttct cctgggttat ggctactttt ggctcctggt acccaaccct gaggcccggc 420
   ttcagcagtt gggcctcttc tgcagtgtct tcaccatcag catgtacctc tcaccactgg 480
   ctgacttggc taaggtgatt caaactaaat caacccaatg tctctcctac ccactcacca 540
   ttgctaccct tctcacctct gcctcctggt gcctctatgg gtttcgactc agagatccct 600
   atatcatggt gtccaacttt ccaggaatcg tcaccagctt tatccgcttc tggcttttct 660
   ggaagtaccc ccaggagcaa gacaggaact actggctcct gcaaacctga ggctgctcat 720
   ctgaccactg ggcaccttag tgccgacctg aaccaaagag acctccttgt ttcagctggg 780
   cctgctgtcc agcttcccag gtgcagtggg ttgtgggaac aagagatgac tttgaggata 840
   aaaggaccaa agaaaaagct ttacttagat gattgattgg ggcctaggag atgaaatcac 900
   tttttatttt ttagagattt ttttttttaa ttttggaggt tggggtgcaa tctttagaat 960
   atgccttaaa aggccgggcg cggtggctca cgcctgtaat cccagcactt tgggaggcca 1020
   aggtgggcgg atcgcctgag gtcaggagtt caagaccaac ctgactaaca tggtgaaacc 1080
   ccatctctac taaaaataca aaattagcca ggcatgatgg cacatgcctg taatcccaga 1140
   tacttgggag gctgaggcag gagaattgct tgaacccagg aggtggaggt tgcagtgagc 1200
   tgagatcgtg ccattgtgat atgaatatgc cttatatgct gatatgaata tgccttaaaa    1260
   taaagtgttc cccacccctg ccataaaaaa aaaaaaaaaa aaaaaaaaaa aaaa          1314
<210> 69
   <211> 1337
   <212> DNA
   <213> Homo sapiens
<400> 69
   gcggcggact cggcttgttg tgttgctgcc tgagtgccgg agacggtcct gctgctgccg 60
   cagtcctgcc agctgtccga cgatgtcgtc ccacctagtc gagccgccgc cgcccctgca 120
   caacaacaac aacaactgcg aggaaaatga gcagtctctg cccccgccgg ccggcctcaa 180
   cagttcctgg gtggagctac ccatgaacag cagcaatggc aatgataatg gcaatgggaa 240
   aaatgggggg ctggaacacg taccatcctc atcctccatc cacaatggag acatggagaa 300
   gattcttttg gatgcacaac atgaatcagg acagagtagt tccagaggca gttctcactg 360
   tgacagccct tcgccacaag aagatgggca gatcatgttt gatgtggaaa tgcacaccag 420
   cagggaccat agctctcagt cagaagaaga agttgtagaa ggagagaagg aagtcgaggc 480
   tttgaagaaa agtgcggact gggtatcaga ctggtccagt agacccgaaa acattccacc 540
   caaggagttc cacttcagac accctaaacg ttctgtgtct ttaagcatga ggaaaagtgg 600
   agccatgaag aaagggggta ttttctccgc agaatttctg aaggtgttca ttccatctct 660
   cttcctttct catgttttgg ctttggggct aggcatctat attggaaagc gactgagcac 720
   accctctgcc agcacctact gagggaaagg aaaagcccct ggaaatgcgt gtgacctgtg 780
   aagtggtgta ttgtcacagt agcttatttg aacttgagac cattgtaagc atgacccaac 840
   ctaccaccct gtttttacat atccaattcc agtaaccctc aaattcaata ttttattcaa 900
   actctgttga ggcattttac taaccttata ccctttttgg cctgaagaca ttttagaatt 960
   tcctaacaga gtttactgtt gtttagaaat ttgcaagggc ttcttttccg caaatgccac 1020
   cagcagatta taattttgtc ggcaatgcta ttatctctaa ttagtgccac cagactagac 1080
   ctgtatcatt catggtataa attttactct tccaacataa ctaccatctc tctcttaaaa 1140
   cgagatcagg ttagcaaatg atgtaaaaga agctttattg tctagttgtt ttttttcccc 1200
   caagacaaag gcaagtttcc ctaagtttga gttgatagtt attaaaaaga aaacaaaaca 1260
   aaaaaaaaag gcaaggcaca acaaaaaaat atcctgggca ataaaaaaaa tattttaaac 1320
   caaaaaaaaa aaaaaaa 1337
<210> 70
   <211> 664
   <212> DNA
   <213> Homo sapiens
<400> 70
   ggattgttgg tctgcgtgga acttctcagg tggacaccag agcatggaac acatccacga 60
   cagcgatggc agttccagca gcagccacca gagcctcaag agcacagcca aatgggcggc 120
   atccctggag aatctgctgg aagacccaga aggcgtgaaa agatttaggg aatttttaaa 180
   aaaggaattc agtgaagaaa atgttttgtt ttggctagca tgtgaagatt ttaagaaaat 240
   gcaagataag acgcagatgc aggaaaaggc aaaggagatc tacatgacct ttctgtccag 300
   caaggcctca tcacaggtca acgtggaggg gcagtctcgg ctcaacgaga agatcctgga 360
   agaaccgcac cctctgatgt tccagaaact ccaggaccag atctttaatc tcatgaagta 420
   cgacagctac agccgctttc ttaagtctga cttgttttta aaacacaagc gaaccgagga 480
   agaggaagaa gatttgcctg atgctcaaac tgcagctaaa agagcttcca gaatttataa 540
   cacatgagcc cccaaaaagc cgggactggc agctttaaga agcaaaggaa tttcctctca 600
   ggacgtgccg ggtttatcat tgctttgtta tttgtaagga ctgaaatgta caaaaccctt 660
   caat 664
<210> 71
   <211> 1345
   <212> DNA
   <213> Homo sapiens
<400> 71
   aaaacagccg gggctccagc gggagaacga taatgcaaag tgctatgttc ttggctgttc 60
   aacacgactg cagacccatg gacaagagcg caggcagtgg ccacaagagc gaggagaagc 120
   gagaaaagat gaaacggacc cttttaaaag attggaagac ccgtttgagc tacttcttac 180
   aaaattcctc tactcctggg aagcccaaaa ccggcaaaaa aagcaaacag caagctttca 240
   tcaagccttc tcctgaggaa gcacagctgt ggtcagaagc atttgacgag ctgctagcca 300
   gcaaatatgg tcttgctgca ttcagggctt ttttaaagtc ggaattctgt gaagaaaata 360
   ttgaattctg gctggcctgt gaagacttca aaaaaaccaa atcaccccaa aagctgtcct 420
   caaaagcaag gaaaatatat actgacttca tagaaaagga agctccaaaa gagataaaca 480
   tagattttca aaccaaaact ctgattgccc agaatataca agaagctaca agtggctgct 540
   ttacaactgc ccagaaaagg gtatacagct tgatggagaa caactcttat cctcgtttct 600
   tggagtcaga attctaccag gacttgtgta aaaagccaca aatcaccaca gagcctcatg 660
   ctacatgaaa tgtaaaaggg agcccagaaa tggaggacat ttcattcttt ttcctgaggg 720
   gaaggactgt gacctgccat aaagactgac cttgaattca gcctgggtgt tcaggaaaca 780
   tcactcagaa ctattgattc aaagttgggt agtgaatcag gaagccagta actgactagg 840
   agaagctggt atcagaacag cttccctcac tgtgtacaga acgcaagaag ggaataggtg 900
   gtctgaacgt ggtgtctcac tctgaaaagc aggaatgtaa gatgatgaaa gagacaatgt 960
   aatactgttg gtccaaaagc atttaaaatc aatagatctg ggattatgtg gccttaggta 1020
   gctggttgta catctttccc taaatcgatc catgttacca catagtagtt ttagtttagg 1080
   attcagtaac agtgaagtgt ttactatgtg caagggtatt gaagttctta tgaccacaga 1140
   tcatcagtac tgttgtctca tgtaatgcta aaactgaaat ggtccgtgtt tgcattgtta 1200
   aaaatgatgt gtgaaataga atgagtgcta tggtgttgaa aactgcagtg tccgttatga 1260
   gtgccaaaaa tctgtcttga aggcagctac actttgaagt ggtctttgaa tacttttaat 1320
   aaatttattt tgataaataa tattg 1345
<210> 72
   <211> 1082
   <212> DNA
   <213> Homo sapiens
<400> 72
   agctcccttt agcgagtcct tcttttcctg actgcagctc ttttcatttt gccatccttt      60
   tccagcacca tgatggttct gcaggtttct gcggcccccc ggacagtggc tctgacggcg     120
   ttactgatgg tgctgctcac atctgtggtc cagggcaggg ccactccaga gaattacctt     180
   ttccagggac ggcaggaatg ctacgcgttt aatgggacac agcgcttcct ggagagatac     240
   atctacaacc gggaggagtt cgcgcgcttc gacagcgacg tgggggagtt ccgggcggtg     300
   acggagctgg ggcggcctgc tgcggagtac tggaacagcc agaaggacat cctggaggag     360
   aagcgggcag tgccggacag gatgtgcaga cacaactacg agctgggcgg gcccatgacc     420
   ctgcagcgcc gagtccagcc tagggtgaat gtttccccct ccaagaaggg gcccttgcag     480
   caccacaacc tgcttgtctg ccacgtgacg gatttctacc caggcagcat tcaagtccga     540
   tggttcctga atggacagga ggaaacagct ggggtcgtgt ccaccaacct gatccgtaat     600
   ggagactgga ccttccagat cctggtgatg ctggaaatga ccccccagca gggagatgtc     660
   tacacctgcc aagtggagca caccagcctg gatagtcctg tcaccgtgga gtggaaggca     720
   cagtctgatt ctgcccggag taagacattg acgggagctg ggggcttcgt gctggggctc     780
   atcatctgtg gagtgggcat cttcatgcac aggaggagca agaaagttca acgaggatct     840
   gcataaacag ggttcctgag ctcactgaaa agactattgt gccttaggaa aagcatttgc     900
   tgtgtttcgt tagcatctgg ctccaggaca gaccttcaac ttccaaattg atactgctgc     960
   caagaagttg ctctgaagtc agtttctatc attctgctct ttgattcaaa gcactgtttc    1020
   tctcactggg cctccaacca tgttcccttc ttcttagcac cacaaataat caaaacccaa    1080
   ca                                                                  1082
<210> 73
   <211> 1487
   <212> DNA
   <213> Homo sapiens
<400> 73
   ctagcactct gacctagcag tcaacatgaa ggctctcatt gttctggggc ttgtcctcct 60
   ttctgttacg gtccagggca aggtctttga aaggtgtgag ttggccagaa ctctgaaaag 120
   attgggaatg gatggctaca ggggaatcag cctagcaaac tggatgtgtt tggccaaatg 180
   ggagagtggt tacaacacac gagctacaaa ctacaatgct ggagacagaa gcactgatta 240
   tgggatattt cagatcaata gccgctactg gtgtaatgat ggcaaaaccc caggagcagt 300
   taatgcctgt catttatcct gcagtgcttt gctgcaagat aacatcgctg atgctgtagc 360
   ttgtgcaaag agggttgtcc gtgatccaca aggcattaga gcatgggtgg catggagaaa 420
   tcgttgtcaa aacagagatg tccgtcagta tgttcaaggt tgtggagtgt aactccagaa 480
   ttttccttct tcagctcatt ttgtctctct cacattaagg gagtaggaat taagtgaaag 540
   gtcacactac cattatttcc ccttcaaaca aataatattt ttacagaagc aggagcaaaa 600
   tatggccttt cttctaagag atataatgtt cactaatgtg gttattttac attaagccta 660
   caacattttt cagtttgcaa atagaactaa tactggtgaa aatttaccta aaaccttggt 720
   tatcaaatac atctccagta cattccgttc tttttttttt ttgagacagt ctcgctctgt 780
   cgcccaggct ggagtgcagt ggcgcaatct cggctcactg caacctccac ctcccgggtt 840
   cacgccattc tcctgcctca gcctcccgag tagctgggat tacgggcgcc cgccaccacg 900
   cccggctaat ttttgtattt tttagtagag acagggtttc accgtgttag ccaggatggt 960
   ctcgatctcc tgaccttgtg atccacccac ctcggcctcc caaagtgctg ggattacagg 1020
   cgtgagccac tgcgcccggc cacattcagt tcttatcaaa gaaataaccc agacttaatc 1080
   ttgaatgata cgattatgcc caatattaag taaaaaatat aagaaaaggt tatcttaaat 1140
   agatcttagg caaaatacca gctgatgaag gcatctgatg ccttcatctg ttcagtcatc 1200
   tccaaaaaca gtaaaaataa ccactttttg ttgggcaata tgaaattttt aaaggagtag 1260
   aataccaaat gatagaaaca gactgcctga attgagaatt ttgatttctt aaagtgtgtt 1320
   tctttctaaa ttgctgttcc ttaatttgat taatttaatt catgtattat gattaaatct 1380
   gaggcagatg agcttacaag tattgaaata attactaatt aatcacaaat gtgaagttat 1440
   gcatgatgta aaaaatacaa acattctaat taaaggcttt gcaacac 1487
<210> 74
   <211> 1543
   <212> DNA
   <213> Homo sapiens
<400> 74
   ggagtggcca ttcgacgaca gtgtggtgta aaggaattca ttagccatgg atgtattcat     60
   gaaaggactt tcaaaggcca aggagggagt tgtggctgct gctgagaaaa ccaaacaggg    120
   tgtggcagaa gcagcaggaa agacaaaaga gggtgttctc tatgtaggct ccaaaaccaa    180
   ggagggagtg gtgcatggtg tggcaacagt ggctgagaag accaaagagc aagtgacaaa    240
   tgttggagga gcagtggtga cgggtgtgac agcagtagcc cagaagacag tggagggagc    300
   agggagcatt gcagcagcca ctggctttgt caaaaaggac cagttgggca agaatgaaga    360
   aggagcccca caggaaggaa ttctggaaga tatgcctgtg gatcctgaca atgaggctta    420
   tgaaatgcct tctgaggaag ggtatcaaga ctacgaacct gaagcctaag aaatatcttt    480
   gctcccagtt tcttgagatc tgctgacaga tgttccatcc tgtacaagtg ctcagttcca    540
   atgtgcccag tcatgacatt tctcaaagtt tttacagtgt atctcgaagt cttccatcag    600
   cagtgattga agtatctgta cctgccccca ctcagcattt cggtgcttcc ctttcactga    660
   agtgaataca tggtagcagg gtctttgtgt gctgtggatt ttgtggcttc aatctacgat    720
   gttaaaacaa attaaaaaca cctaagtgac taccacttat ttctaaatcc tcactatttt    780
   tttgttgctg ttgttcagaa gttgttagtg atttgctatc atatattata agatttttag    840
   gtgtctttta atgatactgt ctaagaataa tgacgtattg tgaaatttgt taatatatat    900
   aatacttaaa aatatgtgag catgaaacta tgcacctata aatactaaat atgaaatttt    960
   accattttgc gatgtgtttt attcacttgt gtttgtatat aaatggtgag aattaaaata   1020
   aaacgttatc tcattgcaaa aatattttat ttttatccca tctcacttta ataataaaaa   1080
   tcatgcttat aagcaacatg aattaagaac tgacacaaag gacaaaaata taaagttatt   1140
   aatagccatt tgaagaagga ggaattttag aagaggtaga gaaaatggaa cattaaccct   1200
   acactcggaa ttccctgaag caacactgcc agaagtgtgt tttggtatgc actggttcct   1260
   taagtggctg tgattaatta ttgaaagtgg ggtgttgaag accccaacta ctattgtaga   1320
   gtggtctatt tctcccttca atcctgtcaa tgtttgcttt atgtattttg gggaactgtt   1380
   gtttgatgtg tatgtgttta taattgttat acatttttaa ttgagccttt tattaacata   1440
   tattgttatt tttgtctcga aataattttt tagttaaaat ctattttgtc tgatattggt   1500
   gtgaatgctg tacctttctg acaataaata atattcgacc atg                      1543
<210> 75
   <211> 1096
   <212> DNA
   <213> Homo sapiens
<400> 75
   gaattcatta gccatggatg tattcatgaa aggactttca aaggccaagg agggagttgt 60
   ggctgctgct gagaaaacca aacagggtgt ggcagaagca gcaggaaaga caaaagaggg 120
   tgttctctat gtaggctcca aaaccaagga gggagtggtg catggtgtgg caacagtggc 180
   tgagaagacc aaagagcaag tgacaaatgt tggaggagca gtggtgacgg gtgtgacagc 240
   agtagcccag aagacagtgg agggagcagg gagcattgca gcagccactg gctttgtcaa 300
   aaaggaccag ttgggcaagg aagggtatca agactacgaa cctgaagcct aagaaatatc 360
   tttgctccca gtttcttgag atctgctgac agatgttcca tcctgtacaa gtgctcagtt 420
   ccaatgtgcc cagtcatgac atttctcaaa gtttttacag tgtatctcga agtcttccat 480
   cagcagtgat tgaagtatct gtacctgccc ccactcagca tttcggtgct tccctttcac 540
   tgaagtgaat acatggtagc agggtctttg tgtgctgtgg attttgtggc ttcaatctac 600
   gatgttaaaa caaattaaaa acacctaagt gactaccact tatttctaaa tcctcactat 660
   ttttttgttg ctgttgttca gaagttgtta gtgatttgct atcatatatt ataagatttt 720
   taggtgtctt ttaatgatac tgtctaagaa taatgacgta ttgtgaaatt tgttaatata 780
   tataatactt aaaaatatgt gagcatgaaa ctatgcacct ataaatacta aatatgaaat 840
   tttaccattt tgcgatgtgt tttattcact tgtgtttgta tataaatggt gagaattaaa 900
   ataaaacgtt atctcattgc aaaaatattt tatttttatc ccatctcact ttaataataa 960
   aaatcatgct tataagcaac atgaattaag aactgacaca aaggacaaaa atataaagtt 1020
   attaatagcc atttgaagaa ggaggaattt tagaagaggt agagaaaatg gaacattaac 1080
   cctacactcg gaattc 1096
<210> 76
   <211> 2691
   <212> DNA
   <213> Homo sapiens
<400> 76
   gcttgcccgt cggtcgctag ctcgctcggt gcgcgtcgtc ccgctccatg gcgctcttcg 60
   tgcggctgct ggctctcgcc ctggctctgg ccctgggccc cgccgcgacc ctggcgggtc 120
   ccgccaagtc gccctaccag ctggtgctgc agcacagcag gctccggggc cgccagcacg 180
   gccccaacgt gtgtgctgtg cagaaggtta ttggcactaa taggaagtac ttcaccaact 240
   gcaagcagtg gtaccaaagg aaaatctgtg gcaaatcaac agtcatcagc tacgagtgct 300
   gtcctggata tgaaaaggtc cctggggaga agggctgtcc agcagcccta ccactctcaa 360
   acctttacga gaccctggga gtcgttggat ccaccaccac tcagctgtac acggaccgca 420
   cggagaagct gaggcctgag atggaggggc ccggcagctt caccatcttc gcccctagca 480
   acgaggcctg ggcctccttg ccagctgaag tgctggactc cctggtcagc aatgtcaaca 540
   ttgagctgct caatgccctc cgctaccata tggtgggcag gcgagtcctg actgatgagc 600
   tgaaacacgg catgaccctc acctctatgt accagaattc caacatccag atccaccact 660
   atcctaatgg gattgtaact gtgaactgtg cccggctcct gaaagccgac caccatgcaa 720
   ccaacggggt ggtgcacctc atcgataagg tcatctccac catcaccaac aacatccagc 780
   agatcattga gatcgaggac acctttgaga cccttcgggc tgctgtggct gcatcagggc 840
   tcaacacgat gcttgaaggt aacggccagt acacgctttt ggccccgacc aatgaggcct 900
   tcgagaagat ccctagtgag actttgaacc gtatcctggg cgacccagaa gccctgagag 960
   acctgctgaa caaccacatc ttgaagtcag ctatgtgtgc tgaagccatc gttgcggggc 1020
   tgtctgtaga gaccctggag ggcacgacac tggaggtggg ctgcagcggg gacatgctca 1080
   ctatcaacgg gaaggcgatc atctccaata aagacatcct agccaccaac ggggtgatcc 1140
   actacattga tgagctactc atcccagact cagccaagac actatttgaa ttggctgcag 1200
   agtctgatgt gtccacagcc attgaccttt tcagacaagc cggcctcggc aatcatctct 1260
   ctggaagtga gcggttgacc ctcctggctc ccctgaattc tgtattcaaa gatggaaccc 1320
   ctccaattga tgcccataca aggaatttgc ttcggaacca cataattaaa gaccagctgg 1380
   cctctaagta tctgtaccat ggacagaccc tggaaactct gggcggcaaa aaactgagag 1440
   tttttgttta tcgtaatagc ctctgcattg agaacagctg catcgcggcc cacgacaaga 1500
   gggggaggta cgggaccctg ttcacgatgg accgggtgct gaccccccca atggggactg 1560
   tcatggatgt cctgaaggga gacaatcgct ttagcatgct ggtagctgcc atccagtctg 1620
   caggactgac ggagaccctc aaccgggaag gagtctacac agtctttgct cccacaaatg 1680
   aagccttccg agccctgcca ccaagagaac ggagcagact cttgggagat gccaaggaac 1740
   ttgccaacat cctgaaatac cacattggtg atgaaatcct ggttagcgga ggcatcgggg 1800
   ccctggtgcg gctaaagtct ctccaaggtg acaagctgga agtcagcttg aaaaacaatg 1860
   tggtgagtgt caacaaggag cctgttgccg agcctgacat catggccaca aatggcgtgg 1920
   tccatgtcat caccaatgtt ctgcagcctc cagccaacag acctcaggaa agaggggatg 1980
   aacttgcaga ctctgcgctt gagatcttca aacaagcatc agcgttttcc agggcttccc 2040
   agaggtctgt gcgactagcc cctgtctatc aaaagttatt agagaggatg aagcattagc 2100
   ttgaagcact acaggaggaa tgcaccacgg cagctctccg ccaatttctc tcagatttcc 2160
   acagagactg tttgaatgtt ttcaaaacca agtatcacac tttaatgtac atgggccgca 2220
   ccataatgag atgtgagcct tgtgcatgtg ggggaggagg gagagagatg tactttttaa 2280
   atcatgttcc ccctaaacat ggctgttaac ccactgcatg cagaaacttg gatgtcactg 2340
   cctgacattc acttccagag aggacctatc ccaaatgtgg aattgactgc ctatgccaag 2400
   tccctggaaa aggagcttca gtattgtggg gctcataaaa catgaatcaa gcaatccagc 2460
   ctcatgggaa gtcctggcac agtttttgta aagcccttgc acagctggag aaatggcatc 2520
   attataagct atgagttgaa atgttctgtc aaatgtgtct cacatctaca cgtggcttgg 2580
   aggcttttat ggggccctgt ccaggtagaa aagaaatggt atgtagagct tagatttccc 2640
   tattgtgaca gagccatggt gtgtttgtaa taataaaacc aaagaaacat a 2691
<210> 77
   <211> 584
   <212> DNA
   <213> Homo sapiens
<400> 77
   acactcgctt ctggaacgtc tgaggttatc aataagctcc tagtccagac gccatgggtc 60
   atttcacaga ggaggacaag gctactatca caagcctgtg gggcaaggtg aatgtggaag 120
   atgctggagg agaaaccctg ggaaggctcc tggttgtcta cccatggacc cagaggttct 180
   ttgacagctt tggcaacctg tcctctgcct ctgccatcat gggcaacccc aaagtcaagg 240
   cacatggcaa gaaggtgctg acttccttgg gagatgccat aaagcacctg gatgatctca 300
   agggcacctt tgcccagctg agtgaactgc actgtgacaa gctgcatgtg gatcctgaga 360
   acttcaagct cctgggaaat gtgctggtga ccgttttggc aatccatttc ggcaaagaat 420
   tcacccctga ggtgcaggct tcctggcaga agatggtgac tggagtggcc agtgccctgt 480
   cctccagata ccactgagct cactgcccat gatgcagagc tttcaaggat aggctttatt 540
   ctgcaagcaa tacaaataat aaatctattc tgctaagaga tcac 584
<210> 78
   <211> 2179
   <212> DNA
   <213> Homo sapiens
<400> 78
   ggcacgaggg tcatggacct cctgcacaag aacatgaaac acctgtggtt cttcctcctc     60
   ctggtggcag ctcccagatg ggtcctgtcc caggtgcagc tacagcagtg gggcgcagga    120
   ctgttgaagc cttcggagac cctgtccctc acctgcggtg tttatggtgg gtccttcagt 180
   ggttactatt ggagctggat tcgccagccc ccagggaagg ggctggagtg gattggggaa 240
   atcaatcata gtggaagcac caactacaac ccgtccctca agagtcgagt caccatatca 300
   gtagacacgt ccaagaagca gctctccctg aagttgagct ctgtgaacgc cgcggacacg 360
   gctgtgtatt actgtgcgag agttattact agggcgagtc ctggcacaga cgggaggtac 420
   ggtatggacg tctggggcca agggaccacg gtcaccgtct cctcagggag tgcatccgcc 480
   ccaacccttt tccccctcgt ctcctgtgag aattccccgt cggatacgag cagcgtggcc 540
   gttggctgcc tcgcacagga cttccttccc gactccatca ctttctcctg gaaatacaag 600
   aacaactctg acatcagcag cacccggggc ttcccatcag tcctgagagg gggcaagtac 660
   gcagccacct cacaggtgct gctgccttcc aaggacgtca tgcagggcac agacgaacac 720
   gtggtgtgca aagtccagca ccccaacggc aacaaagaaa agaacgtgcc tcttccagtg 780
   attgccgagc tgcctcccaa agtgagcgtc ttcgtcccac cccgcgacgg cttcttcggc 840
   aacccccgca agtccaagct catctgccag gccacgggtt tcagtccccg gcagattcag 900
   gtgtcctggc tgcgcgaggg gaagcaggtg gggtctggcg tcaccacgga ccaggtgcag 960
   gctgaggcca aagagtctgg gcccacgacc tacaaggtga ccagcacact gaccatcaaa 1020
   gagagcgact ggctcagcca gagcatgttc acctgccgcg tggatcacag ggggctgacc 1080
   ttccagcaga atgcgtcctc catgtgtgtc cccgatcaag acacagccat ccgggtcttc 1140
   gccatccccc catcctttgc cagcatcttc ctcaccaagt ccaccaagtt gacctgcctg 1200
   gtcacagacc tgaccaccta tgacagcgtg accatctcct ggacccgcca gaatggcgaa 1260
   gctgtgaaaa cccacaccaa catctccgag agccacccca atgccacttt cagcgccgtg 1320
   ggtgaggcca gcatctgcga ggatgactgg aattccgggg agaggttcac gtgcaccgtg 1380
   acccacacag acctgccctc gccactgaag cagaccatct cccggcccaa gggggtggcc 1440
   ctgcacaggc ccgatgtcta cttgctgcca ccagcccggg agcagctgaa cctgcgggag 1500
   tcggccacca tcacgtgcct ggtgacgggc ttctctcccg cggacgtctt cgtgcagtgg 1560
   atgcagaggg ggcagccctt gtccccggag aagtatgtga ccagcgcccc aatgcctgag 1620
   ccccaggccc caggccggta cttcgcccac agcatcctga ccgtgtccga agaggaatgg 1680
   aacacggggg agacctacac ctgcgtggtg gcccatgagg ccctgcccaa cagggtcacc 1740
   gagaggaccg tggacaagtc caccgagggg gaggtgagcg ccgacgagga gggctttgag 1800
   aacctgtggg ccaccgcctc caccttcatc gtcctcttcc tcctgagcct cttctacagt 1860
   accaccgtca ccttgttcaa ggtgaaatga tcccaacaga agaacatcgg agaccagaga 1920
   gaggaactca aaggggcgca gcctccgggt ctggggtcct ggcctgcgtg gcctgttggc 1980
   acgtgtttct cttccccgcc cggcctccag ttgtgtgctc tcacacaggc ttccttctcg 2040
   accggcaggg gctggctggc ttgcaggcca cgaggtgggc tctaccccac actgctttgc 2100
   tgtgtatacg cttgttgccc tgaaataaat atgcacattt tatccatgaa aaaaaaaaaa 2160
   aaaaaaaaaa aaaaaaaaa 2179
<210> 79
   <211> 3558
   <212> DNA
   <213> Homo sapiens
<400> 79
   cagaagccga aagaactgtt cacatggagc tgtttatttt ccggcctgag gttgccgaga     60
   caattggcga gctgtcttga atatatctct atcaattaaa acagcagctg agataaataa    120
   tgcacctttg ccggaactgc cacagggact gcaggctcag gcttctcaag ccagctcacc    180
   gtccagctga gcgagatgtc agcccaagga aggaacttag atgccttgga aattgatgcc    240
   tcacagttat tttctccaga ggaggtgcag ggtctgggct agggaaacgg aaaggactct    300
   gttgcattta ataaagcctg tatcctatgg cagcagccac taaggagctc accagaataa    360
   gccaatgcca ttcctcattt ggcctgagca gctcagagtc aggaagtcag agcgcagaaa    420
   atccagcagc tgtcagaggg ctccatgttt ggccacggtc tgaagcacct gttccacagc    480
   cgccgtcggt ctcgggaaag ggagcaccag acgtctcagg attcccagca gcatcagcag    540
   cagcagggta tgtccgacca tgactcccca gatgagaagg agcgctctcc ggagatgcat    600
   cgcgtctcct acgccatgtc cctgcacgac ctgcccgccc ggcccaccgc cttcaaccgc    660
   gtgctgcagc agatccgctc ccggccctcc atcaagcggg gcgccagcct gcacagcagc    720
   agtgggggcg gcagcagcgg gagcagcagc cggcgcacca agagtagctc cctggagccc    780
   cagcgtggca gccctcacct gctgcgcaag gccccccagg acagcagcct ggccgccatc     840
   ctgcaccagc accagtgccg tccccgctct tcctccacca ccgacactgc tctgctgctg    900
   gccgacggca gcaacgtgta cctcctggct gaggaggccg aaggcatcgg ggacaaggtc    960
   gataagggag acctggtggc cctgagcctc cccgccggcc atggtgacac cgacggcccc   1020
   atcagcctgg acgtgcccga tggggcaccg gacccccagc ggaccaaggc cgccattgac   1080
   cacctgcacc agaagatcct gaagatcacc gagcagatca agattgagca ggaggctcgc   1140
   gacgacaatg tggcggagta tctgaaactg gccaacaacg cggacaagca gcaggtgtca   1200
   cgcatcaagc aagtgttcga gaagaagaac cagaagtcag cccagaccat cgcccagctg   1260
   cacaagaagc tggagcacta ccgccggcgc ctgaaggaga ttgagcagaa cgggccctcg   1320
   cggcagccca aggacgtgct gcgggacatg cagcaggggc tgaaggacgt gggcgccaac   1380
   gtgcgcgcag gcatcagcgg ctttgggggt ggcgtggtgg agggcgtcaa gggcagcctc   1440
   tctggcctct cacaggccac ccacaccgcc gtggtgtcca agccccggga gtttgccagc   1500
   ctcatccgca acaagtttgg cagtgctgac aacatcgccc acctgaagga ccccctggaa 1560
   gatgggcccc ctgaggaggc agcccgggca ctgagcggca gtgccacact cgtctccagc 1620
   cccaagtatg gcagcgatga tgagtgctcc agcgccagcg ccagctcagc cggggcaggc 1680
   agcaactctg gggctgggcc tggtggggcg ctggggagcc ctaagtccaa tgcactgtat 1740
   ggtgctcctg gaaacctgga tgctctgctg gaagagctac gggagatcaa ggagggacag 1800
   tctcacctgg aggactccat ggaagacctg aagactcagc tgcagaggga ctacacctac 1860
   atgacccagt gcctgcagga ggagcgctac aggtatgagc ggctggagga gcagctcaac 1920
   gacctgactg agcttcatca gaacgagatg acgaacctga agcaggagct ggccagcatg 1980
   gaggagaagg tggcctacca gtcctatgag agggcacggg acatccagga ggccgtggag 2040
   tcctgcctga cccgggtcac caagctggag ctgcagcagc aacagcagca ggtggtacag 2100
   ctggagggcg tggagaatgc caacgcgcgg gcgctgctgg gcaagttcat caacgtgatc 2160
   ctggcgctca tggccgtgct gctggtgttc gtgtccacca tcgccaactt catcacgccc 2220
   ctcatgaaga cacgcctgcg catcaccagc accaccctcc tggtcctcgt cctgttcctc 2280
   ctctggaagc actgggactc cctcacctac ctcctggagc acgtgttgct gcccagctga 2340
   gtggccagcc acaccaaccc tgtgctctct ggcccccagc tggccacact tctccaggag 2400
   ggacccttgg acttctttgt gtgtccagtt tggcctcctg cccaaactgt ccattccagc 2460
   agctcctgcc cccttctctg tacttgcttc tgtctgacac cttctccctg ttggcctgaa 2520
   gggagcttag aatgcagccc tacctggaga tagtgcgggc acctgtggcc aagtggagca 2580
   gaggtggaca tggggttgga ttgttttgat tatttatagt tacacaagga cttctcccag 2640
   ctgaccctca ggatgcccca agtcaggaag accattaaga ataggaggag agggctctgc 2700
   ctcaactttc ctaggaaaga gcccacctcg gagatagcta cggtttcctc tggtggagat 2760
   ggtgaggatg aaggctggag agtgagggag gaggctctgc tggccgcaga gaacacaggg 2820
   atgggagggt ccctagcctt cgggcacctc cagggccaga gagcaggctc agagcagcta 2880
   gtgtggagct cagcatcccc accccacccc tcctccctgt agagctgatt tgaggcctcc 2940
   ttctggggct gggctctgca ggccaggtgg gtgtggcctg tgttttccct tctgttcttt 3000
   ctgcctgtac tggatctgtt attttcaggg aaacaggccc cagggccccc ctgagcctca 3060
   ccctaagccc ttaggcctct gagagtgctg ttgggttcta tttatttatt tatttgttcc 3120
   tttgttccct acccgtgccc ccagtgtctt ccctgctgag taccaggaga ggtcctgccc 3180
   catcctctct ctgaagccag ggcccttcca ttccatttag cctttggatc atcctggctg 3240
   ggagaagtgg gaccgagcca cccagcccca ctatccccaa gcagccctac agccgggatg 3300
   ggaggcacgt ggcctctctt ttatccgtct atttattttg taagtgtatt cgtgtggagg 3360
   aggttgttgc tttatttttt taaggctctg gagtgttgtg tatggtttct tttcacatcc 3420
   cagcctccca tgggcacttc taagaagaga ggggatttct tggaaaagga gagaggaatc 3480
   ccctagagca gggaaagcag tgcctgccag ctgttgtgca ccttcctgag aaataaatat 3540
   cctctaaatt ttcaaacc 3558
<210> 80
   <211> 39455
   <212> DNA
   <213> Homo sapiens
<400> 80
<210> 81
   <211> 885
   <212> DNA
   <213> Homo sapiens
<400> 81
   ctgcagctgc gcccagcctg ccccatcccc tgctcatttg catgttccca gagcacagtc 60
   tcctgacctg aagacttatt aacaggctga tcacaccctg tgcaggagtc agacccagtc 120
   aggacacagc atggacatga gggtccccgc tcagctcctg gggctcctgc tgctctggtt 180
   cccaggtaag aaaggagaac actaggatta tactcggtca gtgtgctgag tactgcttta 240
   ctattcaggg aacttctctt acagcatgat taattgtgtg gacatttgtt tttatgtttc 300
   caatctcagg ttccagatgc gacatccaga tgacccagtc tccatcttct gtgtctgcat 360
   ctgtaggaga cagagtcacc atcacttgtc gggcgagtca gggtattagc agctggttag 420
   cctggtatca gcagaaacca gggaaagccc ctaagctcct gatctatgct gcatccagtt 480
   tgcaaagtgg ggtcccatca aggttcagcg gcagtggatc tgggacagat ttcactctca 540
   ctatcagcag cctgcagcct gaagattttg caacttacta ttgtcaacag gctaacagtt 600
   tccctcccac agtgttacca acccgaacat aaacccccag ggaagcagat gtgtgaagct 660
   gggctgcccc agctgctcct cctgatgcct ccattggctg agagtgttgc tcagatgcag 720
   ccacactctg atggtgttgg tagaggggta cgtgaaatcg cctctgcacc ctaattcttt 780
   tctctttctc agccccaact gcacagacat agcaatgcat ctcctgattt gataaataca 840
   gagatcatga cacttgagga gtctagttta tggcttcagc ttgaa 885
<210> 82
   <211> 2167
   <212> DNA
   <213> Homo sapiens
<400> 82
   gcatttgtgc ctgaagctgc cgggtctgct acggcaccgc ggggctgcag aaacccgggg      60
   gccaagggcg ggctgcttgc cgctatggct ggcagtcagg acatattcga tgccatcgtg     120
   atggcggatg agaggtttca tggggaaggg tatcgggaag gctatgaaga aggcagtagt     180
   ttgggtgtga tggagggaag gcagcatggc acgctgcatg gagccaaaat cgggtctgag    240
   atcgggtgct accaaggttt tgcttttgca tggaaatgtc tactgcacag ttgcaccact     300
   gagaaggaca gcagaaagat gaaggtctta gaatcattga ttggaatgat ccagaaattc     360
   ccttatgatg accctactta cgataaactc catgaagact tagacaagat cagaggaaaa 420
   tttaaacagt tttgttcgtt actcaatgtt cagccagact ttaaaattag tgcagaaggt 480
   tccggacttt cattttgagg aggatggatg aacagagacc gaacgtcgag gaacagatgt 540
   gtgtgtgacg tgtttagaaa tgcggtgaag ggccagacgg tgctgggaag gcagttgttc 600
   attgggaggg tgagggttcc ggttcggccg tgggagggct tccttccctg gggttttctg 660
   cctgtgtcac cttggtgccc gtcttggggc ctctccacac atgccctttg ttgggctgaa 720
   gccgtccctg gcagagccct cgtgcattga cttgacagcc tctccggcag cacaggccta 780
   gctggttctg ggttggagtt ggctctggat agggttagtc accaggcctg gactgaaggc 840
   agttattttt attattatta ttatttgcaa tgagagagat ggttggcccc gaatgaggct 900
   catgggaggt ttggacgggt gctgtgccgc atgtcgaggc cgattgtgtg ccaggcggtg 960
   cgggacgtgc ctcccgtgtg ttatttaatc ccttcaggag cccacaagat gggtgttatt 1020
   ctcattttac agaggaggga ggggagacgc gaagggattg cctggtctaa gggcacccag 1080
   cagcagagct aggacttccg ccctaaggct gtgcctcact gccaccaggc acagccgcct 1140
   ccggaatgca caggcgagtc cctgccctcc ctcccaggcc gcacaggtcc tgccaagcct 1200
   cacggagcac gggggagtct gtggtggcca gtttacctgg gcatctggag acgttcttcg 1260
   ccgagagtcg tcggggtttc ctgcttcaac agtgcttgga cggaacccgg cgctcgttcc 1320
   ccaccccggc cggccgccca tagccagccc tccgtcacct cttcaccgca ccctcggact 1380
   gccccaaggc ccccgccgcc gctccagcgc cgcgcagcca ccgccgccgc cgccgcctct 1440
   ccttagtcgc cgccatgacg accgcgtcca cctcgcaggt gcgccagaac taccaccagg 1500
   actcagaggc cgccatcaac cgccagatca acctggagct ctacgcctcc tacgtttacc 1560
   tgtccatgtc ttactacttt gaccgcgatg atgtggcttt gaagaacttt gccaaatact 1620
   ttcttcacca atctcatgag gagagggaac atgctgagaa actgatgaag ctgcagaacc 1680
   aacgaggtgg ccgaatcttc cttcaggata tcaagaaacc agactgtgat gactgggaga 1740
   gcgggctgaa tgcaatggag tgtgcattac atttggaaaa aaatgtgaat cagtcactac 1800
   tggaactgca caaactggcc actgacaaaa atgaccccca tttgtgtgac ttcattgaga 1860
   cacattacct gaatgagcag gtgaaagcca tcaaagaatt gggtgaccac gtgaccaact 1920
   tgcgcaagat gggagcgccc gaatctggct tggcggaata tctctttgac aagcacaccc 1980
   tgggagacag tgataatgaa agctaagcct cgggctaatt tccccatagc cgtggggtga 2040
   cttccctggt caccaaggca gtgcatgcat gttggggttt cctttacctt ttctataagt 2100
   tgtaccaaaa catccactta agttctttga tttgtaccat tccttcaaat aaagaaattt 2160
   ggtaccc 2167
<210> 83
   <211> 1914
   <212> DNA
   <213> Homo sapiens
<400> 83
   ggcacgaggc gtcctgttgc tggtctccgt ccggtcgccg gccgtctagg tctccggccc      60
   tccccagccg ctcctgcgcc cttgccggcc ccgccgcccg cagccctggc gctccctgcg     120
   ggccccgccg aggccgcctg cgccctgtgc cagcgcgcgc cccgggaacc ggtgcgcgcc     180
   gactgcggcc accgcttctg tcgggcgtgc gtggtgcgct tctgggccga ggaggacggg     240
   cccttcccgt gccccgagtg cgccgacgac tgctggcagc gcgccgtgga gcccggcagg     300
   cccccgctca gccgccgcct tctggcgctc gaggaggcgg ccgcggcgcc cgcgcgcgac     360
   ggcccggcca gcgaggccgc gctgcagctg ctgtgccgcg ccgacgccgg cccgctctgc     420
   gccgcctgcc gtatggctgc gggccccgag ccgcccgagt gggaaccgcg ctggaggaag     480
   gcgctgcgcg gcaaggagaa caaggggtct gtggaaatca tgagaaagga cttgaatgac     540
   gcccgggacc tgcatggcca ggcagagtca gcagctgcag tgtggaaggg acacgtgatg     600
   gaccgtagga agaaggcact gaccgactac aagaagctgc gggccttctt tgtggaggag     660
   gaggagcatt tcctgcagga ggctgagaag gaggaggggc tccctgagga cgagctggct     720
   gaccccactg agcggttcag gtcactgctg caggcggtct cggagctgga gaagaagcat     780
   cgcaacctgg gcctcagcat gctgctgcag tgatggcgcc aacccgtggc agtcccagag     840
   ctggaggcag gaggatggat cctcatctcc atgggaagtg tcagcgtgtg gctgccaggg     900
   aagcgtggca ggcgcctggc cttgggtcca tctacatagt tgcgtgtttc aacaatgtcc     960
   atttatcctt caccctgagg cgtgttttgg gggctgcaaa cacctcccgg tagaggctgg    1020
   acctgaggac ccttcccacc tgtgcccgtc ccttcctgaa gtcctagcca cagcccatcc    1080
   tccatgagtc ccggcagctc tgggtcatgc ccttccctgg tcacccatct gcccctcacc    1140
   tcgtcatcca gggacccaga ccctgcacct tccatgtggg cccacagatc cttggcaggt    1200
   acctgaggtg caccattgag tgtcggattt ggggttagca tccagaaaga agaatgcgca    1260
   tgacgctctg tgaaggctgg aactcaggtc ttcagggaga gaaaggaaga ctggattgca    1320
   ccttgatgcc tcctgaggag gcggcccccc tcttgaggtg ggcgtgggcc cggcccagcc    1380
   ttatccaagt cgctctgtcc acctccccct tcctggcccc caccccactc ctgtgcctcc    1440
   caggagccct ccctgtgctc cacctgcctc cgcagaagga agcctctttc tctgtttccc    1500
   tgggtgaggg ggctggcagg tggctaaccc catttagcat ctccaggccc tgccatggtg    1560
   tctcatcttg ctgttatctc tagctctttc cctcctccca tttcctttag tagttgaatt    1620
   ttgcaaagct tgtagcagta gctcagttgc ctgcagcatc cttgtgtgta gataaattag    1680
   tcgacagaaa ctcagcactg gggacaggat tgcaaagtcg gggacataga tgcagacagt 1740
   tgttgagatt tggggatagc cgggcttgtg agcggtgccc atttccagat gaagcctttc 1800
   agcccttctg agtccccggc ccttggtgcg atgtctgtga gtttgacctg cccagcgtgt 1860
   gggctggctc aatgctgaat aaagtgggtt tgtgtcaaaa aaaaaaaaaa aaaa 1914
<210> 84
   <211> 1119
   <212> DNA
   <213> Homo sapiens
<400> 84
   cggccggccg cccatagcca gccctccgtc acctcttcac cgcaccctcg gactgcccca 60
   aggcccccgc cgccgctcca gcgccgcgca gccaccgccg ccgccgccgc cctctcctta 120
   gtcgccgcca tgacgaccgc gtccacctcg caggtgcgcc agaactacca ccaggactca 180
   gaggccgcca tcaaccgcca gatcaacctg gagctctacg cctcctacgt ttacctgtcc 240
   atgtcttact actttgaccg cgatgatgtg gctttgaaga actttgccaa atactttctt 300
   caccaatctc atgaggagag ggaacatgct gagaaactga tgaagctgca gaaccaacga 360
   ggtggccgaa tcttccttca ggatatcaag aaaccagact gtgatgactg ggagagcggg 420
   ctgaatgcaa tggagtgtgc attacatttg gaaaaaaatg tgaatcagtc actactggaa 480
   ctgcacaaac tggccactga caaaaatgac ccccatttgt gtgacttcat tgagacacat 540
   tacctgaatg agcaggtgaa agccatcaaa gaattgggtg accacgtgac caacttgcgc 600
   aagatgggag cgcccgaatc tggcttggcg gaatatctct ttgacaagca caccctggga 660
   gacagtgata atgaaagcta agcctcgggc taatttcccc atagccgtgg ggtgacttcc 720
   ctggtcacca aggcagtgca tgcatgttgg ggtttccttt accttttcta taagttgtac 780
   caaaacatcc acttaagttc tttgatttgt accattcttc aaataaagaa atttggtacc 840
   caggtgttgt ctttgaggtc ttggatgaat cagaaatcta tccaggctat cttccagatt 900
   ccttaagtgc cgttgttcag ttctaatcac actaatcaaa aagaaacgag tatttgtatt 960
   tattaaactc attagtttgg gcagtatact aaggtgtggc tgtcttggat tcagatagaa 1020
   ctaagggttc ccgactctga atccagagtc tgagttaaat gtttccaatg gttcagtcta 1080
   gctttcacag tttttatgaa taaaaggcat taaaggctg 1119
<210> 85
   <211> 520
   <212> DNA
   <213> Homo sapiens
<400> 85
   caggctcgag gcgtctgccg cacctcagcc cacgacctgc cccgctggga ggtgcgggcc 60
   gctggccagg ccctgaccgc aacctggccc agaggcccca gccctcaggc aaggttctcc 120
   ggtgaagcca cagcctggcc acctgtcttg atctccccac cgagaaggcc ccgcccctcc 180
   cgctgcagcc ccacagcatg cagccccagg agagccacgt ccactatagt aggtgggagg 240
   acggcagcag ggacggagtc agcctagggg ctgtgtccag cacagaagag gcctcacgct 300
   gccgcaggat ctcccagagg ctgtgcacgg gcaagctggg catcgccatg aaggtgctgg 360
   gcggcgtggc cctcttctgg atcatcttca tcctgggcta cctcacaggc tactatgtgc 420
   acaagtgcaa ataaatgctg ccccgcatgc acgcgggggg ctggccgcaa aaaaaaaaaa 480
   aaaaaaaaaa aaaaaaaaaa aaaaaaaaaa aaaaaaaaaa 520
<210> 86
   <211> 894
   <212> DNA
   <213> Homo sapiens
<400> 86
   ggcggcgcta tgctgtcctg cttcaggctc ctctccaggc acatcagccc ttcgctggcg 60
   tctctgcgcc cggtgcgctg ctgcttcgcg ctcccgctgc gttgggcccc ggggcgcccc 120
   ttggacccca ggcagatcgc cccccgccgc cccctggccg cagccgcctc ctcccgggac 180
   cctaccgggc ccgccgccgg cccctctcgg gtgcgccaga acttccaccc cgactccgag 240
   gctgccatca accgccagat caacctcgag ctctatgcgt cctacgtgta cttgtccatg 300
   gcctattact tctcccggga tgacgtggcc ttgaacaact tctccaggta tttccttcac 360
   cagtcccggg aggagaccga gcacgcggag aagctgatga ggctgcagaa ccagcgagga 420
   ggccggatcc gcctgcagga catcaagaag ccggaacagg acgactggga aagcgggctg 480
   catgccatgg agtgtgctct actcttggaa aagaacgtga accagtcgtt gctggaattg 540
   cacgctctag cctcagataa aggtgacccc catttgtgcg atttcctgga aacctactac 600
   ctgaatgagc aggtgaagtc tatcaaagaa ctaggtgacc acgtgcacaa cttagtgaag 660
   atgggggccc cggatgctgg cctggcggag tacctttttg acacacatac ccttggaaat 720
   gaaaacaagc agaactaagc cacgagctgc cttcctccca ggctagtgga tccaaagacc 780
   aaagtcagct gtctcctgct ttcttgccct taaaatcacc tccatcttta tattcttctg 840
   ttatactatt cctccaataa agtgatttgt agaaaaaaaa aaaaaaaaaa aaaa 894
<210> 87
   <211> 1613
   <212> DNA
   <213> Homo sapiens
<400> 87
   ggaagaggag gcttgaggcc cagggtgggc accagccagc catggccaca gccgagaccg 60
   ccttgccctc catcagcaca ctgaccgccc tgggcccctt cccggacaca caggatgact 120
   tcctcaagtg gtggcgctcc gaagaggcgc aggacatggg cccgggtcct cctgacccca 180
   cggagccgcc cctccacgtg aagtctgagg accagcccgg ggaggaagag gacgatgaga 240
   ggggcgcgga cgccacctgg gacctggatc tcctcctcac caacttctcg ggcccggagc 300
   ccggtggcgc gccccagacc tgcgctctgg cgcccagcga ggcctccggg gcgcaatatc 360
   cgccgccgcc cgagactctg ggcgcatatg ctggcggccc ggggctggtg gctgggcttt 420
   tgggttcgga ggatcactcg ggttgggtgc gccctgccct gcgagcccgg gctcccgacg 480
   ccttcgtggg cccagccctg gctccagccc cggcccccga gcccaagggg ctggcgctgc 540
   aaccggtgta cccgggggcc ggcgccggct cctcgggtgg ctacttcccg gggaccgggc 600
   tttcagtgcc tgcggagtcg ggcgccccct acgggctact gtccgggtac cccgcgatgt 660
   acccggcgcc tcagtaccaa gggcacttcc agctcttccg cgggctccag ggacccgcgc 720
   ccggtcccgc cacgtccccc tccttcctga gttgtttggg acccgggacg gtgggcactg 780
   gactcggggg gactgcagag gatccaggtg tgatagccga gaccgcgcca tccaagcgag 840
   gccgacgttc gtgggcgcgc aagaggcagg cagcgcacac gtgcgcgcac ccgggttgcg 900
   gcaagagcta caccaagagc tcccacctga aggcgcatct gcgcacgcac acaggggaga 960
   agccatacgc ctgcacgtgg gaaggctgcg gctggagatt cgcgcgctcg gacgagctga 1020
   cccgccacta ccggaaacac acggggcagc gccccttccg ctgccagctc tgcccacgtg 1080
   ctttttcgcg ctctgaccac ctggccttgc acatgaagcg ccacctttga gccctgccct 1140
   ggcacttgga ctctcctagt gactggggat gggacaagaa gcctgtttgg tggtctcttc 1200
   acacggacgc gcgtgacaca atgctgggtg gttttcccac gaatggaccc tctcctggac 1260
   tcgcgttccc aaagatccac ccaaatatca aacacggacc catagacagc cctgggggag 1320
   cctcttacgg aaaatccgac aagccttcag ccacagggga gccacacaga gatgtccaaa 1380
   ctgtcgtgca aacccagtga gacagaccgc caaataaacg gactcagtgg acactcagac 1440
   cagctcccag atggccctgg acagcaggag agggtgtggg atgaggcttc ccagagaccc 1500
   tgggtctaga aagcggctcc tgaaggtccc ttattgtggc tgatattaac tgtcaatggt 1560
   tatgggtcct ataaaaatgc ccctcccaga taaaaaaaaa aaaaaaaaaa aaa 1613
<210> 88
   <211> 14709
   <212> DNA
   <213> Homo sapiens
<400> 88
   agggaattct ctggggcttt ggggaattta gtgcgtgggt gagccaagaa aatactaatt     60
   aataatagta agttgttagt gttggttaag ttgttgcttg gaagtgagaa gttgcttaga    120
   aactttccaa agtgcttaga actttaagtg caaacagaca aactaacaaa caaaaattgt    180
   tttgctttgc tacaaggtgg ggaagactga agaagtgtta actgaaaaca ggtgacacag 240
   agtcaccagt tttccgagaa ccaaagggag gggtgtgtga tgccatctca caggcagggg 300
   aaatgtcttt accagcttcc tcctggtggc caagacagcc tgtttcagag ggttgttttg 360
   tttggggtgt gggtgttatc aagtgaatta gtcacttgaa agatgggcgt cagacttgca 420
   tacgcagcag atcagcatcc ttcgctgccc cttagcaact taggtggttg atttgaaact 480
   gtgaaggtgt gattttttca ggagctggaa gtcttagaaa agccttgtaa atgcctatat 540
   tgtgggcttt taacgtattt aagggaccac ttaagacgag attagatggg ctcttctgga 600
   tttgttcctc atttgtcaca ggtgtcttgt gattgaaaat catgagcgaa gtgaaattgc 660
   attgaatttc aagggaattt agtatgtaaa tcgtgcctta gaaacacatc tgttgtcttt 720
   tctgtgtttg gtcgatatta ataatggcaa aatttttgcc tatctagtat cttcaaattg 780
   tagtctttgt aacaaccaaa taaccttttg tggtcactgt aaaattaata tttggtagac 840
   agaatccatg tacctttgct aaggttagaa tgaataattt attgtatttt taatttgaat 900
   gtttgtgctt tttaaatgag ccaagactag aggggaaact atcacctaaa atcagtttgg 960
   aaaacaagac ctaaaaaggg aaggggatgg ggattgtggg gagagagtgg gcgaggtgcc 1020
   tttactacat gtgtgatctg aaaaccctgc ttggttctga gctgcgtcta ttgaattggt 1080
   aaagtaatac caatggcttt ttatcatttc cttcttccct ttaagtttca cttgaaattt 1140
   taaaaatcat ggttattttt atcgttggga tctttctgtc ttctgggttc cattttttaa 1200
   atgtttaaaa atatgttgac atggtagttc agttcttaac caatgacttg gggatgatgc 1260
   aaacaattac tgtcgttggg atttagagtg tattagtcac gcatgtatgg ggaagtagtc 1320
   tcgggtatgc tgttgtgaaa ttgaaactgt aaaagtagat ggttgaaagt actggtatgt 1380
   tgctctgtat ggtaagaact aattctgtta cgtcatgtac ataattacta atcacttttc 1440
   ttccccttta cagcacaaat aaagtttgag ttctaaactc attagaattg ttgtattgct 1500
   atgttacatt tctcgacccc tatcacattg ccttcataac gactttggat gtatcttcat 1560
   attgtagatt taggtctaga tttgctagct ccaagtaatt aaggccatgt aggagagcat 1620
   ggtaaccaca gatagaactg gtattatccc aagtggtctg cagactgctg agtggggatg 1680
   ggatctgctc tctgttgaga gttggtaatc attggtttga aatgtgatga aaccactcaa 1740
   gccaatgaag gtgggtgtgt aggtggggag tactttgcca taatatttta aaacattacc 1800
   tggttagagt tctaagtggt acttattttt gtttggttag gggaaagcct gaataaaaac 1860
   agaaatggac acataatatg catattccat agtctttggg aggctggaat gtgcctggga 1920
   tttgggtcta agtgtatgcg taattcttac ctcactaaag aatttgcctt gtttttttcc 1980
   ttttggtgag tgactaaaac gtctgggctt ccctgtgtgc gtgctacagt aagcaagcag 2040
   aggctgtgca aaggtgtgag caggatcacg tggaatctgg aggatacatc ttggcttgca 2100
   aactgcctct gtctcctggg tgggactgtt ctgtccttgc actgctgttc tgtgttacct 2160
   cttggggtgt aaggttttgc ttacaggaga caaactttgg gcgtagaatg gaagccactg 2220
   ccagcctctg tgctgagaag gaaggtgctt gtttcaaagg gagcagcaag ggaggcttgt 2280
   tctactcacc tgggcctgtt tgcctgagaa ggggagataa gggctgaact gggactagcc 2340
   agggggacca acacaaatgg tgggggatca tgacctgaag gattctttcc ttcccatgag 2400
   ctgcagggct ggttgccgtc cttgcaactg tgtcttattt gcctgtgccg ttatatcttg 2460
   gtgacccctc cacgtgtaca ctactgacaa acgggtggag tgctggggag aagtcactgt 2520
   gccgcccacc tagtaaacct tctgtctgtg ctcatggcat ctccaagatg gggcactgct 2580
   gtgtgcagaa tccagggtcc tctttctgct tgcaactcct ttccctggat gccccagaaa 2640
   caatccaggc ctcctttcct atcttacccc tttgctttgc tttttacccc agcacctcta 2700
   taaccgcctt ctcttctttt cagaactcct tgtttctcat cctgtttttt atgattacaa 2760
   aactcttgct tccaccctgg aagataactg ctatagatgc ctgtatgtaa atggtgctgt 2820
   ctccagcaac tggcatgctg aagaagaatt gattcacggg gtataaatgt tggggattgg 2880
   aagtggggat gaaatggcac ttgttgatac aggagcagag aggtgaggcc gactgctgaa 2940
   gacagctcgc caccctcctt gcctccactc caatccaggg gctggggcca cattctttgc 3000
   cttcatttat cctcagatca ggtgagatcg acaggaggtg ttgatggcag tgccagcaat 3060
   tattgctaat ccgtttgcat ccttatgcat agatctgaat tcagactttg tgaatttcca 3120
   gaggtgtggg taatataata gaattcagtg agtgggcatg gctgatcttg tgcaaattaa 3180
   aagttatggg gcataagaat agcaaaagtt gaacttcttt taaaaaggaa agtaccctga 3240
   gagccagtat tggttgaggc tcttcagtat gcccaggttg gcagcactga gaaccgcagg 3300
   aacggcctgt tgttacaaaa aggagattga ctcagctgcc cttggtgcat ctgactgact 3360
   atgactgctg agagattcca aggaccctta atgccagggc taacctctcc atgtgcagtg 3420
   agacctctgg aggaagtgtc atcctctggc tttgtgtggt actcattatg gtgcagtgcg 3480
   ggcatgaaat gaagacaccc aaataggctt acagatacga tatgttttaa atgttcgtat 3540
   ttaacaaaaa catactgaca ctgtttggaa atggcaacag gaagatagca aaatgaatac 3600
   taacattacg aaaagatgaa caggtacatg ttccaaggca ggtggctgtg aacttcctct 3660
   gagtgaaggc atcccctcca gcacctttca gcctgctagt taggacgacc cgccgccacc 3720
   ctccaggacc tccagccctg cactgccttt cctctctttt aaataattct tcattgagtt 3780
   ctaatatgta aaaaaaaaaa gtttactgta aagtttgcaa ataaggaaat tttttttaaa 3840
   agtcctcagt aatcttacca gtaacaattg ttatgggcac atttgctttt ggaagatttc 3900
   ttttgtatgc atgggataag tacattttta aacaaaaatg ggattatgcc ataaattcta 3960
   ttttgtgact ttaatatata gtgaacacct tttttaatga tgacaggatg ttcccttgca 4020
   tggctgtatc aatttaaaca atcttgtttc aatgggcata cagggtattt tctagttttt 4080
   ttttcctctt agaaaataat acttgcgatg actttccttg tagctcagac tttttcacgt 4140
   ctgttgttat ctctttggga atgctgaata catacatttc gagaaggaaa tgactgttaa 4200
   actcttaaga cttcaggttc atattgctaa actgcccagc agggagggat tttttcaatt 4260
   agtgttctca ctggtgaggc aaacctgatg ccttcccctc ttcctcagaa ccggctttat 4320
   cacattgaaa acctttgctc ctccgacgga tcgagtctgc tttccctgtg gatgtgagca 4380
   ttgctttgtc tgctggtgac tgaacatctc taccatgtgt caattggcca tttgtggtgt 4440
   gtgtgtgtgt gcgtgtgtgt gtgtgtgtgt gtgtgtatga ttttctaatt cctagtcatt 4500
   tttctattga ttgttttgca aaagccattt acatcttaag gatattgata atcttttgtt 4560
   atatttgatg caaatatttt tttccagttt ataggttgcc ttttaatttt gtgtttcagg 4620
   tagataaaag ttaaacgatt ttcttaggtt agtttatcac tgtggtttct gaacttgtta 4680
   tgtgtagatc ttttccaccc caagagtaca taaatattaa tccatacttt cttatggaac 4740
   ttgtatggtt tcgtttttta catttaaacc ttcttccccg tggtgtgtgt tgtggaatct 4800
   gtgtttgtgt gaggaggggc atggtgctct cagaacccac ctcctgtggc cagagagccc 4860
   tgtcctgtga gggtggttat cacagtggca gggttcaatt cagaagacct tgagggcagg 4920
   ctgatgtttc ctgaatgggc ccctggttgt tgcttgtccc tgactctcca tttccccatc 4980
   tgagtggatt tggacctaat agggcactgg agctggttcg aatcctgact ggactacttg 5040
   gcaactttat gtctgggagc aagttactta acctccccaa gcctgtgtct gtgaaatgcg 5100
   ggtaaatgaa tgtagatgtt tggcagcagc tactccttgt tgagctctca cagtgaactc 5160
   tcctgcctct gccctccttc cccgcctccc ctggtgccta gcgtcaggtc tagccacttc 5220
   ctcctgggcc cctctccctt ttctgtggct ggctgcctgc ccgcctggcg ctggaccttt 5280
   catgtaacgg gaatcagcat gtatattctg gtctggtctg tttctacact taattttgtt 5340
   tccagtagta tttccctgta ccggcagagt tcacaaacac atttgaagag gctttttctc 5400
   aggattctta accttcccaa aggaagtccc atggatgggt ttctagaagt ctataaatgc 5460
   tctgaaattg tatttttctg tggaaagcat aactttcatc tgcttgttcg tgctcaaaaa 5520
   agatcatgaa tgaatgattg catgatttta tgccattgtg cttatactaa aggatatgta 5580
   gcccatctct tgagctgtta aactgttttg actactttaa atcgtgcagc tgtgagcatc 5640
   tctgtaaatt tagtgtacac atgtatcccc tggagtggca ttgcctcggc agtgagcact 5700
   tatggtttta taactctctt cacagactca aatgactcca gaaagctaca cttcctgttg 5760
   tgagtatatg atatccattt ccctacatag ccactaacat caggttttta caattttatt 5820
   tatttcttgc tactttaaga aatttttgtg gtgaaataca tataatagaa gttgactatc 5880
   tgaatcattt ttaagtatac attcagtagt gttaagtatg tcgccattgt tgtacaacca 5940
   atctccagaa ctttttcatc ttgcaaaaca aactctgtac ccattaaata acattaaaca 6000
   ttccattccc tccagcctca gcaaccccat tctactttct gtttctgtga gtttgactat 6060
   tccaagcact tcatatcagt taaatcatga agtatttgtc tgtctgtgac tggcttattt 6120
   ctctgagcac agtgtcctcg agatgcgtct atgttgtagc atatgtcaga atttccttcc 6180
   tttttaaaag atccaaataa tattcttatt ttatatcttt tttttatcca ttcatccatt 6240
   agtggacact tgggttgctt ttggctattg taaataatgg tgctatgtac aaatatctat 6300
   attattgtat ttacaagtat aatgctgtaa tgtacacaca tctttttgag atcctacctt 6360
   cagttctttt gagtatatag ccagaagtgg tattactaaa tcttacgata tttctatttt 6420
   taatttattg aggaaccact gtagtttttc atagcaactg caccatttta cgttctcacc 6480
   aagagtgcac aagggttccg aggttcccac atcctcccca acacttgtta ttttctgctt 6540
   tttttagatt gcagccatca tagtgggtgt gaggtgacat ttcattgtgg ttttgatttg 6600
   catttcccta atgaggagtg atgctgagca tcttttcata tgcttactgg tcatttgtat 6660
   gttgtctttg gaaaaatgtc tattcaagtc ctttgactat tttaaaaatt gggttattag 6720
   agttatcgtt gttgttgact tgtaggagtt tctttctata ttctggatat taatccccta 6780
   tcagatatat gatttgcaaa tatcttctct tattccataa ggttactttt tcactttgtt 6840
   gattgtgttc tttgatgtat agaagttttt agttttgaaa tagtctaatt tatctgtttt 6900
   tacttttgtg gtctgtgctt ttggtgtcat atccaagaaa tccttgccaa atccaacgtt 6960
   ataaggtact tttaaggtat tttagttgtc ttagtctata tttctgtact cacctttctt 7020
   tatccactca tcagttgatg ggcatgtagg ttggttccat atctttgcaa ttctgaattg 7080
   tgctgtgatc aggtgtcttt ttagtataat gatttactct cctttgcgta gatacccagt 7140
   agtgggattg ctggatcgaa tggtttttat aattttctat tttaccacag tttctctctg 7200
   catttttcct ctttgaccac taaccatgtg aaattctcat attgaccttt ataatgatca 7260
   tgaactctta gtatcattgg gaaggccaca tttgccactt atgattgtaa accttatcct 7320
   ccatttttcc tgttattgtt ggtgcaaaaa gcacctatta taccaggact ttaaaaatca 7380
   gtctgataag tctttgataa gtctaataat aataactgat aagtccattg aatttgcttc 7440
   tgattacttt ttctttagta gctaaacatg tatgtactcc tatgattaca atgaacactc 7500
   ctctccattt aaattaatta tttacattga tgaaatagca aaatgttaat gactaaatac 7560
   tgtcttggtt ttttcgttcc aggtcagtca atattaactt cttataattt tctttttttt 7620
   ctttatgtgt gtgtgtgtgt gtattttttt ttttttaatt tcaatggctt ttggggtaca 7680
   aatggctttt ggtcatatag atgaattcta cagtagtgaa gtctgagatt ttactgcacc 7740
   ggtcacctga gtagtgtaca ttgtacccaa tatgtggttt tttatacctt gcccccctct   7800
   taccctcccc actttgagtc tctagtgtcc attatgtcac tctgtatacc tttttgtacc   7860
   cataagttag ctctcactta taagtgagaa cacacagtat ttggttttcc attcctgagt   7920
   tgcttcactt agaataatat cctccagctc catccaaaat tgctgcaaaa aaaaaaaaaa   7980
   ccacaaacat tattttgttc ttttttattg ctaagtcata ttccatggtg tagagatacc   8040
   acattttatt tatccactca ctggttgatg ggttggttcc acatctttgc aattgtgact   8100
   tgtactgcca tcaagtgtct ttctggtata atgacttctt ttcctttggg tagataccca   8160
   ggagtgggat tgctagatca aatggttctt aacattttct ctctggatct atttctggaa   8220
   attttaggct ccagtttttg ttgttgttgt taataaaatg caatggaatg taatgatcat   8280
   cacttttcat tatgctttaa aatctggtaa atggaggcta gaacactcct gtaaggcaag   8340
   aatattctct ctgttggaac tcaaatacac agaactgggt aaatctcaat cttaatcttt   8400
   gattcaggac acaacatggc tctcttttac ttgctttctt taattgtttt ttaataatgt   8460
   ggtaagcatt tctgaatctc ctatccaata caaaaactag gacaatacag acagtaactc   8520
   ctatggttac aatgaacact cctctccact taaattaatt atttacactg atgaaattga   8580
   aatagcaaaa ttttaatgac taaatactgt ctttgatttt ttgttccagg tctgtcaata   8640
   ttaacttctt ataattttct ttttttttct ttatgtgtgt gtgtgtgtgt atatatatat   8700
   atatttaatt tcaatggctt ttggggtaca aatggctttt ggtcatatat atgagttcta   8760
   cagtagtgaa gtctgagatt ttactacacc ttccacttat gtggtcccac accacccgcc   8820
   tcccctgccg cctcctgcca ccccctaggc caaggtaata atcatcctga atcctgggtt   8880
   tatctctcac ttgctttctt ttcatataat tttgcaaaag aatctgatct aaatgtgttt   8940
   ttcagagtat atatttatat tttagctgtt cttagagaaa atttattatt ttgcatgtaa   9000
   tcttatggaa cattctcatt taataccatg gtaagattca gcccttgccc aggggatagt   9060
   tcatttagtt tgtttactgg atagagctca tcatgtgact atacctcagt tagtttatca   9120
   gttctcccat ccatggtgac taggttgcct ctcagcctct caacaacact gtttctcagt   9180
   gtccttgtag aagtgatatg tgggtgtttt ctccttacac agagttgaaa ggtgacgaca   9240
   acaacgttgg cactaccaat cccccaccct ccagaggggt aaccagtgtt accagtttgc   9300
   tgtgtttcct gctacacctc gccttattca cttccatttg tatctgaaaa acgtgttgca   9360
   tggtttcttt tctatagaag tggtaaaatg ctattgtgtc ctgtacatta ttgattactt   9420
   tttttcattt aacagtaggg agatgcctgg gagtacacag agaactgccc tcattgtttt   9480
   caacttctgc actgtatgtc tgtgagttta gccattctgc tgttaatgga aatttacagt   9540
   attctaatct tttgatatta caaacagttc tgtgcgatca tcgtcataca caaccccttg   9600
   tgcacaatgc atgagtgttt ctcagggtag gtaccaagaa gtgaaattcc tgggtcatag   9660
   ggcgtgagtc cgacattttt ctccattctg ccctgttgcc ctccagagtg ggtgtccagc 9720
   tttgcatacc taagtatgag agtatctgtt gttcatatcc tctacgacgc tccatatatg 9780
   aaacttaagt ttctgctagt tgccatcttt gatctatcat gtatgcagtg acctactaag 9840
   actgtaattg gtacagtaga ttcttgtcat ctgtgtgtga atttagcatt catgggctta 9900
   atgctgacaa ggcccccagg gtccaagaca tataatcatg tataattttg tcaaggtata 9960
   attttttaaa ttgcttttgt catgtgtctg ctggtgatgc ccaacccagt gctctgcacc 10020
   caggtcacac tgtggctttg tcctctgctt atgcctgcat tgcagcaact gtcctgaaga 10080
   gaccaaaatt atgcagattt aggtaagtcc atggctaatg ttattatatt atgtgctatt 10140
   gtaatggatg gggctgtgga gtgtatgaat ttataaatca ctggtcttgt aattaaaatt 10200
   caaacactat agaaaaaggc catgtagaag ataaaagttc ctctataatc ccggacccct 10260
   aagataacta ctaatgacaa cttcatttat attccttcag acattttctg gctgtggatg 10320
   tactaaaatg tatcctatta ttctctgccc taaaatggaa tcatacaagg tgtactgtta 10380
   tttttatggc tctataacat gtcatattgt acgtgttggt atggtcattt taaccatttt 10440
   tctagtgatg gctttgaggt tatttgcagt ttcctagcca tctcaaagtg tgctgcgggg 10500
   atctcttttg catccctctg ggtgcagagc tgaggcaccc agaggcagtg tccagaggag 10560
   gcagcatctg taggtgtctt cacctgctct ggctcttggc acatctggtt ggtgacactg 10620
   ttttgtgaga tgggttgaaa gcacgtgctg ccaaaataga ataatgttgg tcctctcctc 10680
   atgtgccgtg gaactggggt aaaactgcgt agtggctgca gctgcctgtc cataccggaa 10740
   tcgagtataa cacggtgcct ggcttagcac aaaacagtag tgggtcctgc aggccccaga 10800
   gtctaattcc tggtattctt tcccctacac agattaaata aaccaaaaac aaactattct 10860
   aggaaagcgt ctgtgacatt tgtaaaaagt ggtatttaat gatcttttat tcacttgtct 10920
   gtttagtttg ttgaaatctt aagtggcatc ctggtctggg aaggagtgct gtctgcgcct 10980
   gccctccgct gggcacagcg tggctgcttc aggggctaag cacacacttt ctgtcttcta 11040
   aagggccgcc acatgccagg agctcaggtg tgagcccggc tctggctctt acctcatagg 11100
   gtcactcata ggggcacagg gagcagaaca ttgtacacag cgaggcacca cccggcttgg 11160
   catctgcctc ggtggactta ctacctctag aaggaaatac ctgagttcct ctggcctcag 11220
   ctcctagagt gactggtgtg ctgtccctgt tactcttctg tcaaggtgac aactgtgtga 11280
   cccatcatct gtgtgtcaaa gcaaggccct gcctgggcct ctgctcctgt gctgacccca 11340
   aaggcaaatg ctttgctagt ttccttccag ttaatttcac ctatgaatag atgtgtgaaa 11400
   actgttcaaa gccatacctg cacatgtttg aacttcaaac cctgtgggtg attcagtggc 11460
   atctttctct aacccccagc ctcccttccc acagaggcca ccgtcatggc cagttgctgc 11520
   agtttctttc cagagaacct gtgtatgtgt aaagctgtac aggcgtgggt acaccacaca 11580
   gcctgtcttg cactgtggac tgttgagtta ctagtacatc taggtaagca ccgcatatct 11640
   gtattcatgt ctgccttggt cttttcaaca tctgtgtggt agccgtgttt gaattaccca 11700
   ttcccttttt ggggaaccat taagttgttt cagcaatttt tactgtagat aaggctatac 11760
   cgcatatctg tgtacatggg tttttatgta catgggcaag tatatctgtg agagaaaagt 11820
   ttcctcagga ggaattctgg gcacagcatg tgtaaatttc taaatatgat ggacaccccc 11880
   agcttccacc tcaaggaggt tggtcccatt gacatttccc cacaccttca cccaggctgt 11940
   gcccttaaac ttggttattt gtcaatgtga gaagtggaaa atagtattta attgtagttt 12000
   ggatttgtat ttctattggg ttgtatactt actgattaat aataagagct ctttacatat 12060
   taaggaaatt aacccttttc aaatacattc ctatttctca ctaatcttta agttttattg 12120
   taatattttg ctctttagtt tatatatata tgtatatata tatatatgta tatatatata 12180
   tatacatata tatatatata tatatatata tatatatata tatatatata tatatatata 12240
   tatatatata tacatatata tatacatata tatatactaa ttttctttta tggttcctgg 12300
   attttgtgag tagtttgaaa aggctaatcc agctgaagat tttgttgttg ttgttaaacc 12360
   ccatgttttc tcctaactct ttttattttt attttggagg actctatcta gacttaattt 12420
   tagcataaca agtgacaggg ttagttagcc tgttgtcctt acaccatttt ctggctaata 12480
   cagctattaa ctattgatct gtctattcac gtgccagttc ctaatggttt tacatagtgt 12540
   aatctgcact tcaaaatagc gaagggaagc cctacctcat tattctactt ttccagaatt 12600
   ctcctggcta ttccaggctg catgtttacc ttaaccttcc ctgtgatgtc ttcatgccgt 12660
   tgtcttctta tgcaagaata aggtacgtct ttccatccac tcacgtctat ttaatttgac 12720
   tttgcattac acagaaagct ggtcttggtc tgtctacctc ggcatctagt tgtcctcact 12780
   gccccctagc cgaccccacc ccatctgact gactacccca tcacagagta cttttattta 12840
   cgttttgctc tgcctaatgg ttacttgata ctgtcacgcc gacagtgtcc agttcagtgg 12900
   tctttgcagt tgaaatgctc ccgtacacac tgtcttgtta aaaatgccag taagttcata 12960
   caaacccagc ttgcacccaa ggtcacattc agagagcgta gggctgggat gggttgtttt 13020
   ccaagcttct gccactgtgt ggctagctct tcccactggg aagttctgtg tacccggaat 13080
   gtcggagtgg agtcctgttc tagtgtccag cacctgaccc tgtgcccaac ccctcaacag 13140
   cctattcctg ctgtccacag cctgctggaa ctttttacaa aatatgttgc catgctggac 13200
   cctgggcact ggacataagc cccctggcag cctttttcat gtcacccaaa ggggtaattg 13260
   tcctactggt ggtctgtaag atgagttagg gtgacttgct aatagacatt gtaaatctta 13320
   atatttatgt atgtatttta ttattaccgg ttttccattt atgatggtaa tattgtttct 13380
   tctaagaata tttatttttc cttctaaata ttgagataaa attcatgctt ttgaaatgtt 13440
   ctattcagtg gcttttagta tatttgctat gttgtgcaac catcgacact atccatttct 13500
   agaacttttt cgtcatccca aacagacgct ctgtattcat aaaaaaataa cttcctacct 13560
   gtctctcccc ctagtctttg gtaacctttg ttatactggt aaactttgtt gtgctctctg 13620
   tctgtgtgaa tttgcctatt ctaggggcct catataagtg taatcataca gtatttgtct 13680
   ttttgggtct gtctgatttc acttagcggg ttttcagggt tcattcatgt tgcagcatat 13740
   aacagtactg cgttcctttt tctggctgaa taatattcca ctgtatggat agaccccatt 13800
   ttgtttattc acacatcatt tggacatttg gattatttct ggtttttggc tattatgaac 13860
   aatggtgcta tgaacagttg cgtacaagtt tttgtgtgaa catatgtttt caattctctc 13920
   attatatacc taggagtaga attactgggt catatggtaa ctgtatattt ttgaggaact 13980
   gccaaactat tttcccacgt ccatgcacca tttcacattc ccaccagtaa gtaagagggt 14040
   tccaatttct gcgcattctt gccaacacta gttattatct gactttctgg ttataatcat 14100
   tctaatgagt gtgaagtagc ctctggtgtc atttggattt gcatttctct gatgagtgat 14160
   gctatcaagc acctttgctg gtgctgttgg ccatatgtgt atgttccctg gagaagtgtc 14220
   tgtgctgagc cttggcccac tttttaatta ggcgtttgtc tttttattac tgagttgtaa 14280
   gagttcttta tatattctgg attctagacc cttatcagat acatggtttg caaatatttt 14340
   ctcccattct gtgggttgtg ttttcacttt atcgataatg tccttagaca tataataaat 14400
   ttgtatttta aaagtgactt gatttggctg tgcaaggtgg ctcacgcttg taatcccagc 14460
   actttgggag actgaggtgg gtggatcata tgaggaggct aggagttcga ggtcagcctg 14520
   gccagcatag cgaaaacttg tctctactaa aaatacaaaa attagtcagg catggtggtg 14580
   cacgtctgta ataccagctt ctcaggaggc tgaggcacga ggatcacttg aacccaggag 14640
   gaggaggttg cagtgagctg agatcatgcc agggcaacag aatgagactt tgtttaaaaa 14700
   aaaaaaaaa
<210> 89
   <211> 1821
   <212> DNA
   <213> Homo sapiens
<400> 89
   aatgaggcca gctggactac gccgagacaa ctgggagagg cgcgggactc gcccgttccg 60
   cggaacgccg ggaaggggtc acctcctgat gaagtttccg gttccggtgt cagcggcggt 120
   tgaattgcca tggcaatgcg gtgggcgcgc gcttgtcgtg ttggtctctt gggaggtagt 180
   ggggctaggc cgggcgggta tccgcctctc ccagcttagg tgagcgtccc cgggcgcctc 240
   cggagcgccg cggccgcatg cagttcgtcg tggcggggag ccggagcctg accggggttc 300
   cagcgctcgg gccgtagcct tggctcctgg actttccctg gctccgccgc cacgtgggag 360
   ctgaggctct ggggcttccg cctccggcgc gcgattattt ctctagaaca gttttcattt 420
   ttaaaatttg taaagcgctt ttgcctgtgt gatttcctct gggttttttt ttttttttct 480
   tcctttttgt agagacggaa ttggcggcgg gggcgggggg tcgatgtctc acttttttgc 540
   ccaggctggt ctcgaactcc tggcttcaag ggatcctcct gcctcggcct cttaaagtgc 600
   tgggattaca ggcgtgagcc accgcccccg gccgcctctg agtttccagc ctcgttggcc 660
   ctccagcctt ttaacctgtt gggcctagga tcaggaaagg tttgttgaat ggggaactaa 720
   gaagtgaatt cgttcgttcg acaaacgttt cctgagcagc cgctgggtgc taggcgcagt 780
   gccagcgcgg aatgtccagg gagacctggt gcccaaagct tggacccatc gtgagaaatg 840
   agaagcagat acaaagcagt gtgggagtgc agaggagaca aagcaagcct catcaggccc 900
   attgcttgct ctgctctccc ttgtacttac cagtgcttga caatatacag ttatttacta 960
   gcttggttat tgacttccta tccagcactc agttttattc actgctgtat cctcagtgcc 1020
   taggacgatg cttggaacgt ggtaagtgct cctattggcg ggaagaataa atccggaaga 1080
   gcaggaccag tggacttgct acataatctg tagtcttgga gccgcacagg gttggtggta 1140
   ccctcgagca caccagactt gcagaaaaag catactccag aggaagctga ggcatgcctg 1200
   ctcgagagcc agctgttcca tgtgcaattt tcctctgata gtttctggtc actgttgcca 1260
   cggtgataat gactgggcta tgtcattatc tatccgccaa cagtaagaga agctttgcag 1320
   tcgagatatt gtttagcaga tggagtgttt tctgttgaac actaagtact gccacaagtt 1380
   actttttttt tttttaaact ttgagtattt ttttacaatg ttgctggagg tgatctgttt 1440
   atgctttgag agtgttcgaa tttaaaatca gaaaatcatg tcagtgagtg agtctttcaa 1500
   ataatccttc ggcatgaaac ctgagcctag taaactatga aagtaaactc ggcacattac 1560
   ccgaaagtct caatgtcata ttttcacccc catcaatatt attgatgatt gctcattttc 1620
   taatgtggga cctgaaattt accaggtgct taaagaatct ttttgttttt cagattcatt 1680
   gattccaggt aaatcagagg aacaagcaac atgaacagaa atatgtagaa aaagctatta 1740
   tgcagaagca taattgttgt ttcagaagtc cagcatctgg tgcacttaac aatagagaat 1800
   atattaaact ctttccaaaa t 1821
<210> 90
   <211> 2856
   <212> DNA
   <213> Homo sapiens
<400> 90
   tagtcgcggg tccccgagtg agcacgccag ggagcaggag accaaacgac gggggtcgga     60
   gtcagagtcg cagtgggagt ccccggaccg gagcacgagc ctgagcggga gagcgccgct    120
   cgcacgcccg tcgccacccg cgtacccggc gcagccagag ccaccagcgc agcgctgcca    180
   tggagcccag cagcaagaag ctgacgggtc gcctcatgct ggctgtggga ggagcagtgc 240
   ttggctccct gcagtttggc tacaacactg gagtcatcaa tgccccccag aaggtgatcg 300
   aggagttcta caaccagaca tgggtccacc gctatgggga gagcatcctg cccaccacgc 360
   tcaccacgct ctggtccctc tcagtggcca tcttttctgt tgggggcatg attggctcct 420
   tctctgtggg ccttttcgtt aaccgctttg gccggcggaa ttcaatgctg atgatgaacc 480
   tgctggcctt cgtgtccgcc gtgctcatgg gcttctcgaa actgggcaag tcctttgaga 540
   tgctgatcct gggccgcttc atcatcggtg tgtactgcgg cctgaccaca ggcttcgtgc 600
   ccatgtatgt gggtgaagtg tcacccacag cctttcgtgg ggccctgggc accctgcacc 660
   agctgggcat cgtcgtcggc atcctcatcg cccaggtgtt cggcctggac tccatcatgg 720
   gcaacaagga cctgtggccc ctgctgctga gcatcatctt catcccggcc ctgctgcagt 780
   gcatcgtgct gcccttctgc cccgagagtc cccgcttcct gctcatcaac cgcaacgagg 840
   agaaccgggc caagagtgtg ctaaagaagc tgcgcgggac agctgacgtg acccatgacc 900
   tgcaggagat gaaggaagag agtcggcaga tgatgcggga gaagaaggtc accatcctgg 960
   agctgttccg ctcccccgcc taccgccagc ccatcctcat cgctgtggtg ctgcagctgt 1020
   cccagcagct gtctggcatc aacgctgtct tctattactc cacgagcatc ttcgagaagg 1080
   cgggggtgca gcagcctgtg tatgccacca ttggctccgg tatcgtcaac acggccttca 1140
   ctgtcgtgtc gctgtttgtg gtggagcgag caggccggcg gaccctgcac ctcataggcc 1200
   tcgctggcat ggcgggttgt gccatactca tgaccatcgc gctagcactg ctggagcagc 1260
   taccctggat gtcctatctg agcatcgtgg ccatctttgg ctttgtggcc ttctttgaag 1320
   tgggtcctgg ccccatccca tggttcatcg tggctgaact cttcagccag ggtccacgtc 1380
   cagctgccat tgccgttgca ggcttctcca actggacctc aaatttcatt gtgggcatgt 1440
   gcttccagta tgtggagcaa ctgtgtggtc cctacgtctt catcatcttc actgtgctcc 1500
   tggttctgtt cttcatcttc acctacttca aagttcctga gactaaaggc cggaccttcg 1560
   atgagatcgc ttccggcttc cggcaggggg gagccagcca aagtgataag acacccgagg 1620
   agctgttcca tcccctgggg gctgattccc aagtgtgagt cgccccagat caccagcccg 1680
   gcctgctccc agcagcccta aggatctctc aggagcacag gcagctggat gagacttcca 1740
   aacctgacag atgtcagccg agccgggcct ggggctcctt tctccagcca gcaatgatgt 1800
   ccagaagaat attcaggact taacggctcc aggattttaa caaaagcaag actgttgctc 1860
   aaatctattc agacaagcaa caggttttat aattttttta ttactgattt tgttattttt 1920
   atatcagcct gagtctcctg tgcccacatc ccaggcttca ccctgaatgg ttccatgcct 1980
   gagggtggag actaagccct gtcgagacac ttgccttctt cacccagcta atctgtaggg 2040
   ctggacctat gtcctaagga cacactaatc gaactatgaa ctacaaagct tctatcccag 2100
   gaggtggcta tggccacccg ttctgctggc ctggatctcc ccactctagg ggtcaggctc 2160
   cattaggatt tgccccttcc catctcttcc tacccaacca ctcaaattaa tctttcttta 2220
   cctgagacca gttgggagca ctggagtgca gggaggagag gggaagggcc agtctgggct 2280
   gccgggttct agtctccttt gcactgaggg ccacactatt accatgagaa gagggcctgt 2340
   gggagcctgc aaactcactg ctcaagaaga catggagact cctgccctgt tgtgtataga 2400
   tgcaagatat ttatatatat ttttggttgt caatattaaa tacagacact aagttatagt 2460
   atatctggac aagccaactt gtaaatacac cacctcactc ctgttactta cctaaacaga 2520
   tataaatggc tggtttttag aaacatggtt ttgaaatgct tgtggattga gggtaggagg 2580
   tttggatggg agtgagacag aagtaagtgg ggttgcaacc actgcaacgg cttagacttc 2640
   gactcaggat ccagtccctt acacgtacct ctcatcagtg tcctcttgct caaaaatctg 2700
   tttgatccct gttacccaga gaatatatac attctttatc ttgacattca aggcatttct 2760
   atcacatatt tgatagttgg tgttcaaaaa aacactagtt ttgtgccagc cgtgatgctc 2820
   aggcttgaaa tcgcattatt ttgaatgtga agggaa 2856
<210> 91
   <211> 920
   <212> DNA
   <213> Homo sapiens
<400> 91
   gcacggaggg gcagagaccc cggagcccca gccccaccat gaccctcggc cgccgactcg     60
   cgtgtctttt cctcgcctgt gtcctgccgg ccttgctgct ggggggcacc gcgctggcct     120
   cggagattgt ggggggccgg cgagcgcggc cccacgcgtg gcccttcatg gtgtccctgc     180
   agctgcgcgg aggccacttc tgcggcgcca ccctgattgc gcccaacttc gtcatgtcgg     240
   ccgcgcactg cgtggcgaat gtaaacgtcc gcgcggtgcg ggtggtcctg ggagcccata     300
   acctctcgcg gcgggagccc acccggcagg tgttcgccgt gcagcgcatc ttcgaaaacg     360
   gctacgaccc cgtaaacttg ctcaacgaca tcgtgattct ccagctcaac gggtcggcca     420
   ccatcaacgc caacgtgcag gtggcccagc tgccggctca gggacgccgc ctgggcaacg     480
   gggtgcagtg cctggccatg ggctggggcc ttctgggcag gaaccgtggg atcgccagcg     540
   tcctgcagga gctcaacgtg acggtggtga cgtccctctg ccgtcgcagc aacgtctgca     600
   ctctcgtgag gggccggcag gccggcgtct gtttcgggga ctccggcagc cccttggtct     660
   gcaacgggct aatccacgga attgcctcct tcgtccgggg aggctgcgcc tcagggctct     720
   accccgatgc ctttgccccg gtggcacagt ttgtaaactg gatcgactct atcatccaac     780
   gctccgagga caacccctgt ccccaccccc gggacccgga cccggccagc aggacccact     840
   gagaagggct gcccgggtca cctcagctgc ccacacccac actctccagc atctggcaca     900
   ataaacattc tctgttttgt                                                920